# EUROPEAN PATENT APPLICATION

(11) **EP 1 167 386 A1**
(43) Date of publication of application: **02.01.2002**
(21) Application number: 01305509.0
(22) Date of filing: 26.06.2001
(51) Int. Cl.: C07K 14/72, C12N 15/85, A01K 67/027, C07K 16/28, C12Q 1/68, C12N 15/12, A61P 1/00, A61K 38/17, G01N 33/50

(54) **Canine and feline melanocortin-4 receptor sequences and screening assays to identify compounds useful in regulating animal appetite and metabolic rate**

(30) Priority: 26.06.2000 US 213909 P
(71) Applicant: Pfizer Products Inc., Groton, Connecticut 06340 (US)
(72) Inventor: Hickman, Mary Anne, Pfizer Global R & D, Groton, Connecticut 06340 (US); Houseknecht, Karen Lynne, Pfizer Global R & D, Groton, Connecticut 06340 (US); Robertson, Alan Scott, Pfizer Global R & D, Groton, Connecticut 06340 (US)
(74) Representative: Simpson, Alison Elizabeth Fraser

(57) **Abstract**

The present invention relates to novel nucleic acids encoding canine and feline melanocortin 4 receptors and their gene products. Furthermore, the present invention relates to screening assays to identify compounds that modulate the activity or expression of the melanocortin 4 receptors of the invention. In addition, the present invention relates to methods and therapeutic compositions for the treatment of appetite-related, metabolic and reproductive disorders related to inadequate food intake and energy metabolism, comprising administering to animals compounds that modulate the activity or expression of melanocortin receptors. In one aspect, the invention relates to methods and compositions that antagonize the activity or expression of melanocortin 4 receptors in order to enhance the appetite of diseased, stressed or injured companion animals, livestock or poultry comprising administering compounds that antagonize the activity or expression of the novel melanocortin 4 receptors of the present invention. In another aspect, the invention relates to methods and compositions that agonize the activity or expression of melanocortin 4 receptors in order to treat, *e.g.*, obesity of companion animals, such as cats and dogs comprising administering compounds that agonize the activity or expression of the novel melanocortin 4 receptors of the present invention.

## Description

### Field of the Invention

The present invention relates to novel nucleic acids encoding canine and feline melanocortin 4 receptors and their gene products. The present invention further relates to screening assays to identify compounds that modulate the activity or expression of the novel melanocortin 4 receptors of the invention. The present invention also relates to methods and therapeutic compositions for the treatment of inappropriate food intake comprising administering to animals compounds that modulate the activity or expression of melanocortin receptors. In one aspect, the invention relates to methods and compositions that antagonize the activity or expression of melanocortin 4 receptors in order to enhance the appetite of companion animals, livestock or poultry comprising administering compounds that antagonize the activity or expression of the novel melanocortin 4 receptors of the present invention. In another aspect, the invention relates to methods and compositions that agonize the activity or expression of melanocortin 4 receptors in order to treat, e.g., obesity of companion animals, such as cats and dogs comprising administering compounds that agonize the activity or expression of the novel melanocortin 4 receptors of the present invention.

### Background of the Invention

The regulation of appetite is of great practical importance in the care and rearing of companion and agricultural animals. Of the factors affecting an animal's body weight, including fat metabolism and storage, appetite is particularly sensitive to conditions encountered by animals used in agriculture. Poor appetite can cause low nutrient intake that increases the health risks faced by an animal. For example, *post partum* sows and dairy cows may suffer from reduced feeding behavior. This has a negative effect on lactation and reproductive performance. Shipping and crowding stress also can reduce an animal's nutrient intake, thereby causing loss of body weight. There is evidence that neonatal animal growth may be limited by low nutrient intake, causing deleterious effects that may persist throughout the animal's life. Appetite-related diseases and conditions also plague companion animals. At one extreme, various feline and canine disease states, particularly cancer, renal failure and cardiac disease, can cause anorexia and elevated basal metabolic rate. At the other extreme, many companion animals suffer from obesity.

Despite intensive study, the regulation of animal appetite is still poorly understood. At the anatomic level, the hypothalamus regulates fat storage by controlling food intake and whole-body metabolic rate. Experimental ablation of the ventromedial nucleus (hereinafter "VMH") or paraventricular nucleus (hereinafter "PVN") in the hypothalamus produces massive obesity. Bray *et al.,* 1990, *Frontiers in Neuroendocrinology* 11:128-181. Conversely, damage to the lateral hypothalamus reduces body fat. *Id.*

Recent research efforts have focused on the molecular mechanisms regulating appetite, body fat stores, energy metabolism and nutrient balance. They have revealed a complex feedback system involving many endocrine, neuroendocrine and metabolite mediators. Flier et al., 1998, *Cell* 92:437-440. Attempts are now being made to understand the role played by the melanocortins in appetite regulation and energy metabolism. Melanocortins (a variety of different peptide products resulting from post-translational processing of pro-opiomelanocortin) are known to have a broad array of physiological actions. Aside from their well-known effects on adrenal cortical function (*e*.*g*., adrenocorticotropic hormone (hereinafter "ACTH")), and on melanocytes (*e*.*g*., by melanocyte stimulating hormone (hereinafter "α-MSH")), melanocortins have been shown to affect behavior, learning and memory, control of the cardiovascular system, analgesia, thermoregulation, and the release of other neurohumoral agents including prolactin, luteinizing hormone, and biogenic amines. Peripherally, melanocortins have been identified to have immunomodulatory and neurotrophic properties and to be involved in events surrounding parturition.

The melanocortins mediate their effects through melanocortin receptors (hereinafter "MCRs") -- a subfamily of G-protein coupled receptors. U.S. Patent No. 5,622,860. The MCR family includes five (5) subtypes that are mostly expressed in various areas of the brain. Adan and Gispen, 1997, *Peptides* 18:1279-1287. The first MCRs cloned were the human and mouse melanocyte MSH receptor, MCR1, and the human adrenocortical ACTH receptor, MC2R. Mountjoy et *al*., 1992, *Science* 257:1248-1251; Chhajlani and Wikberg, 1992, *FEBS Lett.* 309:417-420. Subsequently, three additional melanocortin receptor genes were cloned that recognize the core heptapeptide sequence (*i*.*e*., the peptide sequence MEHFRWG using standard one-letter amino acid abbreviations) of melanocortins. Two of these receptors have been shown to be expressed primarily in the brain, namely MC3R (Roselli-Rehfuss et *al.,* 1993, *Proc. Natl*. *Acad. Sci. USA* 90:8856-8860; Gantz et *al*., 1993, *J. Biol*. *Chem.* 268:8246-8250) and MC4R (Gantz et *al*., *1993*, *supra;* Mountjoy et *al*., 1994, *Mol. Endo.* 8:1298-1308). A fifth melanocortin receptor, originally called MC2R, is expressed in numerous peripheral organs as well as the brain. Chhajlani *et* al., 1993, *Biochem. Biophys. Res. Commun.* 195:866-873; Gantz et *al*., 1994, *Biochem*. *Biophs*. *Res. Commun.* 200:1214-1220. The native ligands and functions of these latter three receptors remain unknown.

Because of their "orphan" status as receptors without an identified native ligand, and the absence of any known physiological role for these new receptors, investigators have attempted to characterize the receptors *in vitro,* by their ability to bind and respond (*e*.*g*., transduce a signal) to a variety of known melanocortins (*see, e.g.*, Roselli-Rehfuss, 1993, *supra*; and Gantz, 1993, *supra)* or agonists and antagonists derived from MSH and ACTH amino acid sequences (see, *e.g.,* Hruby et *al*., 1995, *J. Med*. *Chem.* 38:3454-3461; and Adan *et al.,* 1994, *Eur. J. Pharmacol.* 269:331-337). In another approach, the members of the melanocortin receptor family were differentiated on the basis of their pattern of tissue distribution as a means for hypothesizing a function. See, e.g., Gantz *et al*., 1993, *supra*; and Mountjoy *et al*., 1994, *supra.* For example, expression of MC1R and MC2R is localized to melanocytes and to adrenal cortical cells, respectively. MC3R and MC4R are expressed primarily in the brain but not in the adrenal cortex or melanocytes. MC4R is *not* expressed in the placenta, a tissue that expresses large amounts of MC3R.

MCR4 is a 7-transmembrane spanning G-protein coupled receptor (hereinafter "GPCR") which upon agonist activation stimulates cAMP accumulation (see U.S. Patent No. 5,869,257; U.S. Patent No. 5,703,220; Alvarro *et al.,* 1996, *Molecular Pharmacology* 50:583-591. To date, MC4R has been isolated from rat (Alvarro et *al*., 1996, *supra*), human (Gantz et *al*., 1993, *supra*), mouse (GenBank Accession No. AB009664), chicken (GenBank Accession No. AB012211) and pig (GenBank Accession No. AB021664). Based upon its expression pattern in the hippocampal region of the brain, a role for the MC4R in learning and memory was proposed (see Gantz, *et al*., 1993, *supra*) but was noted to be a "pharmacological paradox" in that the MC4R does not respond well to compounds known to have an effect on retention of learned behaviors (Mountjoy *et al.,* 1994, *supra*). Mountjoy further suggested that the MC4R may participate in modulating the flow of visual and sensory information, or coordinate aspects of somatomotor control, and/or may participate in the modulation of autonomic outflow to the heart. Mountjoy et *al*., 1994, *supra.*

Evidence now suggests that MC4R is of central importance in the regulation of appetite and energy homeostasis (U.S. Patent No. 5,932,779). First, central administration of MC4R agonists and antagonists can regulate feeding behavior in mice (Fan et *al.,* 1997, *Nature* 385:165-168). Second, agouti is a natural antagonist of MC3R and MC4R, (Moussa and Claycombe, 1999, *Obes. Res.* 7:506-514; Fisher et *al*., 1999, *Int*. *J. Obes. Relat*. *Metab. Disord.* 23 Suppl 1:54-58), where in yellow obese (hereinafter "Avy") mice, adult onset ectopic expression of agouti protein is thought to be responsible for the obese phenotype (Lu *et al*., 1994, *Nature* 371:799-802). Third, MC4R knockout mice develop late onset obesity (Huszar et *al*., 1997, *Cell* 88:131-141). Fourth, MC4R knockout mice are resistant to the anorectic effects of MTII, an MSH-like agonist (Marsh et *al*., 1999, *Nature Genetics* 21:119-122). However, these knockout mice are sensitive to the anorectic effects of ciliary neurotrophic factor (hereinafter "CNTF"), corticotropin releasing factor (hereinafter "CRF"), or urocortin, or the orexigenic actions of NPY or peptide YY (hereinafter "PYY"), indicating that these neuromodulators act either independently or downstream of MC4R (Marsh *et al*., 1999, *supra).* Fifth, evidence is now mounting that leptin's effects on appetite and reproductive functions are mediated, at least in part, by MC4R (Kask *et al.,* 1998, *European Journal of Pharmacology* 360:15-19; Marsh *et al*., 1999, *supra).* Finally, recently it was shown by *in situ* quantitative autoradiography that MC4R, but not MC3R, appears to be upregulated in particular areas of the hypothalamus during underfeeding and downregulated in diet-induced obesity in rats (Harrold *et al*., 1999, *Diabetes* 48:267-271; Harrold *et al*., 1999, *Biochem. Biophys. Res. Commun.* 258:574-577).

Thus, while circumstantial evidence suggests that MC4R might play a role in regulating appetite and metabolic rate in rodents, it is not known what that role is, nor is it known how these observations in rodents may be exploited to treat wasting or obese disorders in companion and agricultural animals. The instant invention addresses the need of clarifying those questions by providing, *e*.*g*., feline and canine MC4R polypeptides and peptides, and nucleic acids encoding the same, methods of using the same to identify agonists and antagonists of MC4R, agonists and antagonists identified by these screens, pharmaceutical compositions containing the agonists and antagonists, and methods of using the agonists, antagonists and compositions to treat appetite and associated disorders.

### Summary of the Invention

The present invention relates to an isolated nucleic acid encoding a functional MC4R, or the complement thereof, said nucleic acid comprising a nucleic selected from the group consisting of:
(a) a nucleotide sequence which hybridizes under conditions of moderate stringency to the coding region of SEQ ID NO:1;
(b) a nucleotide sequence which hybridizes under conditions of moderate stringency to a polynucleotide which is complementary to the coding region of SEQ ID NO:1;
(c) a nucleotide sequence which hybridizes under conditions of moderate stringency to the coding region of the feline MC4R as deposited with the ATCC and having ATCC Accession No. **PTA-1762**; and
(d) a nucleotide sequence which hybridizes under conditions of moderate stringency to a polynucleotide which is complementary to the coding region of the feline MC4R as deposited with the ATCC and having ATCC Accession No. **PTA-1762**, with the proviso that said functional MC4R is not human, porcine, murine, rat or chicken.

The present invention further relates to an isolated nucleic acid encoding a functional MC4R, or the complement thereof, said nucleic acid comprising a nucleic selected from the group consisting of:
(a) a nucleotide sequence which hybridizes under conditions of high stringency to the coding region of SEQ ID NO:1;
(b) a nucleotide sequence which hybridizes under conditions of high stringency to a polynucleotide which is complementary to the coding region of SEQ ID NO:1;
(c) a nucleotide sequence which hybridizes under conditions of high stringency to the coding region of the feline MC4R as deposited with the ATCC and having ATCC Accession No. **PTA-1762;** and
(d) a nucleotide sequence which hybridizes under conditions of high stringency to a polynucleotide which is complementary to the coding region of the feline MC4R as deposited with the ATCC and having ATCC Accession No. **PTA-1762.**

The present invention also relates to an isolated nucleic acid comprising a nucleotide sequence that:
(a) encodes a polypeptide according to SEQ ID NO:3; or
(b) encodes a polypeptide encoded by the feline MC4R clone as deposited with the ATCC and having ATCC Accession No. **PTA-1762.**

A further embodiment of the present invention is an isolated nucleic acid comprising a nucleotide sequence that:
(a) encodes a polypeptide according to SEQ ID NO:3; or
(b) encodes a polypeptide encoded by the feline MC4R clone as deposited with the ATCC and having ATCC Accession No. **PTA-1762**, wherein said nucleic acid has a nucleotide sequence according to SEQ ID NO:1 or the feline MC4R clone as deposited with the ATCC and having ATCC Accession No. **PTA-1762.**

A preferred embodiment of the present invention is an isolated nucleic acid comprising a nucleotide sequence having more than 87.4% identity to SEQ ID NO:1 or the feline MC4R clone as deposited with the ATCC and having ATCC Accession No. **PTA-1762.**

A further preferred embodiment of the present invention is an isolated nucleic acid comprising a nucleotide sequence encoding a polypeptide having more than 98.2% identity to SEQ ID NO:3 or the polypeptide encoded by the feline MC4R clone as deposited with the ATCC and having ATCC Accession No. **PTA-1762.**

Yet another preferred embodiment of the present invention is an isolated nucleic acid comprising a nucleotide sequence encoding an ECD of a feline MC4R corresponding to amino acids 1-46, 96-123, 186-190, or 268-279 of SEQ ID NO:3 or of the polypeptide encoded by the feline MC4R clone as deposited with the ATCC and having ATCC Accession No. **PTA-1762**.

Yet another preferred embodiment of the present invention is an isolated nucleic acid comprising a nucleotide sequence encoding a CD of a feline MC4R corresponding to amino acids 70-76, 146-165, 212-244, or 302-333 of SEQ ID NO:3 or of the polypeptide encoded by the feline MC4R clone as deposited with the ATCC and having ATCC Accession No. **PTA-1762.**

The present invention further relates to an isolated nucleic acid encoding a functional MC4R, or the complement thereof, said nucleic acid comprising a nucleic selected from the group consisting of:
(a) a nucleotide sequence which hybridizes under conditions of moderate stringency to the coding region of SEQ ID NO:2;
(b) a nucleotide sequence which hybridizes under conditions of moderate stringency to a polynucleotide which is complementary to the coding region of SEQ ID NO:2;
(c) a nucleotide sequence which hybridizes under conditions of moderate stringency to the coding region of the canine MC4R as deposited with the ATCC and having ATCC Accession No. **PTA-1762**; and
(d) a nucleotide sequence which hybridizes under conditions of moderate stringency to a polynucleotide which is complementary to the coding region of the canine MC4R as deposited with the ATCC and having ATCC Accession No. **PTA-1761**, with the proviso that said functional MC4R is not human, porcine, murine, rat or chicken.

Another embodiment of the present invention is an isolated nucleic acid encoding a functional MC4R, or the complement thereof, said nucleic acid comprising a nucleic selected from the group consisting of:
(a) a nucleotide sequence which hybridizes under conditions of high stringency to the coding region of SEQ ID NO:2;
(b) a nucleotide sequence which hybridizes under conditions of high stringency to a polynucleotide which is complementary to the coding region of SEQ ID NO:2;
(c) a nucleotide sequence which hybridizes under conditions of high stringency to the coding region of the canine MC4R as deposited with the ATCC and having ATCC Accession No. **PTA-1761**; and
(d) a nucleotide sequence which hybridizes under conditions of high stringency to a polynucleotide which is complementary to the coding region of the canine MC4R as deposited with the ATCC and having ATCC Accession No. **PTA-1761.**

A further embodiment of the present invention is an isolated nucleic acid comprising a nucleotide sequence that:
(a) encodes a polypeptide according to SEQ ID NO:4; or
(b) encodes a polypeptide encoded by the canine MC4R clone as deposited with the ATCC and having ATCC Accession No. **PTA-1761**.

A further preferred embodiment of the present invention is an isolated nucleic acid comprising a nucleotide sequence that:
(a) encodes a polypeptide according to SEQ ID NO:4; or
(b) encodes a polypeptide encoded by the canine MC4R clone as deposited with the ATCC and having ATCC Accession No. **PTA-1761**, wherein said nucleic acid has a nucleotide sequence according to SEQ ID NO:2 or the canine MC4R clone as deposited with the ATCC and having ATCC Accession No. **PTA-1761.**

A preferred embodiment of the present invention is an isolated nucleic acid comprising a nucleotide sequence having more than 81.3% identity to SEQ ID NO:2 or the canine MC4R clone as deposited with the ATCC and having ATCC Accession No. **PTA-1761**.

A further preferred embodiment of the present invention is an isolated nucleic acid comprising a nucleotide sequence encoding a polypeptide having more than 98.1% identity to SEQ ID NO:4 or to the polypeptide encoded by the canine MC4R clone as deposited with the ATCC and having ATCC Accession No. **PTA-1761**.

Yet another preferred embodiment of the present invention is an isolated nucleic acid comprising a nucleotide sequence encoding an ECD of a canine MC4R corresponding to amino acids 1-46, 98-124, 187-191, or 268-279 of SEQ ID NO:4 or of the polypeptide encoded by the canine MC4R clone as deposited with the ATCC and having ATCC Accession No. **PTA-1761**.

A further preferred embodiment of the present invention is an isolated nucleic acid comprising a nucleotide sequence encoding a CD of a canine MC4R corresponding to amino acids 69-77, 147-163, 216-244, or 302-333 of SEQ ID NO:4 or of the polypeptide encoded by the canine MC4R clone as deposited with the ATCC and having ATCC Accession No. **PTA-1761.**

Another aspect of the present invention relates to a nucleotide vector comprising the novel nucleic acids disclosed herein. Such novel nucleic acids includes those nucleic acids disclosed above and described in claims 1, 2, 3, 4, 5, 6, 7, 8, 9, 10, 11, 12, 13 14, 15 and 16.

This invention further relates to an expression vector comprising such novel nucleic acids in operative association with a nucleotide regulatory element that controls expression of the polypeptide encoded by said nucleotide sequence.

Another aspect of the present invention relates to a genetically engineered host cell comprising the novel nucleic acids described herein.

Yet another aspect of the present invention is a genetically engineered host cell comprising the novel nucleic acids described herein, wherein said nucleic acid is in operative association with a nucleotide regulatory element that controls expression of said nucleotide sequence in the host cell.

The present invention further relates to a substantially pure polypeptide encoded by the novel nucleic acids described herein.

Preferred embodiments of the present invention include a substantially pure polypeptide comprising the amino acid sequence of:
(a) SEQ ID NO:3;
(b) SEQ ID NO:4;
(c) the feline MC4R clone as deposited with the ATCC and having the ATCC Accession NO. **PTA-1762**;
(d) the canine MC4R clone as deposited with the ATCC and having the ATCC Accession NO. **PTA-1761**;
(e) an ECD of a feline MC4R corresponding to amino acids 1-46, 96-123, 186-190, or 268-279 of SEQ ID NO:3 or of the polypeptide encoded by the feline MC4R clone as deposited with the ATCC and having ATCC Accession No. **PTA-1762**;
(f) an ECD of a canine MC4R corresponding to amino acids 1-46, 98-124, 187-191, or 268-279 of SEQ ID NO:4 or of the polypeptide encoded by the canine MC4R clone as deposited with the ATCC and having ATCC Accession No. **PTA-1761**;
(g) a CD of a feline MC4R corresponding to amino acids 70-76, 146-165, 212-244, or 302-333 of SEQ ID NO:3 or of the polypeptide encoded by the feline MC4R clone as deposited with the ATCC and having ATCC Accession No. **PTA-1762**; or
(h) a CD of a canine MC4R corresponding to amino acids 69-77, 147-163, 216-244, or 302-333 of SEQ ID NO:4 or of the polypeptide encoded by the canine MC4R clone as deposited with the ATCC and having ATCC Accession No. **PTA-1761**.

Yet another aspect of the present invention relates to an antibody that immunospecifically binds a substantially pure polypeptide encoded by the novel nucleic acids described herein.

This invention further relates to a method for producing a recombinant polypeptide, comprising:
(a) culturing a host cell transformed with the expression vector of Claim 16 and which expresses the recombinant polypeptide; and
(b) recovering the recombinant polypeptide from the cell culture.

This invention further relates to a composition comprising a substantially pure polypeptide encoded by the novel nucleic acids described herein and a carrier.

Another aspect of this invention relates to a method for detecting a polynucleotide comprising a novel nucleic acid described herein, in a sample, comprising:
(a) contacting the sample with a compound that binds to and forms a complex with the polynucleotide for a period sufficient to form the complex; and
(b) detecting the complex,
   so that if a complex is detected, a polynucleotide described is detected.

This invention further relates to a method for detecting such a polynucleotide in a sample, comprising:
(a) contacting the sample under stringent hybridization conditions with nucleic acid primers that anneal to a polynucleotide described herein under such conditions; and
b) amplifying the annealed polynucleotides,
   so that if a polynucleotide is amplified, the described herein is detected.

In a preferred embodiment the polynucleotide is an RNA molecule that encodes a functional MC4R, and the method further comprises reverse transcribing an annealed RNA molecule into a cDNA polynucleotide.

Another aspect of the present invention relates to a method for identifying a compound that binds to a substantially pure polypeptide encoded by the novel nucleic acids described herein, comprising:
(a) contacting a compound with the polypeptide for a time sufficient to form a polypeptide/compound complex; and
b) detecting the complex,
   so that if a polypeptide/compound complex is detected, a compound that binds to the polypeptide is identified.

This invention further relates to a method for identifying a compound that binds to a substantially pure polypeptide encoded by the novel nucleic acids described herein comprising:
(a) contacting a compound with the polypeptide in a cell, for a time sufficient to form a polypeptide/compound complex, wherein the complex drives expression of a reporter gene sequence in said cell; and
b) detecting the complex by detecting reporter gene sequence expression, so that if a polypeptide/compound complex is detected, a compound that binds to the polypeptide is identified.

This invention further relates to a method of modulating activity of a substantially pure polypeptide encoded by the novel nucleic acids described herein comprising contacting a cell that expresses the polypeptide with a compound that modulates activity of the polypeptide for a time sufficient to modulate said activity.

This invention further relates to a method for screening and identifying antagonists of MC4R, comprising:
(a) contacting a cell line that expresses a substantially pure polypeptide encoded by the nucleic acids described herein with a test compound in the presence of an MC4R agonist; and
(b) determining whether the test compound inhibits the binding and cellular effects of the MC4R agonist on the cell line,

in which antagonists are identified as those compounds that inhibit both the binding and cellular effects of the MC4R agonist on the cell line.

This invention further relates to a method for screening and identifying agonists of MC4R, comprising:
(a) contacting a cell line that expresses a substantially pure polypeptide encoded by the novel nucleic acids described herein with a test compound in the presence and in the absence of an MC4R agonist;
(b) determining whether, in the presence of the MC4R agonist, the test compound inhibits the binding of the MC4R agonist to the cell line; and
(c) determining whether, in the absence of the MC4R agonist, the test compound mimics the cellular effects of the MC4R agonist on the cell line,
   in which agonists are identified as those test compounds that inhibit the binding but mimic the cellular effects of the MC4R agonist on the cell line.

In a preferred embodiment the cell line is a genetically engineered cell line.

This invention further relates to a method for screening and identifying antagonists of MC4R comprising:
(a) contacting a substantially pure polypeptide encoded by the novel nucleic acids described herein with a random peptide library such that the polypeptide will recognize and bind to one or more peptide species within the library;
(b) isolating the polypeptide/peptide combination;
(c) determining the sequence of the peptide isolated in step (b); and
(d) determining whether the test compound inhibits the binding and cellular effects of an MC4R agonist,
   in which antagonists are identified as those peptides that inhibit both the binding and cellular effects of the MC4R agonist.

This invention further relates to a method for screening and identifying agonists of MC4R comprising:
(a) contacting a substantially pure polypeptide encoded by the novel nucleic acids described herein with a random peptide library such that the polypeptide will recognize and bind to one or more peptide species within the library;
(b) isolating a polypeptide/peptide combination;
(c) determining the sequence of the peptide isolated in step (b); and
(d) determining whether, in the absence of a MC4R agonist, the peptide mimics the cellular effects of the MC4R agonist, in which agonists are identified as those peptides that inhibit the binding of the MC4R agonist to a MC4R polypeptide but mimic the cellular effects of the MC4R agonist.

This invention further relates to a method of modulating activity of a substantially pure polypeptide encoded by the novel nucleic acids described herein, comprising contacting the polypeptide with a compound that modulates activity of the polypeptide for a time sufficient to modulate said activity.

This invention further relates to a method of modulating the endogenous enzymatic activity of MC4R in an animal comprising administering to the animal an amount of an MC4R ligand effective to modulate said endogenous enzymatic activity.

In a preferred embodiment, the animal is a cow, a pig, a goat, a sheep, a horse, a dog, or a cat.

In a further preferred embodiment, the ligand to said MC4R receptor is an MC4R agonist.

In another preferred embodiment, the ligand to said MC4R receptor is an MC4R antagonist.

In a preferred embodiment, the antagonist is a monoclonal antibody that immuno specifically binds to an epitope of said MC4R.

In yet another preferred embodiment, the enzymatic activity of said MC4R is increased or decreased.

Yet another aspect of the present invention relates to a transgenic animal in which a nucleic acid, comprising an isolated nucleic acid encoding a functional MC4R, or the complement thereof, said nucleic acid comprising a nucleic selected from the group consisting of:
(a) a nucleotide sequence which hybridizes under conditions of moderate stringency to the coding region of SEQ ID NO:1;
(b) a nucleotide sequence which hybridizes under conditions of moderate stringency to a polynucleotide which is complementary to the coding region of SEQ ID NO:1;
(c) a nucleotide sequence which hybridizes under conditions of moderate stringency to the coding region of the feline MC4R as deposited with the ATCC and having ATCC Accession No. **PCT-1762**; and
(d) a nucleotide sequence which hybridizes under conditions of moderate stringency to a polynucleotide which is complementary to the coding region of the feline MC4R as deposited with the ATCC and having ATCC Accession No. **PTA-1762**, with the proviso that said functional MC4R is not human, porcine, murine, rat or chicken, or an isolated nucleic acid encoding a functional MC4R, or the complement thereof, said nucleic acid comprising a nucleic selected from the group consisting of:

(a) a nucleotide sequence which hybridizes under conditions of moderate stringency to the coding region of SEQ ID NO:2;
(b) a nucleotide sequence which hybridizes under conditions of moderate stringency to a polynucleotide which is complementary to the coding region of SEQ ID NO:2;
(c) a nucleotide sequence which hybridizes under conditions of moderate stringency to the coding region of the canine MC4R as deposited with the ATCC and having ATCC Accession No. **PTA-1761**; and
(d) a nucleotide sequence which hybridizes under conditions of moderate stringency to a polynucleotide which is complementary to the coding region of the canine MC4R as deposited with the ATCC and having ATCC Accession No. **PTA-1761**, with the proviso that said functional MC4R is not human, porcine, murine, rat or chicken, is an expressed transgene contained in the genome of the animal.

This invention further relates to a transgenic animal in which expression of genomic sequences encoding substantially pure polypeptides encoded by the novel nucleic acids described herein, is prevented or repressed.

This invention further relates to a method for modulating the appetite and/or metabolic rate of an animal comprising administering to the animal an effective amount of an MC4R ligand.

In a preferred embodiment, the animal has an appetite-related or metabolic disorder.

In a further preferred embodiment, the disorder causes, is caused by, or is characterized by a reduction in appetite, feeding behavior or body weight, or an increase in metabolic rate, and the ligand is an MC4R antagonist.

In yet another preferred embodiment, the disorder causes, is caused by, or is characterized by an increase in appetite, feeding behavior, or body weight, or a decrease in metabolic rate, and the ligand is an MC4R agonist.

In another embodiment, the animal is a *post partum* sow or dairy cow.

In another embodiment, the animal is a companion animal.

In another embodiment, the animal is a livestock animal.

In another embodiment, the animal is a poultry animal.

In another embodiment, the animal suffers from shipping or crowding stress.

In another embodiment, the animal is lactating.

In another embodiment, the animal is gravid.

In another embodiment, the disorder is cachexia, anorexia or weaning-induced inappetence and growth lag.

In another embodiment, the disorder is a metabolic disorder.

In another embodiment, the metabolic disorder is diabetes.

In another embodiment, the disorder is a disease.

In another embodiment, the disease is cancer, renal failure, cardiac disease, endotoxemia, fever, hepatic lipidosis, infection or inflammation.

In another embodiment, wherein the animal is obese.

In another embodiment, the MC4R ligand is part of a pharmaceutical composition.

This invention further relates to a method of increasing the reproductive performance of an animal comprising administering to the animal an effective amount of an MC4R ligand.

This invention further relates to a method of increasing the growth performance of an animal comprising administering to the animal an effective amount of an MC4R ligand.

This invention further relates to a method for modulating the appetite and/or metabolic rate of an animal comprising administering to the animal an effective amount of an MC4R ligand, wherein the ligand is part of a pharmaceutical composition.

In a preferred embodiment, the pharmaceutical composition is administered orally.

This invention further relates to a kit comprising a pharmaceutical composition comprising an MC4R ligand.

### Description of the Figures

Figure 1 depicts the nucleotide sequence of the feline MC4R (SEQ ID NO: 1)
Figure 2 depicts the nucleotide sequence of the canine MC4R (SEQ ID NO: 2)
Figure 3 depicts the polypeptide sequence of the feline MC4R (SEQ ID NO: 3)
Figure 4 depicts the polypeptide sequence of the canine MC4R (SEQ ID NO: 4)
Figure 5 is a graph depicting the specific binding of radiolabeled NDP-MSH ligand to HEK293 cells expressing human MC4R (hMC4R), canine MC4R (cMC4R) and feline MC4R (fMC4R).
Figure 6 is a graph depicting the specific binding of radiolabeled NDP-MSH ligand to various cell clones which stably express hMC4R, cMC4R or fMC4R.
Figure 7 is a graph depicting the specific binding of ¹²⁵I-NDP-MSH to whole cells expressing MC3R and MC4R as a function of cell number.
Figures 8A, 8B and 8C are graphs depicting the specific binding of ¹²⁵I-NDP-MSH to MC3R and MC4R in membrane fractions as a function of ligand concentration, and corresponding Scatchard plots.
Figures 9A, 9B, 9C and 9D are a description and graphs depicting the competitive displacement of ¹²⁵I-NDP-MSH from MC3R and MC4R containing membranes by various ligands.
Figure 10 is a graph depicting cAMP accumulation in the HEK293 cells following stimulation by α-MSH as functional validation of the cMC4R clone.
Figures 11A and 11B are graphs depicting the results of FLIPR® assays for cells transfected with cMC4R, and Gα15-16 and treated with agonists NDP-MSH, α-MSH, or MTII, or NDP-MSH and antagonist SHU9119.
Figures 12A, 12B, 12C is the nucleotide sequence of the coding strand of the gene encoding G-alpha 15 (SEQ ID NO: 5)
Figures 13A, 13B, 13C is the nucleotide sequence of the coding strand of the gene encoding G-alpha 16 (SEQ ID NO: 6).

### Detailed Description of the Invention

### General Overview Of The Invention

The present invention relates to novel nucleic acids encoding melanocortin 4 receptors (hereinafter "MC4R") and their gene products. In particular, the present invention provides nucleic acids encoding feline and canine MC4R polypeptides and their gene products. The present invention also relates to screening assays to identify compounds that modulate the activity or expression of the novel MC4R of the invention. In particular, the present invention provides *in vitro* assays for MC4R-binding compounds using MC4R-transfected cell lines. The MC4R-transfected cell lines may further comprise a reporter gene whose level of expression is regulated by MC4R. The present invention further relates to pharmaceutical compositions that modulate the activity or expression of MC4R. In particular, the pharmaceutical compositions may be agonists or antagonists of MC4R. Antagonists may act, *e.g.*, by competitively inhibiting another MC4R agonist or antagonist, by blocking the interaction of activated MC4R with its downstream signaling pathway, by inhibiting transcription of the MC4R gene, or by inhibiting processing or translation of the MC4R mRNA. Agonists may act by activating and/or enhancing the natural biological effects of the MC4R signal transduction response or its expression. In yet another aspect, the present invention relates to methods of treating appetite-related disorders in companion animals, livestock and poultry, comprising administering pharmaceutical formulations which modulate MC4R expression or activity. In particular, pharmaceutical compositions that enhance appetite and reproductive performance, or maintenance or recovery of body weight in sick or stressed animals, or that reduce appetite and ameliorate obesity, may be administered to companion animals, livestock and poultry.

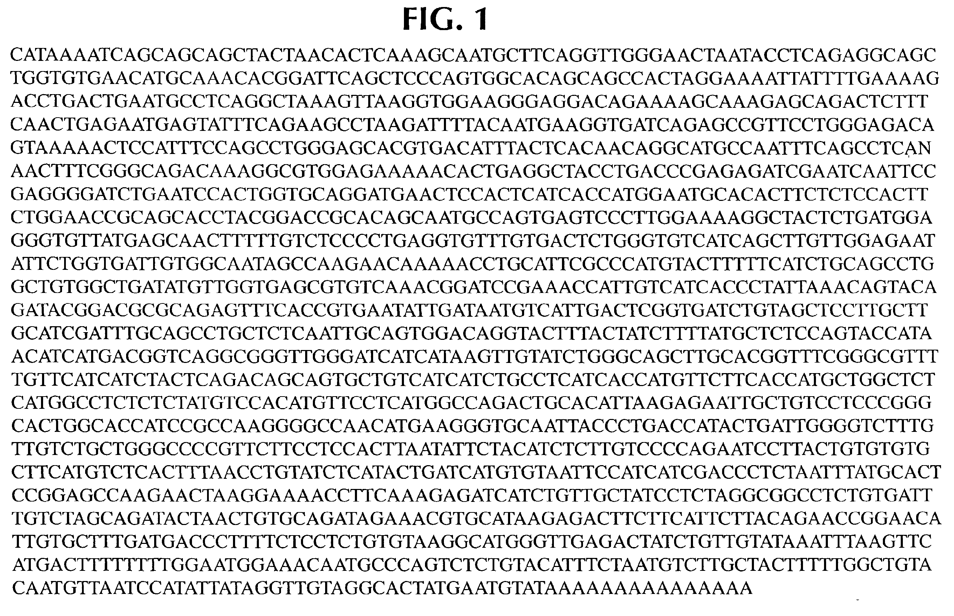

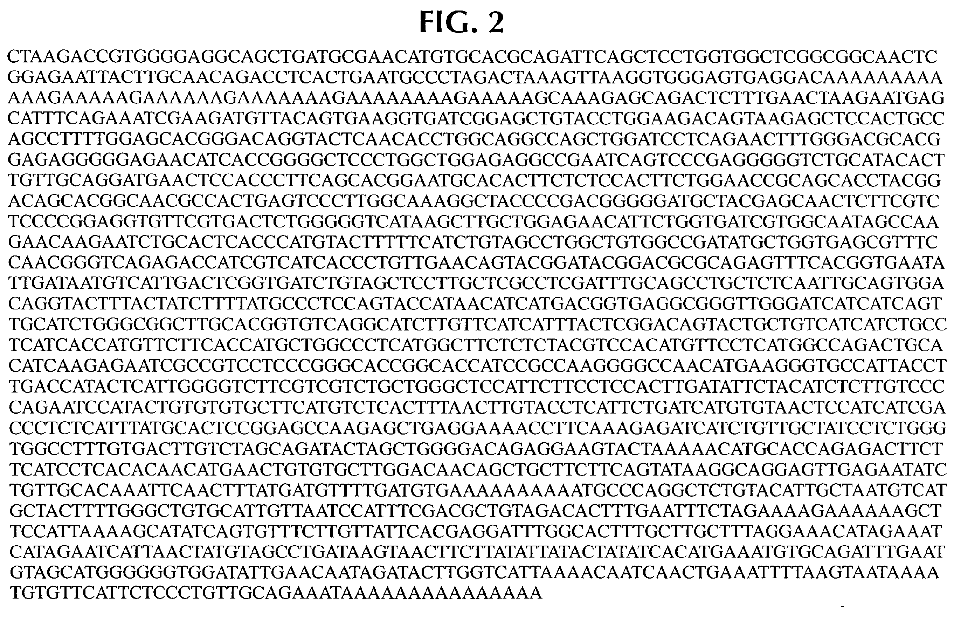

The present invention is based, in part, on the discovery of novel MC4R nucleic acids that encode the canine and feline forms of MC4R. The present invention encompasses: (a) nucleic acids encoding canine MC4R (as shown in Figure 1, SEQ ID NO:1); (b) nucleic acids encoding feline MC4R (as shown in Figure 2, SEQ. ID. NO:2); (c) mutations, truncations or fragments thereof; (d) recombinant proteins, peptides, fragments or derivatives thereof comprising canine MC4R or feline MC4R; (e) recombinant proteins, peptides, fragments or derivatives thereof of MC4R variants of the present invention; and (f) recombinant fusion proteins, peptides, fragments or derivatives thereof comprising canine or feline MC4R. Altered nucleic acids which may be used in accordance with the invention include deletions, additions or substitutions of different nucleotide residues resulting in a nucleic acid that encodes the same or a functionally equivalent gene product. The gene product itself may contain deletions, additions or substitutions of amino acid residues within the MC4R polypeptide, which result in a functionally equivalent MC4R. Such amino acid substitutions may be made on the basis of similarity in polarity, charge, solubility, hydrophobicity, hydrophilicity, and/or the amphipathic nature of the residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, alanine; asparagine, glutamine; serine, threonine; phenylalanine, tyrosine. As used herein, a functionally equivalent MC4R refers to a receptor which binds to MC4R ligand or ligand analogs, but not necessarily with the same binding affinity of its counterpart native MC4R. In addition, any nucleic acid that selectively hybridizes under highly stringent conditions to the complement of SEQ ID NO:1 or SEQ ID NO:2, and is capable of binding α-MSH (Sigma, St. Louis, MO; BACHEM Bioscience Inc., King of Prussia, PA), [Nle₄,D-Phe₇]-α-MSH (hereinafter "NDP-MSH"; Sigma, BACHEM, or NEN®, Boston MA), MTII (BACHEM; a melanocortin receptor agonist (Bednarek *et al*., 1999, Biochem. & Biophys. Res. Comm. 261:209-13)) or SHU9119 (BACHEM; a melanocortin receptor antagonist), is a subject of the instant invention, with the proviso that the nucleic acid does not encode human, porcine, rat, mouse or chicken MC4R.

Unless otherwise indicated, all nucleic acid sequences are written 5' to 3', and all polypeptide and protein sequences are written N-terminal to C-terminal.

### Isolation And Characterization Of The Nucleic Acids And Polypeptides Of The Invention

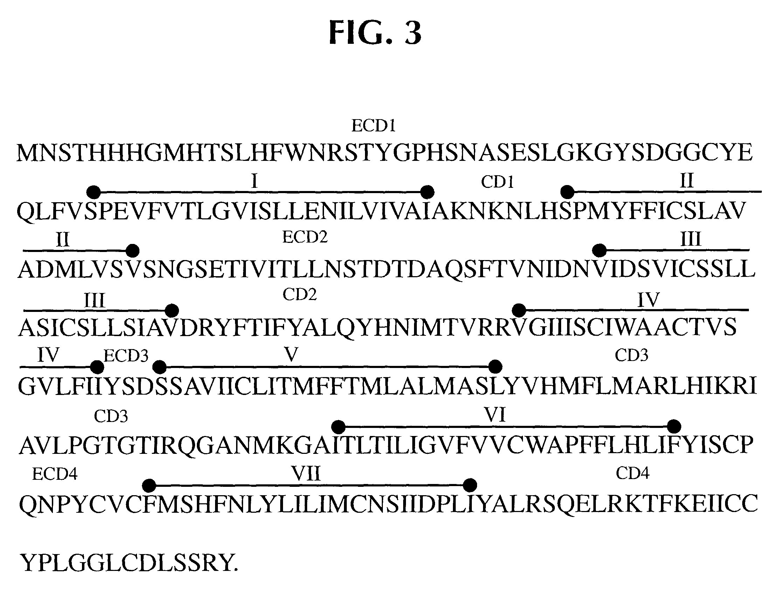

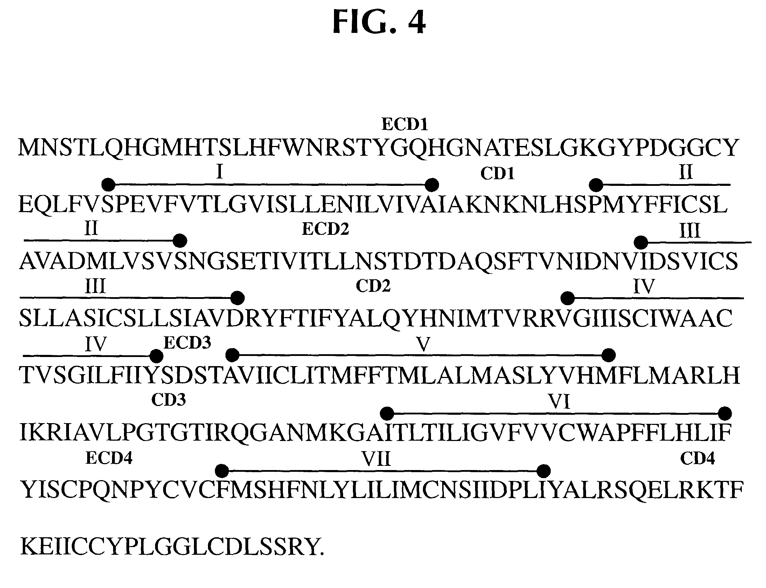

The feline and canine derived nucleic acid sequences (SEQ ID NOS:1 and 2, respectively) encoding the deduced amino acid sequences of the feline (SEQ ID NO:3) and canine (SEQ ID NO:4) MC4R proteins, are shown in Figures 1, 2, 3, and 4, respectively. Predicted transmembrane (hereinafter "TM") domains are indicated by overbars and Roman numerals, and the four extracellular domains and the four cytoplasmic domains are denoted by ECD1-4 and CD1-4, respectively. Figures 3 and 4 are described as a standard single letter amino acid sequence. Roman numerals above sequence dictate transmembrane areas. ECD = extra cellular domain, CD = cytoplasmic domain.

The serpentine structure of the melanocortin receptors suggests that the hydrophilic domains located between the TM domains are arranged alternately outside and within the cell to form the ECDs (in the feline sequence, amino acid residues 1-46, 96-123, 186-190 and 268-279 in Figure 3; in the canine sequence, amino acid residues 1-46, 98-124, 187-191 and 268-279 in Figure 4) and the CDs (in the feline sequence, amino acid residues 70-76, 146-165, 212-244 and 302-333 in Figure 3; in the canine sequence, amino acid residues 69-77, 147-163, 216-244 and 302-333 in Figure 4) of the receptor.

In a specific embodiment described herein, the feline and canine MC4R genes were isolated by stringently hybridizing a specific non-degenerate oligonucleotide (ttgactctgtgatctgtagctccttgct) tagged with biotin to clones in a feline and a canine cDNA library (each library was constructed by LifeTechnologies (Rockville, MD) using its SUPERSCRIPT™ cDNA library construction technology). The complex was separated from all other clones in the library using Streptaviden magnetic beads which were pelleted *via* a magnet. MC4R clones were identified by PCR using MC4R specific primer pairs designed to flank the capture oligonucleotide site (forward primer: atgaggcagatgatgacagc; reverse primer: gtgatctgtagctccttgc). Six feline and one canine MC4R clones were isolated from the cDNA libraries. Of these, one feline clone and one canine clone were deposited with the ATCC (under deposit numbers **PTA-1762** and **PTA-1761,** respectively). The identity of the clones was confirmed by PCR using different MC4R gene-specific primer pair (forward primer: tgagacatgaagcacac; reverse primer: gtgatctgtagctccttgc). Sequencing of the MC4R genes was completed using one standard and three primer walking reactions from both ends of each clone. Sequences of the MC4R gene fragments have been assembled based on their overlapping regions. Conceptual translation of the open reading frames within each clone revealed that each comprised an MC4R-encoding nucleic acid. The open reading frame for the canine MC4R gene comprises nucleotides 447-1445 (Figure 2). The open reading frame for the feline MC4R gene comprises nucleotides 451-1449 (Figure 1).

The invention also relates to nucleic acids encoding MC4R variants and MC4R nucleic acids isolated from other animals in which MC4R activity exists, with the proviso that the other animal is not human, pig, rat, mouse, or chicken. In one embodiment, the MC4R nucleic acid is derived from a mammal, including but not limited to cow, horse, goat, sheep, elk, deer, bison, buffalo, ox, ape, monkey and lemur. In another embodiment, the MC4R nucleic acid is derived from a bird, including but not limited to goose, ostrich, emu, turkey and duck. In again another embodiment, the MC4R nucleic acid is derived from a fish, including salmon, carp and trout. Preferably, the nucleic acids of the present invention are more than 87.4% identical to the feline MC4R gene sequence, or greater than 81.3% identical to the canine MC4R gene sequence using the LASERGENE-MEGALIGN™ software package (DNASTAR, Inc., Madison, WI; percent similarity compares sequences directly, without accounting for phylogenetic relationships). Alternatively, the preferred polypeptides of the present invention are more than 98.2% identical to the feline MC4R protein sequence, or greater than 98.1% identical to the canine MC4R protein sequence, also using the LASERGENE-MEGALIGN™ software package. In another preferred embodiment, the polypeptides of the invention, and the gene products encoded by the nucleic acids of the invention, are a functional MC4R that is able to bind to and be activated by α-MSH, NDP-MSH, MTII or SHU9119 under physiological conditions.

Typically, a nucleic acid of the invention is capable of hybridizing to the feline or canine MC4R nucleic acid under stringent conditions. The term "stringent" is used to refer to conditions that are commonly understood in the art as stringent. In a preferred embodiment, the nucleic acids of the invention hybridize to the feline and canine MC4R encoding polynucleotides disclosed herein under highly stringent conditions. Procedures using such conditions of high stringency are as follows: Prehybridization of filters containing DNA is carried out for 8 h to overnight at 65°C in buffer composed of 6X SSC, 50 mM Tris-HCI (pH 7.5), 1 mM EDTA, 0.02% PVP, 0.02% Ficoll, 0.02% BSA, and 500 µg/ml denatured salmon sperm DNA. Filters are hybridized for 48 h at 65°C in prehybridization mixture containing 100 µg/ml denatured salmon sperm DNA and 5-20 X 10⁶ cpm of ³²P-labeled probe. Washing of filters is done at 37°C for 1 h in a solution containing 2X SSC, 0.01% PVP, 0.01% Ficoll, and 0.01% BSA. This is followed by a wash in 0.1X SSC at 50°C for 45 min before autoradiography. Other conditions of high stringency which may be used are well known in the art.

In another embodiment, the nucleic acids of the invention hybridize to the feline or canine MC4R-encoding polynucleotides disclosed herein under moderate stringency conditions. Procedures using such conditions of moderate stringency are as follows: Filters containing DNA are pretreated for 6 h at 55EC in a solution containing 6X SSC, 5X Denhart's solution, 0.5% SDS and 100 µg/ml denatured salmon sperm DNA. Hybridizations are carried out in the same solution and 5-20 X 10⁶ cpm ³²P-labeled probe is used. Filters are incubated in hybridization mixture for 18-20 h at 55°C, and then washed twice for 30 minutes at 60°C in a solution containing 1X SSC and 0.1% SDS. Filters are blotted dry and exposed for autoradiography. Other conditions of moderate stringency which may be used are well-known in the art. Washing of filters is done at 37°C for 1 h in a solution containing 2X SSC, 0.1% SDS.

In another embodiment, the nucleic acids of the invention include those that hybridize to the feline and canine MC4R-encoding polynculeotides disclosed herein under conditions of low stringency. Procedures using such conditions of low stringency are as follows (see *also,* Shilo and Weinberg, 1981, *Proc*. *Natl*. *Acad. Sci.* USA 78:6789-6792): Filters containing DNA are pretreated for 6 h at 40°C in a solution containing 35% formamide, 5X SSC, 50 mM Tris-HCI (pH 7.5), 5mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20 X 10⁶ cpm ³²P-labeled probe is used. Filters are incubated in hybridization mixture for 18-20 h at 40°C, and then washed for 1.5 h at 55°C in a solution containing 2X SSC, 25 mM Tris-HCI (pH 7.4), 5 mM EDTA, and 0.1% SDS. The wash solution is replaced with fresh solution and incubated an additional 1.5 h at 60°C. Filters are blotted dry and exposed for autoradiography. If necessary, filters are washed for a third time at 65-68°C and re-exposed to film. Other conditions of low stringency which may be used are well known in the art (*e.g.*, as employed for cross-species hybridizations).

The nucleic acids of the invention may be isolated by screening of a genomic or cDNA library, *e.g.*, a bacteriophage cDNA library, under conditions of reduced stringency, using a radioactively labeled fragment of a feline or canine MC4R clone. Alternatively, a feline or canine MC4R sequence may be used to design degenerate or fully degenerate oligonucleotide probes which may be used as PCR probes or to screen bacteriophage cDNA libraries. In another alternative, a polymerase chain reaction (PCR) based strategy may be used to clone nucleic acids encoding MC4R variants or MC4R genes from other species. Two pools of degenerate oligonucleotides, corresponding to a conserved motif between the feline and canine MC4R cDNA sequences, may be designed to serve as primers in a PCR reaction. The template for the reaction is cDNA obtained by reverse transcription of mRNA prepared from cell lines or tissue known to express MC4R. The PCR product may be subcloned and sequenced to insure that the amplified sequences represent the desired MC4R sequences. The PCR fragment may be used to isolate a full length MC4R cDNA clone by radioactively labeling the amplified fragment and screening a bacteriophage cDNA library. Alternatively, the labeled fragment may be used to screen a genomic library. For a review of cloning strategies which may be used, see *e*.*g*., Sambrook *et al*., 1989, Molecular Cloning, A Laboratory Manual, 2d ed., Cold Springs Harbor Press, N.Y.; and Ausubel *et al*., 1999, Current Protocols in Molecular Biology, (Green Publishing Associates and Wiley Interscience, N.Y.)

Alternatively, isolation of an MC4R cDNA of the invention also may be achieved by construction of a cDNA library in a mammalian expression vector such as pcDNA1, that contains SV40 origin of replication sequences which permit high copy number expression of plasmids when transferred into COS cells. The expression of MC4R on the surface of transfected COS cells may be detected in a number of ways, including the use of a labeled ligand such as MSH or a MSH agonist labeled with a radiolabel, fluorescent label or an enzyme. Cells expressing MC4R may be enriched by subjecting transfected cells to a FACS (fluorescent activated cell sorter) sort.

Of course, in addition to the exemplary methods and procedures outlined above for the isolation of the MC4R encoding polynucleotides of the invention, any other method of isolating a cDNA or genomic DNA known in the art may also be used. *See, e*.*g*., Sambrook *et al*., *supra* and Ausubel *et al*., *supra.*

### Expression Of The Nucleic Acids Of The Invention

In accordance with the invention, an MC4R nucleic acid that encodes an MC4R polypeptide, a peptide fragment of an MC4R polypeptide, an MC4R fusion protein or functional equivalent thereof may be used to generate a recombinant nucleic acid molecule that directs the expression of an MC4R polypeptide or a functional equivalent thereof, in an appropriate host cell line. Alternatively, a nucleic acid that hybridizes to one or more portions of an MC4R nucleic acid also may be used in a nucleic acid hybridization assay, Southern or Northern blot analysis, etc. Of course, related nucleic acids that encode substantially the same or a functionally equivalent polypeptide, may be used in the practice of the invention for the cloning and expression of the MC4R polypeptide.

A nucleic acid of the invention may be engineered in order to alter the MC4R coding sequence for a variety of ends including, but not limited to, an alteration that modifies processing and expression of the gene product. For example, a mutation may be introduced using techniques, which are well known in the art, *e*.*g*. site-directed mutagenesis, to insert a new restriction site, to alter the glycosylation pattern, to alter the phosphorylation pattern, etc. For example, in certain expression systems such as yeast, a host cell line may over glycosylate the gene product. When using such an expression system it may be preferable to alter the MC4R coding sequence to eliminate all of the MC4R gene product's N-linked glycosylation sites.

In another embodiment of the invention, the MC4R nucleic acid or a modified MC4R nucleic acid may be ligated to a heterologous sequence to encode a fusion protein. For example, for screening of peptide libraries it may be advantageous to use a chimeric MC4R protein comprising a heterologous epitope that is recognized by a commercially available antibody. A fusion protein also may be engineered to contain a cleavage site located between the MC4R polypeptide and the heterologous polypeptide, so that the MC4R polypeptide can be cleaved away from the heterologous polypeptide.

In an alternate embodiment of the invention, an MC4R nucleic acid may be synthesized in whole or in part, using chemical methods well-known in the art (*see, e.g.*, Caruthers, *et al*., 1980, *Nuc. Acids Res*. *Symp*. *Ser.* 7:215-233; Crea and Horn, 1980, *Nuc. Acids Res.* 9:2331; Matteucci and Caruthers, 1980, *Tetrahedron Letters* 21:719; Chow and Kempe, 1981, *Nuc*. *Acids Res.* 9:2807-2817). Alternatively, an MC4R polypeptide could be produced in whole or in part using chemical methods. For example, the MC4R polypeptide may be synthesized by solid phase techniques, cleaved from the resin, and purified by preparative high performance liquid chromatography (*see*, *e*.*g*., Creighton, 1983, *Proteins Structures And Molecular Principles,* W.H. Freeman and Co., N.Y. pp. 50-60). The composition of the synthetic polypeptide may be confirmed by amino acid analysis or sequencing (*e*.*g*., the Edman degradation procedure; *see,* Creighton, 1983, *Proteins, Structures and Molecular Principles,* W.H. Freeman and Co., N.Y., pp. 34-49).

In order to express a biologically active MC4R, an MC4R-encoding nucleic acid, or a functional equivalent thereof (as described above) is inserted into an appropriate expression vector (*i*.*e*., a vector which contains the necessary elements for the transcription and translation of the inserted coding sequence) to create an MC4R-comprising vector. An appropriate host cell line (*i*.*e*., a cell line that will allow for expression of an MC4R gene product from the MC4R-encoding nucleic acid) is transformed with the MC4R-comprising vector. The MC4R gene product and the transformed cell line may be used for a variety of purposes. These purposes include but are not limited to generating an antibody *(i.e.,* monoclonal or polyclonal) that binds to the MC4R gene product. The antibody may, *e*.*g*., competitively inhibit binding of ligand to an MC4R receptor (*see*, *infra),* or be used for screening and selecting ligands or drugs that act *via* an MC4R receptor (*see*, *infra);* etc.

Methods that are well known to those skilled in the art may be used to construct expression vectors containing an MC4R nucleic acid and appropriate transcriptional/translational control signals. These methods include *in vitro* recombinant DNA techniques, synthetic techniques and *in vivo* genetic recombination. *See, e*.*g*., the techniques described in Sambrook *et al.,supra;* Ausubel *et al*., *supra.*

A variety of host-expression vector systems may be utilized to express an MC4R nucleic acid. These systems include, but are not limited to, a strain of microorganism (*e*.*g*., a strain of bacterium) transformed with a recombinant bacteriophage DNA, plasmid DNA or cosmid DNA expression vector comprising the MC4R nucleic acid; a strain of yeast (*e.g.*, a strain of *S. cerevisiae* or *S. pombe)* transformed with a recombinant yeast expression vector comprising the MC4R nucleic acid; an insect cell line infected with a recombinant virus expression vector (*e*.*g*., a *baculovirus* derived expression vector) comprising the MC4R nucleic acid; a plant cell line infected with a recombinant virus expression vector (*e*.*g*., a cauliflower mosaic virus (hereinafter "CaMV") or tobacco mosaic virus (hereinafter "TMV") derived expression vector), or transformed with a recombinant plasmid expression vector (*e*.*g*., a Ti plasmid derived expression vector) containing the MC4R nucleic acid; an animal cell system infected with a recombinant virus expression vector (*e*.*g*., an adenovirus or vaccinia virus derived expression vector), or an animal cell line engineered to contain multiple copies of the MC4R nucleic acid, either stably amplified (*e*.*g*., using methotrexate selection of a *dhfr* CHO cell line transformed with an expression vector comprising the MC4R nucleic acid and a DHFR gene) or unstably amplified in double-minute chromosomes (e.g., murine cell lines).

In a mammalian host cell line, a number of viral based expression systems may be utilized. In cases where an adenovirus is used as an expression vector, the MC4R nucleic acid may be ligated to an adenovirus transcription/translation control complex (*e.g.*, the late promoter and tripartite leader sequence) to produce a chimeric gene. The chimeric gene then may be inserted in the adenovirus genome by *in vitro* or *in vivo* recombination. Insertion in a non-essential region of the viral genome (*e*.*g*., region E1 or E3) will result in a recombinant virus that is viable and capable of expressing the MC4R nucleic acid in an infected host (*see*, *e*.*g*., Logan & Shenk, 1984, *Proc. Natl. Acad. Sci. USA* 81:3655-3659). Alternatively, the vaccinia 7.5K promoter may be used (see, *e*.*g*., Mackett et *al*., 1982, *Proc. Natl. Acad. Sci. USA* 79:7415-7419; Mackett *et al*., 1984, *J. Virol.* 49:857-864; Panicali et *al*., 1982, *Proc. Natl. Acad. Sci. USA* 79:4927-4931).

One or more specific initiation signals also may be required for efficient translation of the MC4R nucleic acid. These signals include an ATG initiation codon and adjacent sequences. In cases where the MC4R nucleic acid comprises an entire MC4R gene, including its own initiation codon and adjacent sequences, no additional translational control signals may be needed. However, in cases where the MC4R nucleic acid does not comprise an MC4R gene's own initiation codon and adjacent sequences, one or more exogenous translational control signals, including the ATG initiation codon, must be provided. Furthermore, the initiation codon must be in phase with the reading frame of the MC4R coding sequence to ensure translation of the entire insert. Each of the exogenous translational control signals and initiation codon can be of either natural or synthetic origin. The efficiency of expression may be enhanced by the inclusion of an appropriate transcription enhancer element, transcription terminator, etc. (*see*, Bittner *et al*., 1987, *Methods in Enzymol.* 153:516-544).

In addition, a host cell strain may be used that modulates the expression of the MC4R nucleic acid, or modifies and processes the MC4R gene product in the specific fashion desired. Such a modification (*e.g.*, glycosylation) or processing (*e.g.*, cleavage) of the MC4R gene product may be important for the function of the protein. Different host cells have characteristic and specific mechanisms for the post-translational processing and modification of proteins. An appropriate cell line or host system can be chosen to ensure the correct modification and processing of the MC4R gene product. To this end, a eukaryotic host cell line that possesses the cellular machinery for proper processing of the primary transcript, and glycosylation and phosphorylation of the gene product, may be used. Examples of such a mammalian host cell line include, but are not limited to, CHO, VERO, BHK, HeLa, COS, MDCK, 293, and Wl38 cell lines.

For long-term, high-yield production of a recombinant protein, stable expression may be preferred. For example, a cell line that stably expresses the MC4R nucleic acid may be engineered. Rather than using an expression vector that contains a viral origin of replication, a host cell line can be transformed with a recombinant plasmid comprising the MC4R nucleic acid controlled by one or more appropriate expression control elements (*e.g.*, a promoter, enhancer, transcription terminator, polyadenylation site, etc.) and a selectable marker. Following transformation, the cell line may be allowed to grow for 1-2 days in an enriched medium, and then switched to a selective medium. The selectable marker in the recombinant plasmid confers resistance to the selection and allows a transformed cell to stably integrate the plasmid into a chromosome and grow to form a focus which in turn can be cloned and expanded into a cell line. This method may advantageously be used to engineer a cell line that expresses the MC4R gene product on the cell surface, and which responds to MC4R ligand mediated signal transduction. Such an engineered cell line is particularly useful in screening MC4R ligands and ligand analogs.

A host cell containing the MC4R nucleic acid and which express the biologically active MC4R gene product may be identified by any one of at least four general approaches, which are, not by way of limitation, as follows: (a) DNA-DNA or DNA-RNA hybridization; (b) the presence or absence of "marker" gene functions; (c) assessing the level of transcription as measured by the expression of MC4R mRNA transcripts in the host cell; and (d) detection of the gene product as measured by immunoassay or by its biological activity.

In the first approach, the presence of the MC4R nucleic acid inserted in the expression vector can be detected by DNA-DNA or DNA-RNA hybridization using probes comprising nucleotide sequences that are homologous to the MC4R coding sequence, respectively, or portions or derivatives thereof.

In the second approach, the recombinant expression vector/host system can be identified and selected based upon the presence or absence of certain "marker" gene functions (*e.g.*, thymidine kinase activity, resistance to antibiotics, resistance to methotrexate, transformation phenotype, occlusion body formation in baculovirus, etc.). For example, if the MC4R nucleic acid is inserted within a marker gene sequence of the vector, recombinants containing the MC4R nucleic acid can be identified by the absence of the marker gene function. Alternatively, a marker gene can be placed in tandem with the MC4R nucleic acid under the control of the same or a different promoter used to control the expression of the MC4R nucleic acid. Expression of the marker in response to induction or selection indicates expression of the MC4R nucleic acid.

In the third approach, transcriptional activity for the MC4R nucleic acid can be assessed by a hybridization assay. For example, RNA can be isolated the transformed cell line and analyzed by Northern blot analysis using a probe identical or similar to the MC4R nucleic acid or particular portions thereof. Alternatively, total nucleic acids of the transformed cell may be extracted and assayed for hybridization to such probes.

In the fourth approach, the expression of the MC4R nucleic acid can be assessed by immunological detection of the MC4R gene product, *e.g.*, by Western blots, immunoassays such as radioimmuno-precipitation, enzyme-linked immunoassays, *etc.* The ultimate test of the success of the expression system, however, involves the detection of the biologically active MC4R gene product. A number of assays can be used to detect receptor activity including, but not limited to, an MC4R ligand binding assay; and a biological assay using engineered cell lines as the test substrate.

Of course, any other approach known to one skilled in the art may be employed to identify MC4R nucleic acid comprising host cells and expression of biologically active MC4R gene product.

### Screening For Compounds Modulating The Activity Of The MC4R Of The Invention

The aspect of the invention described in the paragraphs below encompasses screening methods (*e.g.*, assays) for the identification of compounds that modulate appetite and/or metabolic rate in animals *via* modulation of the activity of the MC4R gene product. Such a compound may be, *e.g.,* an MC4R agonist or an MC4R antagonist. An MC4R antagonist causes an increase in appetite, and perhaps body weight, by reducing MC4R-dependent signaling. An MC4R agonist causes a decrease in appetite, and perhaps body weight, and an increase in metabolic rate by increasing MC4R-dependent signaling. The invention of this aspect also encompasses the agonists and antagonists of MC4R identified using the disclosed assays, including small molecules (*e.g.*, small organic compounds), large molecules (*e.g.*, peptides), and antibodies, as well as nucleotide sequences that can be used to inhibit MC4R gene expression (*e.g.*, antisense and ribozyme molecules), and gene or regulatory sequence replacement constructs designed to enhance MC4R gene expression (*e.g.*, expression constructs that place the MC4R gene under the control of a strong promoter system). These compounds may be used to treat appetite and metabolic disorders in animals in need thereof.

In particular, the invention encompasses cellular and non-cellular assays that can be used to identify compounds that interact with the MC4R, *e.g.*, modulate the activity of the MC4R and/or bind to the MC4R. The cell based assays can be used to identify compounds or compositions that affect the signal-transduction activity of MC4R, whether they bind to MC4R or act on intracellular factors involved in the MC4R signal transduction pathway. To this end, cells that endogenously express MC4R may be used to screen for compounds. Alternatively, cell lines, such as HEK293 cells, COS cells, CHO cells, fibroblasts, and the like, genetically engineered to express the MC4R may be used for screening purposes. The cells can be further engineered to incorporate a reporter molecule linked to the signal transduced by the activated MC4R to aid in the identification of compounds that modulate MC4R signaling activity. Preferably, a host cell line genetically engineered to express a functional receptor that responds to activation by melanocortin peptides can be used as an endpoint in the assay; *e.g.*, as measured by a chemical, physiological, biological, or phenotypic change, induction of a host cell gene or a reporter gene, change in cAMP levels, adenylyl cyclase activity, host cell G protein activity, extracellular acidification rate, host cell kinase activity, proliferation, differentiation, etc.

By way of example but not limitation, a cell line suitable for a cell based assay may be made, *e.g.*, by transfecting HEK293 cells with a feline or canine MC4R nucleic acid using any method known in the art. For example, a 50% confluent plate containing HEK293 cells can be transiently transfected with a vector construct comprising a feline or canine MC4R nucleic acid using FuGENE 6™ (F. Hoffmann-La Roche Ltd, Basel, Switzerland) as a transfection carrier (4µl FuGENE 6™ per ug plasmid DNA). The transfected cells may be harvested 48 hours post transfection using Sigma dissociation buffer (Sigma, St. Louis, MO) centrifuged and resuspended in binding buffer (50mM HEPES, 5mM MgCl, 0.1% BSA, and a protease inhibitor cocktail (Sigma P-8340™ (Sigma, St. Louis, MO), pH=7.5). The resuspended cell population is counted using a hemacytometer. A cell volume titration is performed by, e.g., pipetting varying volumes in triplicate into tubes. Either buffer or excess non-radioactive NDP-MSH (2µM final conc.) is added. Excess non-radioactive NDP-MSH is used to ascertain non-specific binding. Radiolabled NDP-MSH ([¹²⁵I][Nle₄,D-Phe₇]-α-MSH; NEN®, Boston MA) is added to a final concentration of 75pM. The reaction is incubated at 37°C for one hour, then the cells are centrifuged at 5000xG for 10 minutes. The supernatant is aspirated, to remove unbound label, and the amount of membrane-associated (*i*.*e*., receptor-bound) label is determined using a gamma counter. One or more transfected cell lines exhibiting a high level of specific binding of labeled NDP-MSH are selected for further use.

In utilizing such cell systems, the cells expressing the melanocortin receptor are contacted with a test compound or vehicle controls (*e*.*g*., placebos). The cells then may be assayed to measure the expression and/or activity of components of the signal transduction pathway of the melanocortin receptor, or the activity of the signal transduction pathway itself may be assayed. For example, after exposure, cell lysates may be assayed for induction of cAMP. The ability of a test compound to increase levels of cAMP, above those levels seen with cells treated with a vehicle control, indicates that the test compound induces signal transduction mediated by the melanocortin receptor expressed by the host cell *(i.e.,* the test compound is an agonist). When screening a compound that may act as an antagonist of MC4R, it is necessary to include a ligand that activates the MC4R, *e*.*g*., α-MSH, β-MSH or ACTH, to test for inhibition of signal transduction by the test compound as compared to a vehicle control.

In one specific embodiment, a cell based assay is used to determine whether a candidate ligand binds to MC4R. In an example of such an assay, cells from an MC4R transfected cell line (created, *e*.*g*., as above) are contacted with radiolabeled NDP-MSH. Other cells from the same MC4R transfected cell line are contacted with radiolabeled NDP-MSH and a candidate ligand. Each group of cells is incubated at 37°C for one hour, then each group of cells is centrifuged at 5000xG for 10 minutes. The supernatant is aspirated, to remove unbound label, and the amount of membrane-associated (*i*.*e*., receptor-bound) label is determined using a gamma counter. A candidate ligand that displaces radiolabeled NDP-MSH from MC4R, as evidenced by a candidate ligand-dependent reduction in membrane-bound radioactivity, is selected for further analysis.

In another specific embodiment, a cell based assay is used to determine whether a candidate ligand is an agonist or antagonist of MC4R. In one such assay, an MC4R transfected cell line (created, *e.g.,* as above) is further transfected with a reporter construct. The reporter construct is a gene comprising two characteristics. First, its level of expression is regulated by the level of activity of MC4R. For example, the reporter gene's expression can be dependent upon a cAMP-responsive element. The responsive element can increase transcription of the reporter gene in response to MC4R-dependent signaling, in which case increased expression of the reporter gene correlates with increased MC4R-dependent signaling, or the responsive element can decrease transcription of the reporter gene in response to MC4R-dependent signaling, in which case decreased expression of the reporter gene correlates with decreased MC4R-dependent signaling. Second, the reporter construct comprises a reporter gene. The reporter gene comprises a nucleic acid that encodes a gene product that can be assayed. A wide range of reporter genes may be employed. For example, the reporter gene may be a nucleic acid encoding chloramphenicol acetyltransferase (hereinafter "CAT"), luciferase, GUS, growth hormone, or placental alkaline phosphatase (hereinafter "SEAP"). Following exposure of the cells to the test compound, the level of reporter gene expression may be quantitated to determine the test compound's ability to regulate receptor activity. Particularly useful in the practice of the invention is the use of an alkaline phosphatase encoding reporter gene as this enzyme is secreted from the cell, thus tissue culture supernatant may be assayed for secreted alkaline phosphatase. In addition, alkaline phosphatase activity may be measured by colorimetric, bioluminescent or chemilumenscent assays such as those described in Bronstein et *al*., 1994, *Biotechniques* 17: 172-77. Such assays provide a simple, sensitive and easily automatable detection system for pharmaceutical screening. Other reporter genes that may be used include those that result in bioluminescence, colorimetric reactions or fluorescence. For example, the reporter gene may encode for a pigment (*see*, *e*.*g*., Bonhoeffer, 1995, *Arzneimittelforschung* 45:351-356) such as bacterial rhodopsin (*see*, Ng *et al*., 1995, *Biochemistry* 34:879-890), melanin (*see*, Vitkin et *al*., 1994, *Photochemistry and Photobiology* 59:455-62), aquorins (Molecular Probes, Eugene, OR), green fluorescent protein (hereinafter "GFP"; Clonetech, Palo Alto, CA; Chalfie *et al*., 1994, Science 263:802-805; Cubitt et *al*., 1995, *TIBS* 20:448-455), yellow fluorescent protein (*see*, Daubner *et al*., 1987, *Proc. Natl*. *Acad*. *Sci*. *U.S.A.* 84:8912-8916), flavins, bioflavinoids, hemoglobin (*see*, Chance *et al*., 1995, *Analytical Biochemistry* 227:351-362; Shen *et al*., 1993, *Proc. Natl*. *Acad*. *Sci. U.S.A.* 90:8108-8112), heme (Pieulle *et al.,* 1996, *Biochem. Biophys. Acta* 1273: 51-61), indigo dye (Murdock *et al*., 1993, *Biotechnology* 11:381-386), peridinin-chlorophyll-a protein (hereinafter "PCP") (Ogata *et al*., 1994, *FEBS Letters* 356:367-371), or pyocyanine (al-Shibib and Kandela, 1993, *Acta Microbiologica Polonica* 42:275-280). Alternatively, the reporter gene may encode an enzyme that can cleave a color absorbing substrate such as β-lactamase, a luminescent or fluorescent protein, an enzyme with a fluorescent substrate, or any other gene that encodes an optically active chemical or that can convert a substrate to an optically active compound. In a further alternative, the reporter gene may encode photoproteins. In each case, the reporter gene is operatively linked to an inducible promoter which is activated by MC4R-dependent signal transduction (*e.g.*, a cAMP responsive promoter element).

In a specific embodiment of the invention, a bioluminescent reporter gene is employed. Several types of bioluminescent reporter genes are known, including the luciferase family (*see*, *e.g.,* Wood et *al*., 1989, *Science* 244:700-702). Members of the luciferase family have been identified in a variety of prokaryotic and eukaryotic organisms. Luciferase and other enzymes involved in the prokaryotic luminescent (*lux*) systems, as well as the corresponding *lux* genes, have been isolated from marine bacteria in the *Vibrio* and *Photobacterium* genera and from terrestrial bacteria in the *Xenorhabdus* genus, also called photorhalodus. An exemplary eukaryotic organism containing a luciferase system (*luc*) is the North American firefly *Photinus pyralis*. Firefly luciferase has been extensively studied, and is widely used in ATP assays. cDNAs encoding luciferases from *Pyrophorus plagiophthalamus,* another species, click beetle, have been cloned and expressed (*see*, Wood *et al*., *supra*). This beetle is unusual in that different members of the species emit bioluminescence of different colors. Four classes of clones, having 95-99% similarity with each other, were isolated. They emit light at 546 nm (green), 560 nm (yellow-green), 578 nm (yellow) and 593 nm (orange).

Luciferases requires a source of energy, such as ATP, NAD(P)H, and the like, and a substrate, such as luciferin, decanal (bacterial enzymes) or coelentrizine and oxygen. The substrate luciferin must be supplied to the luciferase enzyme in order for it to luminesce. Thus, a convenient method for providing luciferin is to express not only the luciferase but also the biosynthetic enzymes for the synthesis of the substrate decanal. Oxygen is then the only extrinsic requirement for bioluminescence, in bacteria expressing these proteins from the Lux operon.

For example, the *lux* operon obtained from the soil bacterium *Xenorhabdus luminescence* (Frackman *et al*., 1990, *J. Bact*. 172:5767-5773) may be used as the reporter gene, as it confers on transformed *E*. *coli* the ability to emit photons through the expression of the two subunits of the heterodimeric luciferase and three accessory proteins (Frackman et *al*., 1990, *supra).* Optimal bioluminescence for *E*. *coli* expressing the lux genes of *X. luminescence* is observed at 37°C (Szittner and Meighen 1990, *J. Biol. Chem.* 265:16581-16587; Xi et *al.,* 1991, *J. Bact.* 173:1399-1405), which contrasts the low temperature optima of luciferases from eukaryotic and other prokaryotic luminescent organisms (*see,* Campbell, 1988, *Chemiluminescence*. *Principles and Applications in Biology and Medicine* (Chichester, England: Ellis Horwood Ltd. and VCH Verlagsgesellschaft mbH)). Thus, the reporter gene may be chosen according to the nature and the requirements of a specific application. For example, the luciferase from *X. luminescence,* therefore, is well-suited for use as a marker for studies in animals.

Luciferase vector constructs can be adapted for use in transforming a variety of host cells, including most bacteria, and many eukaryotic cells. In addition, certain viruses, such as herpes virus and vaccinia virus, can be genetically-engineered to express luciferase. For example, Kovacs and Mettenlieter, 1991, *J*. *Gen*. *Virol.* 72:2999-3008, teach the stable expression of the gene encoding firefly luciferase in a herpes virus. Brasier and Ron, 1992, *Meth. in Enzymol.* 216:386-96, teach the use of luciferase gene constructs in mammalian cells. Luciferase expression from mammalian cells in culture has been studied using CCD imaging both macroscopically *(see,* Israel and Honigman, 1991, *Gene* 104:139-145) and microscopically (*see*, Hooper *et al*., 1990, *J*. *Biolum. and Chemilum.* 5:123-130). To be useful in this screening assay, the host cell line expressing functional MC4R should give a significant response to MC4R ligand, preferably greater than 5-fold induction over background. The host cell line should preferably possess a number of characteristics to maximize the response induced by melanocortin peptides: (a) a low natural level of cAMP, (b) G proteins capable of interacting with the MC4R, (c) a high level of adenylyl cyclase, (d) a high level of protein kinase A, (e) a low level of phosphodiesterases, and (f) a high level of cAMP response element binding protein would be advantageous. To increase response to melanocortin peptide, host cells could be engineered to express a greater number of favorable factors or a lesser number of unfavorable factors. In addition, alternative pathways for induction of the CRE reporter could be eliminated to reduce basal levels.

In a specific embodiment, a transfected cell line comprising both an MC4R nucleic acid and a reporter gene is contacted with the candidate ligand at 37°C for one hour. Expression of the reporter gene is then measured and compared to reporter gene expression in a similar batch of cells treated identically but for contact with the candidate ligand. A candidate ligand causing a five-fold or greater increase in reporter gene expression is selected for further study as a potential MC4R agonist. Another version of this screen can be used to identify a potential antagonist of MC4R. In this version of the screen, prior to contacting the transfected cells with the candidate antagonist, the cells are contacted with a known agonist (*e*.*g*., NDP-MSH). A candidate ligand causing a five-fold or greater decrease in reporter gene expression is selected for further study as a potential MC4R antagonist.

When it is desired to discriminate between the melanocortin receptors and to identify compounds that selectively agonize or antagonize the MC4R, the assays described above should be conducted using a panel of host cells, each genetically engineered to express one of the melanocortin receptors (MC1R through MC5R). To this end, host cells can be genetically engineered to express any of the amino acid sequences known for melanocortin receptors 1 through 5. The cloning and characterization of each receptor from one or more organisms has been described, e.g.: murine and human MC1R and MC2R (Mountjoy, 1992, *Science* 257:1248-1251; Chhajlani and Wikberg, 1992, *FEBS Lett.* 309: 417-420); rat MC3R (*see*, Roselli-Rehfuss *et al*., 1993, *Proc*. *Natl*. *Acad*. *Sci*. *USA* 90: 8856-8860; Gantz *et al*., 1993, *J. Biol.* Chem. 268: 8246-8250); feline and canine MC4R (described herein); and murine and human MC5R (Chhajlani *et al*., 1993, *Biochem. Biophys. Res. Commun*. 195:866-873; Gantz *et al.,* 1994, *Biochem*. *Biophys. Res*. *Commun.* 200:1214-1220), each of which is incorporated by reference herein in its entirety. Thus, each of the foregoing sequences can be utilized to engineer a cell or cell line that expresses one of the melanocortin receptors for use in screening assays described herein. To identify compounds that specifically or selectively regulate MC4R activity, the activation, or inhibition of MC4R activation is compared to the effect of the test compound on the other melanocortin receptors.

Alternatively, if the host cells express more than one melanocortin peptide receptor, the background signal produced by these receptors in response to melanocortin peptides must be "subtracted" from the signal (Gantz et *al.,* 1993, *supra*). The background response produced by these non-MC4R melanocortin receptors can be determined by a number of methods, including elimination of MC4R activity by antisense, antibody or antagonist. In this regard, it should be noted that *wild type* CHO cells demonstrate a small endogenous response to melanocortin peptides which must be subtracted from background. Alternatively, activity contributed from other melanocortin receptors could be eliminated by activating host cells with a MC4R-specific ligand, or including specific inhibitors of the other melanocortin receptors.

In another aspect, the invention comprises a plurality of *in vitro* assays using preparations of MC4R for determining whether a test compound is an agonist or antagonist of MC4R. Such preparations of MC4R may be obtained by methods readily known in the art, *see*, *e*.*g*., Ausubel et *al.,* 1988, *supra.* In a preferred embodiment, a group of test compounds is used serially in the *in vitro* assays, which together comprise a high throughput screen for MC4R agonists and antagonists. In the high throughput screen, a compound identified as a candidate agonist or antagonist by one assay in the series is used in the next assay in the series. A compound that is not identified as a candidate agonist or antagonist by one assay in the series is not used in the next assay in the series. In an especially preferred embodiment, this series of *in vitro* assays comprises an *in vitro* binding assay, a fluorescence imaging plate reader (hereinafter "FLIPR®" (Molecular Devices, Sunnyvale CA)) assay, a cAMP functional assay, and an assay to determine whether the candidate agonist or antagonist binds preferentially to any of MC1R through MC5R. Each of these assays is described in turn below. Of course, the assays may be performed in different orders as described.

First, an *in vitro* binding assay is performed, in which compounds are screened for specific binding to canine or feline MC4R-containing HEK293 cell membranes *in vitro.* Such an *in vitro* assay allows quantification of binding of a ligand to MC4R, which cannot be achieved using whole-cell assays (due to MC4R internalization and recycling). For this assay, canine or feline MC4R-containing membranes are prepared from cells transfected as above using any method known in the art. In a specific, but exemplary embodiment, cells are harvested in a reaction tube using Sigma dissociation buffer, centrifuged and resuspended in ice cold homogenization buffer (1mM EDTA, 1mM EGTA, 10 mM HEPES and a protease inhibitor cocktail (Sigma P-8340™), pH=7.5). The resuspended cells are incubated on ice for at least 10 minutes, then homogenized with 20 strokes of a tight fitting glass/glass dounce homogenizer. The cell extracts are centrifuged at 1000xG for 10 minutes at 4°C to pellet nuclei and unlysed cells. The supernatant fraction is transferred to a new tube and centrifuged at 25000xG for 20 minutes at 4°C, in order to pellet the plasma membrane. The supernatant fraction from this centrifugation is discarded, and the pellet is washed by resuspending it in homogenization buffer, homogenizing it with 20 strokes of a tight fitting glass/glass dounce homogenizer, and centrifuging the homogenate at 25000xG for 20 minutes at 4°C. The supernatant is again discarded, and the pellet is resuspended in a volume of homogenization buffer sufficient to yield a protein concentration of 1-5 mg/ml. 500 µl aliquots are frozen at -70°C for long term storage. The protein concentration of the extracts may be determined using any method known in the art. For example, an aliquot can be diluted 5-10 fold and the BCA kit (Pierce, Rockford, III.) may be used.

The membrane preparation is then subjected to the actual MC4R-binding assay to identify test compounds that bind to canine or feline MC4R. In this binding assay, membranes are incubated with labeled ligand in the presence or absence of test compound. Compounds that bind to the receptor and compete with labeled ligand for binding to the membranes reduce the signal compared to the vehicle control samples.

In a preferred embodiment, radiolabeled NDP-MSH, thawed canine or feline MC4R/HEK293 membrane, and unlabeled test compound are resuspended in assay buffer (50 mM HEPES, 5mM MgCI, 0.1% BSA, and a protease inhibitor cocktail (Sigma P-8340™), pH=7.5) are mixed to a final concentration of NDP-MSH of 0.05 nM and incubated at 37°C for 1 hour. This binding reaction is terminated by harvesting membranes onto a UNIFILTER® GF/C® 96-well microplate (Packard Instrument Co., Meriden, CT) treated with polyethylimmine (Sigma-Aldrich Co., St. Louis, MO). Scintillant is added and a beta counting instrument is utilized. Assays can be performed in a 96 well plate format. Unlabeled test compounds are examined over a range of concentrations from 0.01nM to 20000 nM, and IC₅₀ values are determined. A compound that "hits" in the initial binding screen (*i*.*e*., that binds to MC4R with a K_{d} <10 µM) preferably is re-tested in a 7-point dose-titration assay for determination of IC₅₀ and/or K₁ values. The candidate compounds that show potent binding in this binding assay are then screened for functional activity in the following assays.

In alternative *in vitro* binding assay for MC4R agonists and antagonists, soluble MC4R may be recombinantly expressed and utilized in non-cell based assays to identify compounds that bind to MC4R. The recombinantly expressed MC4R polypeptides or fusion proteins containing one or more of the ECDs of MC4R prepared as described below, can be used in the non-cell based screening assays. Alternatively, peptides corresponding to one or more of the CDs of MC4R, or fusion proteins containing one or more of the CDs of MC4R can be used in non-cell based assay systems to identify compounds that bind to the cytoplasmic portion of the MC4R; such compounds may be useful to modulate the signal transduction pathway of the MC4R. In non-cell based assays the recombinantly expressed MC4R is attached to a solid substrate such as a test tube, microtitre well or a column, by means well known to those in the art. See, Ausubel *et al*., *supra.* The test compounds are then assayed for their ability to bind to the MC4R.

As a second assay, a FLIPR® assay is performed with the candidate compounds identified to potently bind to MC4R in the above binding assay. Because MC4R proteins are members of the G protein coupled receptor (hereinafter "GPCR") family, this assay is designed to involve a functional G-protein coupled screen. In a preferred embodiment, an MC4R is linked to the phospholipase C (hereinafter "PLC") pathway by the use of a special G protein. The G protein may be a naturally occurring "promiscuous" G protein (*i*.*e*., a G protein which can link a plurality of GPCR types to the PLC pathway (Offermans et *al*., 1995, *J. Biol. Chem.* 270:15175-15180), or it may be a modified, chimeric G protein designed to link a selected GPCR to the PLC pathway (Conklin *et al.,* 1996, *Molecular Pharmacology* 50:885-890). The activation or inhibition of a GPCR by an agonist or antagonist can be measured using either of these approaches by measuring changes in the intracellular calcium level caused by activation or inhibition of PLC.

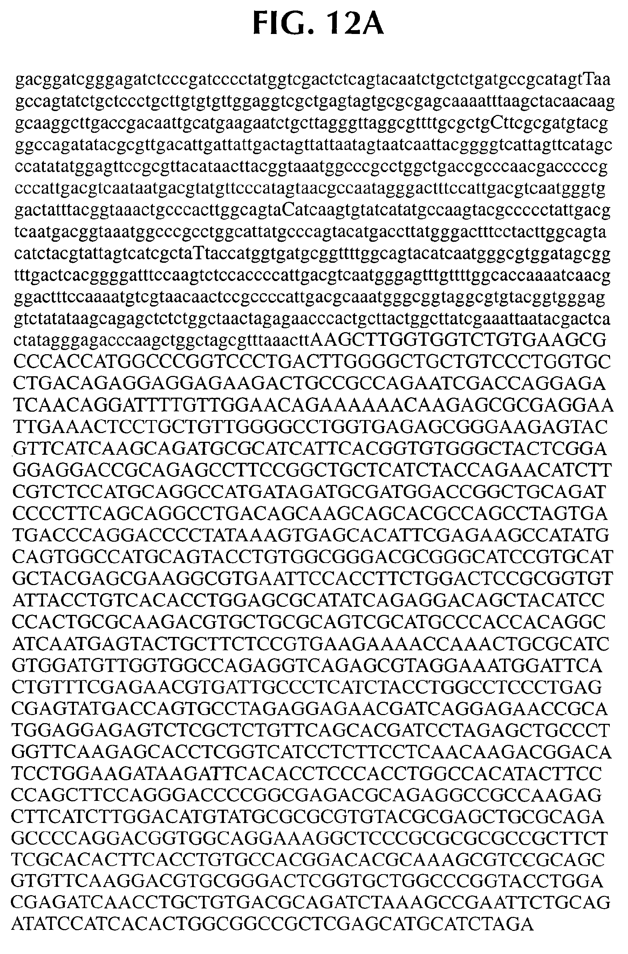

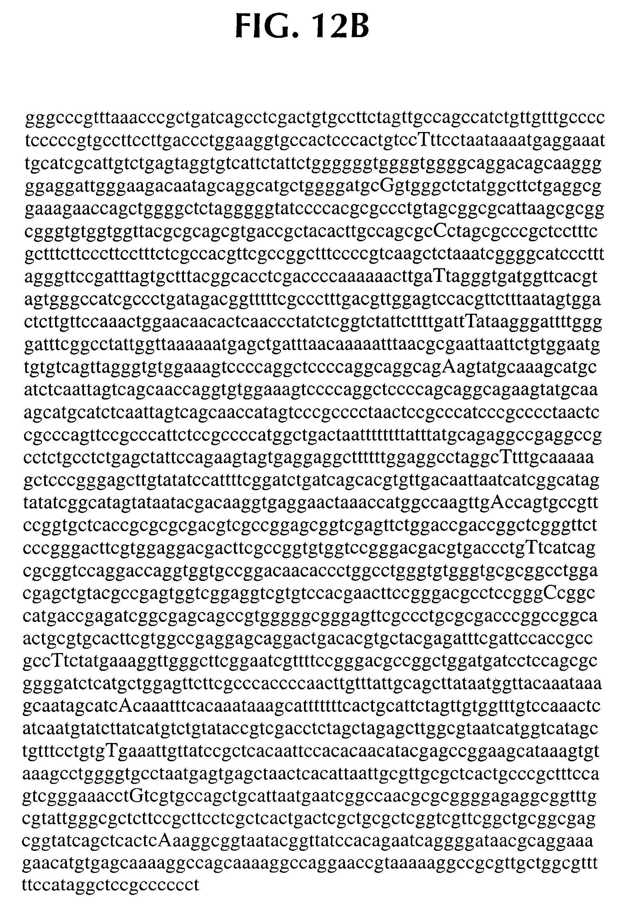

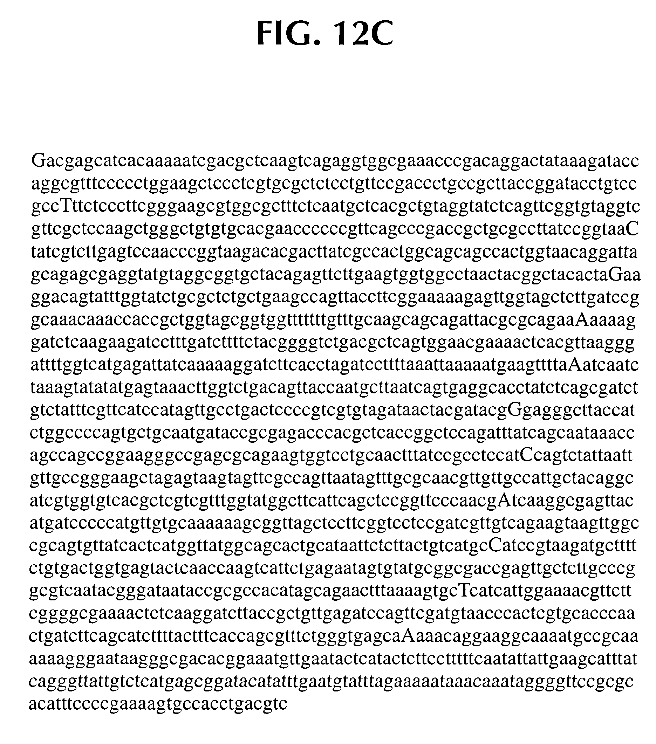

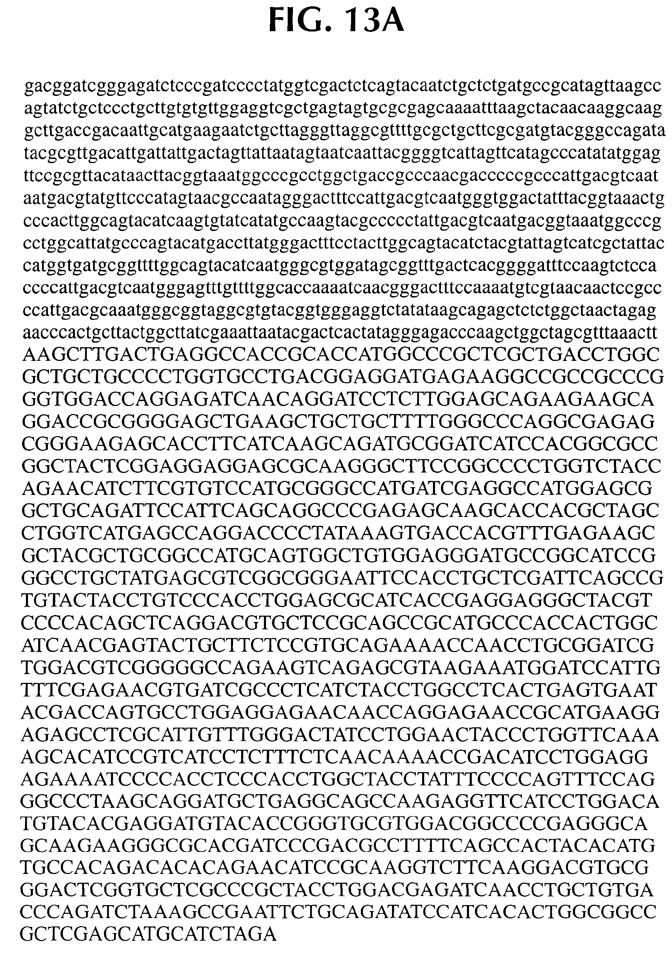

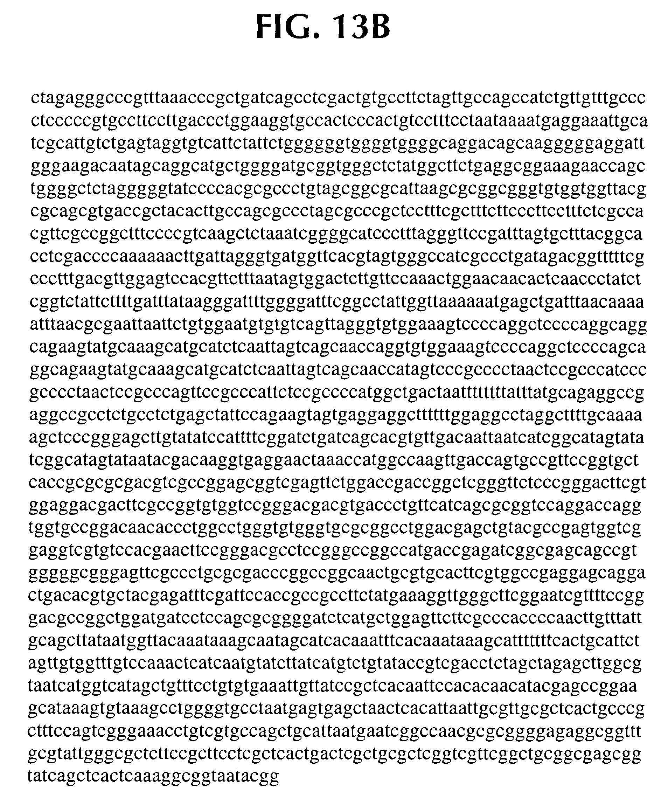

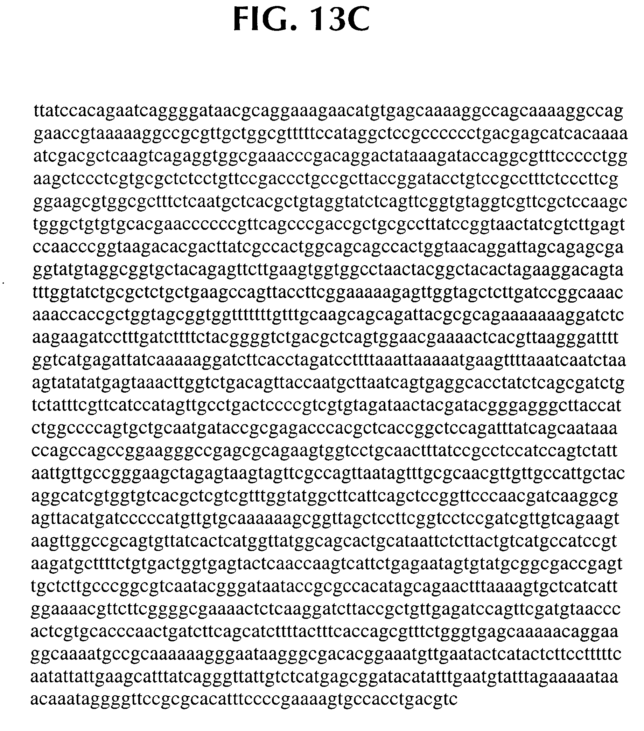

In a specific but exemplary embodiment, a HEK293a stable cell line expressing cMC4R is grown in culture medium (DMEM, 10% FBS, 100 units Penicillin/Streptomycin, 300 mg/ml GENETICIN® (GIBCO BRL, Rockville MD)), transfected with vectors carrying two different G-protein genes, Gα15 and Gα16 (see figures 12 and 13), and selected for stable incorporation of these DNA using a cell medium solution containing 300µg/ml of ZEOCIN™ (Invitrogen, Carlsbad, CA), using techniques well-known in the art. Alternatively, a vector containing a chimeric G-protein (e.g. Gαqi5, a Gα with the last 5 amino acids replaced with those from Gαs) can be generated and used. Single clonal colonies are selected and expanded using cloning cylinders (Sigma-Aldrich Co., St. Louis, MO). Individual clones are tested for the receptor's ability to couple with the incorporated promiscuous G-proteins using a FLIPR® assay, as described below. Gα15 and Gα16 are known as promiscuous G-proteins because of their ability to functionally couple to any G-Protein receptor and transduce signaling to increase intracellular calcium. A FLIPR® machine (Molecular Devices, Sunnyvale CA) allows one to quantify such a calcium signaling cascade using fluorescent dyes available commercially.

HEK293a/cMC4R/Gα15, Gα16 are grown to confluence and harvested with Trypsin-EDTA (GIBCO-BRL, Rockville MD). Cells are resuspended in fresh culture medium containing 300 µg/ml ZEOCIN™. The cell suspension is counted using a hemacytometer and approximately 50,000 cells per well are added to poly-d-lysine-coated black/clear 96 well plates (Becton Dickinson Labware, Bedford MA). Approximately 48 hours after plating, the growth medium is aspirated off, and replaced with serum-free medium containing 25 µg per 96 well plate of calcium-sensitive fluorescent dye Fluo-4 (Molecular Probes, Eugene OR) and 2.5 mM Probenicid (Sigma-Aldrich, St. Louis, MO). The plates are incubated for 1 hour at 37°C, after which the cells are washed 3 times with Hepes Saline solution containing 2.5 mM Probenicid to remove excess dye. The plates are then added to the FLIPR® individually, and fluorescence level is continuously monitored over a 2-minute period. A candidate agonist (in the presence or absence of antagonist), or candidate antagonist (in the presence or absence of agonist) is added to each of the 96 wells simultaneously after 20 seconds of baseline recording. A six-point dose-titration assay if performed for each compound. An increase in fluorescence is indicative of increases in intracellular calcium levels. Time-courses are exported as ASCII files to EXCEL™ (Microsoft, Redman WA) and subsequently analyzed. An agonist candidate that increases intracellular calcium levels, or an antagonist candidate that decreases intracellular calcium levels, is selected for further study.

As a third assay, a cAMP functional assay is performed to test the candidate agonists or antagonists identified by the above FLIPR® assay. Any cAMP functional assay known in the art may be used. This assay is based on the MC4R's characteristic to be a receptor that, when activated by its agonist ligand, stimulates cAMP accumulation. Direct measurement of cAMP produced by a stable canine or feline MC4R-expressing HEK293 cell line contacted with the candidate compound can determine whether the compound is an MC4R agonist or antagonist.

In a specific but exemplary embodiment the cAMP assay is performed using the Adenylyl Cyclase Activation FLASHPLATE™ Assay (NEN® Life Science Products, Inc., Boston MA). An MC4R-transfected cell line is harvested using Sigma Dissociation Buffer, washed, centrifuged and resuspended in Stimulation Buffer (provided in kit). The resuspended cell population is counted using a hemacytometer. Cells are pelleted again by centrifugation and resuspended with Stimulation Buffer to 0.5-5 x 10⁶ cells/ml. A cell volume titration is performed by varying volumes in triplicate into wells. For each cell concentration tested, a full candidate agonist or antagonist dose titration is performed. A known agonist, such as NDP-MSH or α-MSH, may be used as a positive control. The time of agonist stimulation is also varied from 15 to 45 minutes. To stop agonist stimulation, detection mix is added at the appropriate time to all wells. Each plate is covered and incubated 20 hours at room temperature, then placed in a Wallac 1450 MICROBETA™ multidetector (PerkinElmer Life Sciences, Gaithersburg, MD) for scintillation counting. Other cAMP assay kits can be used such as the cAMP SPA™ kit (RPA559; Amersham Pharmacia Biotech, Inc., Piscataway, NJ) or other kits by other vendors.

In a fourth assay, the candidate MC4R agonists or antagonists may be tested to determine whether it binds preferentially to MC4R or MC3R using any method known in the art. In a preferred embodiment, a candidate agonist or antagonist that exhibits potency in the *in vitro* binding and functional screens described above is assayed for selectivity of binding to MC4R versus MC3R receptors. One cell line that expresses MC4R and another cell line that expresses MC3R are created using an HEK293 cell line as described above. For each of these transfected cell lines, whole cell and membrane fraction binding assays are performed as described above. For a more thorough investigation of receptor number, ligand affinity and for comparison to current procedures and literature values, a saturation binding study is performed using membrane fractions. The protein concentration of each preparation is determined so that it can be normalized to the protein concentration of a specific membrane preparation using the BCA protein assay kit. A constant amount of membrane fraction (as measured by total protein amount) is used for each membrane fraction preparation (for a membrane fraction from an MC4R-expressing cell line, this amount is 30 µg protein; for a membrane fraction from an MC3R-expressing cell line, this amount is 70 µg). A saturation binding isotherm is determined by titrating in various amounts of a radioactively labeled candidate agonist or antagonist. The dissociation constants (hereinafter "K_{d}") and the maximum number of specific binding sites per mg of membrane protein (hereinafter "Bₘₐₓ") for binding of the candidate agonist or antagonist to MC4R and MC3R are determined by saturation binding isotherm and non-linear regression analysis using the software package GraphPad PRISM® (GraphPad Software Inc., San Diego, CA). A linear Scatchard line verifies that each membrane preparation contains a population of receptors having a single affinity for the radioactive ligand.

*In vitro* cell based assays also may be designed to screen for compounds that modulate MC4R expression at either the transcriptional or translational level. In one embodiment, a nucleic acid encoding a reporter gene (*see*, *supra)* may be linked to a regulatory element of the MC4R gene and used in appropriate intact cells, cell extracts or lysates to identify compounds that modulate MC4R gene expression. Appropriate cells or cell extracts are prepared from any cell type that normally expresses the MC4R gene, thereby ensuring that the cell extracts contain the transcription factors required for *in vitro* or *in vivo* transcription. The screen may be used to identify compounds that modulate the expression of the reporter construct. In such screens, the level of reporter gene expression is determined in the presence of the test compound and compared to the level of expression in the absence of the test compound.

To identify compounds that modulate MC4R translation, cells or *in vitro* cell lysates containing MC4R transcripts may be tested for modulation of MC4R mRNA translation. To assay for inhibitors of MC4R translation, test compounds are assayed for their ability to modulate the translation of MC4R mRNA in *in vitro* translation extracts.

Compounds that decrease the level of MC4R expression, either at the transcriptional or translational level, may be useful for treatment of decreased appetite-related disorders such as anorexia and cachexia. In contrast, those compounds that increase the expression of MC4R may be useful for treatment of increased appetite-related disorders such as obesity.

The assays described above can identify compounds that affect MC4R activity. For example, compounds that affect MC4R activity include but are not limited to compounds that bind to the MC4R, inhibit binding of the natural ligand, and either activate signal transduction (agonists) or block activation (antagonists), and compounds that bind to the natural ligand of the MC4R and neutralize ligand activity. Compounds that affect MC4R gene activity (by affecting MC4R gene expression, including molecules, *e.g.*, proteins or small organic molecules, that affect transcription or interfere with splicing events so that expression of the full length or the truncated form of the MC4R can be modulated) also can be identified using the screens of the invention. However, it should be noted that the assays described also can identify compounds that modulate MC4R signal transduction (*e*.*g*., compounds which affect downstream signaling events, such as inhibitors or enhancers of G protein activities that participate in transducing the signal activated by ligand binding to the MC4R). The identification and use of such compounds that affect signaling events downstream of MC4R and thus modulate effects of MC4R on the development of body weight disorders are within the scope of the invention. In some instances, G protein-coupled receptor response has been observed to subside, or become desensitized with prolonged exposure to ligand. In an embodiment of the invention assays may be utilized to identify compounds that block the desensitization of MC4R. Such compounds may be used to sustain the activity of MC4R, and can be used as part of a therapeutic method for the treatment of appetite disorders.

Compounds identified *via* assays such as those described herein may be useful, for example, in elaborating the biological function of the MC4R gene product, and for ameliorating appetite disorders and metabolic disorders in animals. Assays for testing the efficacy of compounds identified in the cellular screen can be tested in an animal model system for appetite disorders. Such animal models may be used as test substrates for the identification of drugs, pharmaceuticals, therapies and interventions which may be effective in treating such disorders in animals, *e*.*g*., cats, dogs and livestock. For example, an animal model may be exposed to a compound, suspected of exhibiting an ability to ameliorate appetite disorder symptoms, at a sufficient concentration and for a time sufficient to elicit such an amelioration of appetite disorder symptoms in the exposed animal. The response of the animal to the exposure may be monitored by assessing the reversal of symptoms associated with appetite disorders such as cachexia or obesity. With regard to intervention, a treatment which reverses any aspect of appetite disorder-like symptoms should be considered as a candidate for appetite disorder therapeutic intervention in animals, particularly in cats, dogs and livestock. Dosages of test agents may be determined by deriving dose-response curves, as discussed below.

The animal model may be an animal that overexpresses the MC4R gene product. Animals of any species, including, but not limited to, mice, rats, rabbits, guinea pigs, pigs, micro-pigs, goats, cattle, and non-human primates, *e*.*g*., baboons, monkeys, and chimpanzees may be used to generate MC4R transgenic animals. Transgenic mice and rats are especially preferred because of their relatively small size, ease of care, short generation time, extensively studied genetic constitution, and well-known laboratory rearing conditions.

Any technique known in the art may be used to introduce the MC4R transgene into an animal to produce the founder line of a transgenic animal. Such techniques include, but are not limited to pronuclear microinjection (*see*, Hoppe and Wagner, 1989, U.S. Pat. No. 4,873,191); retrovirus mediated gene transfer into germ lines (*see*, Van der Putten *et al*., 1985, *Proc. Natl*. *Acad. Sci.* USA 82:6148-6152); gene targeting in embryonic stem cells (Thompson *et al.,* 1989, *Cell* 56:313-321); electroporation of embryos (Lo, 1983, *Mol Cell. Biol.* 3:1803-1814); and sperm-mediated gene transfer (Lavitrano *et al*., 1989, *Cell* 57:717-723); etc. For a review of such techniques, *see,* Gordon, 1989, *Transgenic Animals, Intl*. *Rev. Cytol.* 115:171-129, which is incorporated by reference herein in its entirety.

The present invention provides for a transgenic animal that carries the MC4R transgene in all of its cells, as well as an animal which carries the transgene in some, but not all, of its cells, i.e., mosaic animals. The transgene may be integrated as a single transgene or in a concatamer, *e.g.,* head-to-head or head-to-tail tandem repeats. The transgene may also be selectively introduced into and activated in a particular cell type by following, for example, the teaching of Lasko *et al*., 1992, *Proc. Natl*. *Acad*. *Sci*. *USA* 89: 6232-6236. The regulatory sequences required for such a cell-type specific activation will depend upon the particular cell type of interest, and will be apparent to those of skill in the art. When it is desired that the MC4R transgene be integrated into the chromosomal site of the endogenous MC4R gene, gene targeting is preferred. Briefly, when such a technique is to be utilized, a vector containing nucleic acids with sequences having a high percentage of identical nucleotide residues to the endogenous MC4R gene and/or sequences flanking the gene are designed for the purpose of integrating, via homologous recombination with chromosomal sequences, into and disrupting the function of the endogenous MC4R gene. The transgene also may be selectively expressed in a particular cell type with concomitant inactivation of the endogenous MC4R gene in only that cell type, by following, for example, the teaching of Gu *et al*., 1994, *Science* 265:103-06. The regulatory sequences required for such a cell-type specific recombination will depend upon the particular cell type of interest, and will be apparent to those of skill in the art.

Once a founder animal has been generated, standard techniques such as Southern blot analysis or PCR techniques are used to analyze animal tissues to determine whether integration of the transgene has taken place. The level of mRNA expression of the transgene in the tissues of the founder animal also may be assessed using techniques which include, but are not limited to, Northern blot analysis of tissue samples obtained from the animal, *in situ* hybridization analysis, and RT-PCR. Samples of MC4R gene-expressing tissue may also be evaluated immunocytochemically using antibodies specific for the MC4R transgene product.

A compound identified by an assay described above that stimulates or enhances the signal transduced by activated MC4R, *e*.*g*., by activating downstream signaling proteins in the MC4R cascade and thereby by-passing the defective MC4R, may be used to achieve weight loss, as described below. The formulation and mode of administration will depend upon the physico-chemical properties of the compound. The administration should include known techniques that allow for a crossing of the blood-brain barrier.

### Sources For Compounds Modulating The Activity Of The MC4R Of The Invention

The compounds that may be tested using the assays described above for MC4R agonist or antagonist activity include, but are not limited to, peptides such as, for example, soluble peptides, including but not limited to members of random peptide libraries; (*see*, *e*.*g*., Lam et *al.,* 1991, *Nature* 354:82-84; Houghten *et al*., 1991, *Nature* 354:84-86), and combinatorial chemistry-derived molecular library made of D- and/or L- configuration amino acids, phosphopeptides (including, but not limited to, members of random or partially degenerate, directed phosphopeptide libraries (*see*, *e*.*g*., Songyang et *al*., 1993, *Cell* 72:767-78), antibodies (including, but not limited to, polyclonal, monoclonal, humanized, anti-idiotypic, chimeric or single chain antibodies, and FAb, F(ab')₂ and FAb expression library fragments, and epitope-binding fragments thereof), the ECD of the MC4R (or a portion thereof) and bind to and "neutralize" natural ligand, and small organic or inorganic molecules.

Other compounds that can be screened in accordance with the invention include but are not limited to small organic molecules that are able to cross the blood-brain barrier, gain entry into an appropriate cell and affect the expression of the MC4R gene or some other gene involved in the MC4R signal transduction pathway (*e.g.*, by interacting with the regulatory region or transcription factors involved in gene expression); or such compounds that affect the activity of the MC4R or the activity of some other intracellular factor involved in the MC4R signal transduction pathway, such as, for example, the MC4R associated G protein.

### Identification Of Ligands Using Computer Modeling

Computer modeling and searching technologies permit identification of a ligand, or the improvement of an already identified ligand, that can modulate MC4R expression or activity. Having identified the ligand, its active site or region is identified. The active site might typically be the MC4R binding site. The active site can be identified using methods known in the art including, for example, by examination of the amino acid sequence if the ligand is a peptide, from its nucleotide sequence if it is a nucleic acid, or from study of complexes of the compound or composition with MC4R. In the latter case, chemical or X-ray crystallographic examination of the complex can be used to find the active site by finding where the ligand and MC4R contact each other.

Next, the three dimensional geometric structure of the active site of the ligand is determined. This can be done by known methods, including X-ray crystallography, which can determine a complete molecular structure. On the other hand, solid or liquid phase NMR can be used to determine certain intra-molecular distances. Any other experimental method of structure determination can be used to obtain partial or complete geometric information. The geometry of the active site may be measured while the ligand is complexed with MC4R, or with another binding partner, which may increase the accuracy of the measurements.

If an incomplete or insufficiently accurate molecular structure of the ligand's active site is determined, the methods of computer based numerical modeling may be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active site of the ligand, candidate ligands can be identified by searching databases containing compounds and information about their molecular structure. The search seeks compounds having structures that are identical or similar to the active site structure of the ligand. Such a search can be manual, but is preferably computer assisted. A compound identified in this search is a potential MC4R agonist or antagonist.

Alternatively, these methods can be used to create an improved agonist or antagonist from one that is already known. The composition of the known agonist or antagonist is modified and the structural affects of modification are determined using the experimental and computer modeling methods described above. Binding of the modified agonist or antagonist to MC4R, or its effect on MC4R-dependent signaling, is then compared to that of the original agonist or antagonist. Using these methods systematic variations of the known agonist or antagonist (*e.g.*, systematic variation of a particular side group), can be quickly evaluated to obtain modified agonists or antagonists of improved specificity or activity. One or a plurality of these steps may be repeated serially to identify or create increasingly effective MC4R agonists or antagonists.

Further experimental and computer modeling methods useful for identifying an MC4R agonist or antagonist based upon identification of the active site of MC4R, and the active sites of related transduction and transcription factors, will be apparent to those of skill in the art.

Examples of molecular modeling systems are the CHARMm™ and QUANTA™ programs (Polygen Corporation, Waltham, MA). CHARMm™ performs the energy minimization and molecular dynamics functions. QUANTA™ performs the construction, graphic modeling and analysis of molecular structure. QUANTA™ allows interactive construction, modification, visualization, and analysis of the behavior of molecules with each other.

A number of articles review computer modeling of drugs interactive with specific proteins, such as Rotivinen, *et al*., 1988, *Acta Pharmaceutical Fennica* 97:159-166; Ripka, 1988, *New Scientist* 118:54-57; McKinaly and Rossmann, 1989, *Annu. Rev. Pharmacol. Toxiciol.* 29:111-122; Perry and Davies, 1989, *OSAR: Quantitative Structure-Activity Relationships in Drug Design* pp. 189-193 Alan R. Liss, Inc.; Lewis and Dean, 1989, Proc. *R. Soc. Lond.* 236:125-140 and 141-162; and, with respect to a model receptor for nucleic acid components, Askew, *et al.,* 1989, *J. Am. Chem. Soc.* 111:1082-1090. Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario, Canada). Although these are primarily designed for application to drugs specific to particular proteins, they can be adapted to design of drugs specific to regions of DNA or RNA, once that region is identified.

### MC4R Peptides

In another aspect of the invention, canine or feline MC4R protein, polypeptides and peptide fragments, mutated, truncated or deleted forms of the MC4R and/or MC4R fusion proteins are prepared for a variety of uses, including but not limited to the generation of antibodies, as reagents in diagnostic assays, the identification of other cellular gene products involved in the regulation of appetite in animals, as reagents in assays for screening for compounds that can be used in the treatment of appetite disorders in animals, and as pharmaceutical reagents related to the MC4R useful in the treatment of appetite disorders in animals.

A peptide corresponding to one or more domains of the MC4R (*e.g.*, ECDs, TMs or CDs), a truncated or deleted MC4R (*e.g.*, MC4R in which one or more of the ECDs, TMs and/or CDs is deleted) as well as a fusion protein in which the full length MC4R, an MC4R peptide or truncated MC4R is fused to an unrelated protein are also within the scope of the invention. Such a soluble peptide, protein, fusion protein, or antibody (including an anti-idiotypic antibody) that binds to and "neutralizes" circulating natural ligand for the MC4R can be used as described below to effectuate an increase in appetite. To this end, a peptide corresponding to an individual ECD of MC4R, a soluble deletion mutant of MC4R (*e.g.*, ΔTM mutants), or the entire MC4R ECD (engineered by linking the four ECDs together as described below) can be fused to another polypeptide (*e.g.*, an IgFc polypeptide). Fusion of the MC4R or the MC4R ECD to an IgFc polypeptide should not only increase the stability of the preparation, but will increase the half-life and activity of the MC4R-Ig fusion protein *in vivo.* The Fc region of the Ig portion of the fusion protein may be further modified to reduce immunoglobulin effector function.

Such a peptide, polypeptide, or fusion protein can be prepared by recombinant DNA techniques. For example, a nucleic acid encoding one or more of the four domains of the ECD of the serpentine MC4R can be synthesized or cloned and ligated together to encode a soluble ECD of the MC4R. Two or more nucleic acids, each encoding one or more of the four ECDs (ECD1-4 in Figures 1 and 2), may be ligated together directly or *via* a linker oligonucleotide that encodes a peptide spacer. The linker may encode a flexible, glycine-rich polypeptide thereby allowing the ECDs that are strung together to assume a conformation that can bind an MC4R ligand. Alternatively, a nucleic acid encoding an individual domain within the ECD can be used to express an MC4R-derived peptide.

A variety of host-expression vector systems may be utilized to express a nucleic acid encoding the appropriate regions of the MC4R to produce the polypeptides described above. Where the resulting peptide or polypeptide is a soluble derivative (*e*.*g*., a peptide corresponding to an ECD; a truncated or internally-deleted MC4R) the peptide or polypeptide can be recovered from the culture medium. Where the polypeptide or protein is not secreted, the MC4R product can be recovered from the host cell itself.

The host-expression vector systems also encompass engineered host cells that express the MC4R or functional equivalents *in situ, i.e.,* anchored in the cell membrane. Purification or enrichment of the MC4R from such expression systems can be accomplished using appropriate detergents and lipid micelles and methods well known to those skilled in the art. However, such engineered host cells themselves may be used in situations where it is important not only to retain the structural and functional characteristics of the MC4R, but to assess biological activity, *e.g.*, in drug screening assays, see, *supra.*

A fusion protein may be readily purified by utilizing an antibody specific for the fusion protein being expressed. For example, one such system allows for the ready purification of non-denatured fusion proteins expressed in human cell lines (*see*, Janknecht, et *al.,* 1991, *Proc. Natl. Acad. Sci. USA* 88: 8972-76). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺· nitriloacetic acid-agarose columns and histidine-tagged proteins are selectively eluted with imidazole-containing buffers.

### MC4R Antibodies

In again another aspect, antibodies that specifically recognize one or more epitopes of feline or canine MC4R, or epitopes of conserved variants of MC4R, or peptide fragments of the MC4R are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

An antibody of the invention may be used, for example, in the detection of MC4R in a biological sample and may, therefore, be utilized as part of a diagnostic or prognostic technique whereby an animal may be tested for an abnormal amount of MC4R. The antibody also may be utilized in conjunction with, for example, a compound screening scheme, as described, above, for the evaluation of the effect of a test compound on expression and/or activity of the MC4R gene product. Additionally, the antibody may be used in conjunction with the transgenic techniques described, below, *e.g.*, to evaluate the normal and/or engineered MC4R-expressing cells prior to their introduction into the animal subject. The antibody additionally may be used as a method for the inhibition of abnormal MC4R activity. Thus, the antibody may be utilized as part of an appetite disorder treatment method.

For the production of the antibody, a host animal may be immunized by injection with MC4R, an MC4R peptide (*e*.*g.*, one corresponding the a functional domain of the receptor, such as ECD, TM or CD), a truncated MC4R polypeptide (*i.e*, MC4R in which one or more domains, *e*.*g*., the TM or CD, has been deleted), a functional equivalent of the MC4R or a mutant of the MC4R. The host animal may be, *e.g.*, a goat, rabbit, mouse, hamster or rat. An adjuvant may be used to increase the immunological response, depending on the host species. The adjuvant may be, *e*.*g*., Freund's (complete and incomplete), mineral gels (*e.g.* aluminum hydroxide), surface active substances such as lysolecithin, pluronic polyols, polyanions, peptides, oil emulsions, keyhole limpet hemocyanin, dinitrophenol, and potentially useful human adjuvants such as bacille Calmette-Guerin(hereinafter "BCG") and *Corynebacterium parvum.*

A polyclonal antibody is a heterogeneous population of antibody molecules derived from the serum of an immunized animal. A monoclonal antibody (hereinafter "mAb") is a homogeneous population of antibodies. A mAb to a particular antigen may be generated by any technique that provides for the production of antibody molecules by a continuous cell line in culture. These techniques include, but are not limited to, the hybridoma technique of Kohler and Milstein, 1975, Nature 256:495-497; and U.S. Patent No. 4,376,110, the human B-cell hybridoma technique (Kosbor et *al*., 1983, *Immunology Today* 4:72; Cole *et al*., 1983, *Proc. Natl. Acad. Sci. USA* 80:2026-2030), and the EBV-hybridoma technique (*see*, Cole et *al.,* 1985, *Monoclonal Antibodies And Cancer Therapy,* Alan R. Liss, Inc., pp. 77-96). The antibody may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (*see*, Morrison et *al*., 1984, *Proc. Natl. Acad. Sci. USA* 81:6851-6855; Neuberger et *al*., 1984, *Nature* 312:604-608; Takeda et *al*., 1985, *Nature* 314:452-454) may be used. A chimeric antibody is a molecule comprising portions from different animal species, such as those having a variable region derived from a murine mAb and a human immunoglobulin constant region. A chimeric antibody may be generated, *e.g.*, by splicing the genes from a mouse antibody molecule of appropriate antigen specificity together with genes from a human antibody molecule of appropriate biological activity.

Alternatively, techniques described for the production of a single chain antibody (*see*, U.S. Patent 4,946,778; Bird, 1988, *Science* 242:423-26; Huston *et al*., 1988, *Proc*. *Natl. Acad. Sci. USA* 85:5879-83; and Ward et *al.,* 1989, *Nature* 334:544-546) may be adapted to produce single chain antibodies against MC4R gene products. A single chain antibody is formed by linking the heavy and light chain fragments of the Fv region *via* an amino acid bridge, resulting in a single chain polypeptide.

An antibody fragment that recognizes a specific epitope may be generated by known techniques. For example, the antibody fragment may be, *e.g.,* one of the F(ab')₂ fragments that results from pepsin digestion of the antibody molecule, or one of the Fab fragments that is generated by reducing the disulfide bridges of the F(ab')₂ fragments. Alternatively, a Fab expression library may be constructed (*see*, Huse *et al.,* 1989, *Science* 246:1275-1281) to allow rapid and easy identification of a monoclonal Fab fragment with the desired specificity.

An antibody to the MC4R may, in turn, be utilized to generate an anti-idiotype antibody that "mimics" MC4R, using techniques well known to those skilled in the art (see, *e*.*g*., Greenspan and Bona, 1993, *FASEB J.* 7:437-444; and Nissinoff, 1991, *J. Immunol.* 147:2429-2438). For example, an antibody that binds to the MC4R ECD and competitively inhibits the binding of a melanocortin to MC4R may be used to generate an anti-idiotype that "mimics" the ECD and, therefore, binds to and neutralizes melanocortins. The neutralizing anti-idiotype (or Fab fragments of the anti-idiotype) can be used in a therapeutic regimen to neutralize the native ligand and promote increased appetite in a subject animal.

Alternatively, an antibody to MC4R that acts as an agonist of MC4R may be generated. The antibody binds to MC4R and activates its signal transducing activity. The antibody is particularly useful for treating appetite-related disorders such as obesity in a subject animal. In addition, an antibody that acts as antagonist of MC4R, *i*.*e*. that inhibits the activation of MC4R receptor, may be used to treat appetite-related disorders such as anorexia or cachexia in a subject animal.

### The Treatment Of Appetite-Related Disorders Using The Compounds Identified By The Methods Of The Invention

The invention encompasses methods and compositions for modifying appetite and/or metabolic rate and treating appetite- and/or metabolic rate-related disorders in animals, including but not limited to obesity, cachexia, anorexia, reproductive incompetence, endotoxemia, fever, renal failure, hepatic lipidosis, weaning-induced inappetance and growth lag, cancer, infection, inflammation and lactation. Because a loss of normal MC4R function results in the development of an obese phenotype, an increase in MC4R activity, or activation of the MC4R pathway (*e.g.*, downstream activation) would facilitate progress towards a normal body weight state in obese animals exhibiting a deficient level of MC4R gene expression and/or MC4R activity. Alternatively, symptoms of certain disorders such as, for example, cachexia, which include inappetence and perhaps a lower than normal body weight phenotype, may be ameliorated by decreasing the level of MC4R gene expression, and/or MC4R gene activity, and/or downregulating activity of the MC4R pathway (*e.g.*, by targeting downstream signaling events). Different approaches are discussed below.

In one embodiment, a compound that modulates MC4R activity is administered to a subject animal, preferably a mammal or a bird, in need thereof. The subject animal may, *e*.*g*., suffer from one of the diseases or conditions listed above. The compound may be an agonist or an antagonist of MC4R. An agonist of MC4R may be used to reduce food intake and/or increase metabolic rate to induce weight loss for treating obesity in a subject animal. In a preferred embodiment, a preparation comprising an MC4R agonist that induces safe, effective appetite reduction is administered to an animal in need thereof. In particularly preferred embodiments, the animal is a dog or a cat, the weight loss is between 4-8% of the excess weight of the animal per month, and/or the preparation is administered orally. An antagonist of MC4R activity may be used to induce increased appetite for treating conditions such as anorexia or cachexia. In a preferred embodiment, a preparation comprising an MC4R antagonist that acutely stimulates the appetite is administered to an animal in need thereof. In a particularly preferred embodiment, the animal is a dog or a cat suffering from pathology that results in inappropriately low food intake and weight loss (*e.g.*, hepatic lipidosis or cachexia).

It is not necessary that the compound demonstrate absolute specificity for the MC4R. For example, a compound that agonizes both MC4R and MC1R could be used; the compound could be administered so that delivery to the brain is optimized to achieve reduced appetite and weight reduction, and side effects, such as peripheral melanin production resulting in the skin, hide or fur, are reduced to a tolerable level. A compound that does not demonstrate a specificity for MC4R may be administered in conjunction with another therapy or drug to control the side-effects that may result from modulating another melanocortin receptor; however, a compound that demonstrates a preference or selectivity for MC4R over MC3R is preferred since both receptors are expressed in the brain where localized delivery cannot be used to compensate for lack of receptor specificity.

Toxicity and therapeutic efficacy of the compound can be determined by standard pharmaceutical procedures in cell cultures or experimental animals, such as rats and mice, *e.g.*, for determining the LD₅₀ (the dose lethal to 50% of the population) and the ED₅₀ (the dose therapeutically effective in 50% of the population). The dose ratio between toxic and therapeutic effects (*i*.*e*. the ratio LD₅₀/ED₅₀) is the therapeutic index. A compound that exhibits a large therapeutic index is preferred. While a compound that exhibits toxic side effects may be used, care should be taken to design a delivery system that targets the compound to the site of affected tissue in order to minimize potential damage to uninfected cells and, thereby, reduce side effects.

The data obtained from the cell culture assays and experimental animal studies for a compound may be used in formulating a dosage range for use of the compound in a subject animal, such as a cat, dog or livestock. The dosage of the compound lies preferably within a range of circulating concentrations that include the ED₅₀ with little or no toxicity. The dosage may vary within this range depending upon the dosage form employed and the route of administration utilized. The therapeutically effective dose of the compound may be estimated initially from cell culture assays. A dose may be formulated in experimental animal models to achieve a circulating plasma concentration range that includes the IC₅₀ (*i*.*e*., the concentration of the test compound which achieves a half-maximal inhibition of symptoms) as determined in cell culture. Such information may be used to more accurately determine a useful dose in a subject animal. Levels of the compound in plasma may be measured, for example, by high performance liquid chromatography.

### Antisense Oligonucleotides Used For Inhibiting The Expression Of MC4R

In yet another aspect of the invention, a nucleic acid molecule is used to inhibit the expression of a component of the MC4R signal transduction pathway, thereby modulating the appetite of a subject animal. In one embodiment, a therapy is designed wherein the level of endogenous MC4R gene expression in the subject animal is reduced by using an antisense nucleic acid or a ribozyme to inhibit or prevent translation of MC4R mRNA transcripts; a nucleic acid that forms a triple helix with all or part of the MC4R gene or its regulatory elements to inhibit transcription of the MC4R gene; or a nucleic acid useful for targeted homologous recombination to inactivate or "knock out" the MC4R gene or its endogenous promoter. The therapy may be utilized for treatment of appetite and body weight disorders in the animal subject such as cachexia and anorexia where the inhibition of MC4R expression is designed to increase appetite. Because the MC4R gene is expressed in the brain, delivery techniques preferably should be designed to allow the nucleic acid to cross the blood-brain barrier *(see,* PCT WO89/10134, which is incorporated by reference herein in its entirety). For example, the nucleic acids can be modified, or appropriately formulated, to increase their ability to cross the blood-brain barrier. Alternatively, the antisense, ribozyme or nucleic acid constructs described herein could be administered directly to the site containing the target cells.

An antisense approach involves the design of an oligonucleotide that is complementary to an mRNA. The oligonucleotide may comprise any suitable polymeric molecule, e.g., DNA, RNA, a DNA/RNA hybrid, modified DNA or RNA, or a synthetic DNA or RNA analog (e.g., peptide nucleic acid (hereinafter "PNA"); *see,* WO 92/20702), as described more fully below. The antisense oligonucleotide will bind to the complementary mRNA transcripts and prevent translation. Absolute complementarity, although preferred, is not required. A sequence "complementary" to a portion of an RNA, as referred to herein, means a sequence having sufficient complementarity to be able to hybridize with the RNA, forming a stable duplex; in the case of a double-stranded antisense nucleic acid, a single strand of the duplex DNA thus may be tested, or triplex formation may be assayed. The ability to hybridize will depend on both the degree of complementarity and the length of the antisense oligonucleotide. Generally, the longer the hybridizing oligonucleotide, the more base mismatches with the RNA it may contain and still form a stable duplex (or triplex, as the case may be). One skilled in the art can ascertain a tolerable degree of mismatch by use of standard procedures to determine the melting point of the hybridized complex.

While antisense oligonucleotides complementary to the coding region sequence may be used, those complementary to the transcribed untranslated region are most preferred. Oligonucleotides that are complementary to the 5' end of the message, *e.g.*, the 5' untranslated region up to and including the AUG initiation codon, should work most efficiently at inhibiting translation (*see*, Figures 1 and 2). However, sequences complementary to the 3' untranslated sequences of mRNAs recently have proven to be effective at inhibiting translation of mRNA as well. *See, generally,* Wagner, 1994, *Nature* 372:333-335. Thus, an oligonucleotide complementary to either the 5'- or 3'- non- translated, non-coding region of MC4R may be used in an antisense approach to inhibit translation of endogenous mRNA. An oligonucleotide complementary to the 5' untranslated region of the mRNA preferably includes the complement of the AUG start codon. An antisense oligonucleotide complementary to mRNA coding regions is a less efficient inhibitor of translation but may be used in accordance with the invention. Whether designed to hybridize to the 5'-, 3'- or coding region of MC4R mRNA, an antisense oligonucleotide should be at least six nucleobases in length, and preferably ranging from 6 to about 50 nucleobases in length. A nucleobase is a monomer unit from which the oligonucleotide is comprised (*e.g.*, for DNA and RNA oligonucleotides, a nucleobase is a nucleotide). In specific aspects the oligonucleotide is at least 10 nucleobases, at least 17 nucleobases, at least 25 nucleobases or at least 50 nucleobases.

### Ribozymes

A ribozyme molecule designed to catalytically cleave MC4R mRNA transcripts also may be used to prevent translation of MC4R mRNA and expression of MC4R in a subject animal (*see, e.g.*, PCT International Publication WO90/11364, published October 4, 1990; Sarver *et al.,* 1990, *Science* 247:1222-1225). While a ribozyme that cleaves mRNA at site specific recognition sequences can be used to destroy MC4R mRNAs, the use of a hammerhead ribozyme is preferred. A hammerhead ribozyme cleaves mRNAs at locations dictated by flanking regions that form complementary base pairs with the target mRNA. The sole requirement is that the target mRNA have the following sequence of two bases: 5'-UG-3'. The construction and production of a hammerhead ribozyme is well known in the art and is described more fully in Haseloff and Gerlach, 1988, *Nature* 334:585-591. There are hundreds of potential hammerhead ribozyme cleavage sites within the nucleotide sequences of feline and canine MC4R cDNA (*see*, Figures 1 and 2). Preferably the ribozyme is engineered so that the cleavage recognition site is located near the 5' end of the MC4R mRNA; *i*.*e*., to increase efficiency and minimize the intracellular accumulation of non-functional mRNA transcripts.

The ribozymes of the present invention also include RNA endoribonucleases (hereinafter "Cech-type ribozymes") such as the one which occurs naturally in Tetrahymena Thermophila (known as the IVS, or L-19 IVS RNA) and which has been extensively described by Thomas Cech and collaborators (*see*, Zaug *et al.,* 1984, *Science* 224:574-578; Zaug and Cech, 1986, *Science* 231:470-475; Zaug *et al.,* 1986, *Nature* 324:429-433; published International patent application No. WO 88/04300 by University Patents Inc.; Been and Cech, 1986, *Cell* 47:207-216). A Cech-type ribozyme has an eight base pair active site which hybridizes to a target RNA sequence whereafter cleavage of the target RNA takes place. The invention encompasses those Cech-type ribozymes which target eight base-pair active site sequences that are present in MC4R.

As in the antisense approach, the ribozymes can be composed of modified oligonucleotides (*e*.*g*. for improved stability, targeting, etc.) and should be delivered to cells which express the MC4R *in vivo, e.g.*, hypothalamus. For example, the ribozyme can be modified, or appropriately formulated, to cross the blood-brain barrier (*see*, PCT WO89/10134, which is incorporated by reference herein in its entirety). A preferred method of delivery involves using a DNA construct encoding the ribozyme under the control of a strong constitutive pol III or pol II promoter, so that transfected cells will produce sufficient quantities of the ribozyme to destroy endogenous MC4R messages and inhibit translation. Because ribozymes, unlike antisense molecules, are catalytic, a lower intracellular concentration is required for efficiency.

### Transgenic Animals

In another aspect of the invention, transgenic animals are generated that exhibit altered appetite regulation and body weight control compared to their *wild type* counterparts. Specifically, in such animals, the expression of MC4R is controlled *in vivo, e.g.*, at the transcriptional or translational level. Preferably, the transgenic animal is a mammal, *e.g.*, a dog, a cat, a cow, a horse, a sheep, a goat, or a pig. Certain approaches are described below.

With respect to an increase in the level of normal MC4R gene expression and/or MC4R gene product activity, canine or feline MC4R nucleic acid sequences can be utilized to generate transgenic animals less prone to suffer from appetite and body weight disorders, including obesity.

Alternatively, targeted homologous recombination can be utilized to correct a defective endogenous MC4R gene in the appropriate tissue of an animal subject; *e*.*g*., brain tissue. Targeted homologous recombination can be used to correct the defect in ES cells in order to generate offspring with a corrected trait.

In another alternative, endogenous MC4R gene expression in an animal subject can be reduced by inactivating or "knocking out" the MC4R gene or its promoter using targeted homologous recombination. Smithies *et al*., 1985, *Nature* 317:230-234; Thomas and Capecchi, 1987, *Cell* 51:503-512; Thompson *et al*., 1989, *Cell* 5:313-321; each of which is incorporated by reference herein in its entirety. For example, a mutant, non-functional MC4R (or a completely unrelated DNA sequence) flanked by DNA homologous to the endogenous MC4R gene can be used, with or without a selectable marker and/or a negative selectable marker, to transfect cells that express MC4R *in vivo.* Insertion of the DNA construct, via targeted homologous recombination, results in inactivation of the MC4R gene. This approach is particularly suited for agricultural applications where modifications to embryonic stem cells (hereinafter "ES cells") can be used to generate animal offspring with an inactive MC4R. *See, e*.*g*., Thomas and Capecchi and Thompson, 1987, *supra.* However this approach may be adapted for use in companion animals such as cats and dogs.

Alternatively, endogenous MC4R gene expression in an animal subject may be reduced by targeting deoxyribonucleotide sequences complementary to the regulatory region of the MC4R gene (*i*.*e*., the MC4R promoter and/or enhancers) to form triple helical structures that prevent transcription of the MC4R gene in target cells in the body. See, *generally* Helene, 1991, *Anticancer Drug* Des. 6:569-584; Helene et *al.,* 1992, Ann. *N*.*Y*. *Acad. Sci.* 660:27-36; and Maher, 1992, *Bioassays* 14:807-815.

Genetically engineered cells that express soluble MC4R ECDs or fusion proteins, *e*.*g*. fusion Ig molecules, may be administered *in vivo* where they may function as "bioreactors" that deliver a supply of the soluble molecules. Such soluble MC4R polypeptides and fusion proteins, when expressed at appropriate concentrations, should neutralize or "mop up" the native ligand for MC4R, and thus act as inhibitors of MC4R activity and induce appetite and perhaps weight gain in the subject animal.

### Pharmaceutical Formulations And Methods Of Administration

A pharmaceutical composition comprising the MC4R agonists and antagonists of the invention for use in accordance with the present invention may be formulated in conventional manner. The pharmaceutical composition comprises a compound that modulates MC4R activity (as described above) and one or more physiologically acceptable carriers or excipients. The carriers or excipients are selected according to the method of administration to be used. The compound (and its physiologically acceptable salts and solvates) may be formulated, *e.g.*, for administration by inhalation or insufflation (either through the mouth or the nose) or oral, buccal, parenteral, topical, transdermal or rectal administration.

For oral administration, the pharmaceutical composition may take the form of, for example, tablets or capsules prepared by conventional means with pharmaceutically acceptable excipients such as a binding agent (*e.g.*, pregelatinised maize starch, polyvinylpyrrolidone or hydroxypropyl methylcellulose); a filler (*e.g.*, lactose, microcrystalline cellulose or calcium hydrogen phosphate); a lubricant (*e*.*g*., magnesium stearate, talc or silica); a disintegrant (*e.g.*, potato starch or sodium starch glycolate); or a wetting agent (*e.g.*, sodium lauryl sulphate). The tablets may be coated by methods well known in the art. A liquid preparation for oral administration may take the form of, for example, a solution, syrup or suspension, or it may be presented as a dry product for constitution with water or other suitable vehicle before use. The liquid preparation may be prepared by conventional means with a pharmaceutically acceptable additive such as a suspending agent (*e*.*g*., sorbitol syrup, cellulose derivatives or hydrogenated edible fats); emulsifying agent (*e*.*g*., lecithin or acacia); non-aqueous vehicle (*e*.*g*., almond oil, oily esters, ethyl alcohol or fractionated vegetable oils); or preservative (*e*.*g*., methyl or propyl-p-hydroxybenzoates or sorbic acid). The preparation also may contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the compound is conveniently delivered in the form of an aerosol spray presentation from a pressurized pack or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.*, gelatin for use in an inhaler or insufflator may be formulated containing a powder mix of the compound and a suitable powder base such as lactose or starch.

The compound may be formulated for parenteral administration by injection, *e*.*g*., by bolus injection or continuous infusion. A formulation for injection may be presented in unit dosage form, *e*.*g*., in ampoules or in multi-dose containers, with an added preservative. The formulation may take the form of a suspension, solution or emulsion in an oily or aqueous vehicle, and may contain formulatory agents such as suspending, stabilizing and/or dispersing agents. Alternatively, the compound may be in powder form for constitution with a suitable vehicle, *e.g.*, sterile pyrogen-free water, before use.

The compound may also be formulated in a rectal composition such as a suppository or retention enema, *e.g.*, containing a conventional suppository base such as cocoa butter or other glyceride.

In addition to the formulations described previously, the compound also may be formulated as a long-acting depot preparation. The preparation may be administered by implantation (*e.g.*, subcutaneously or intramuscularly), by intramuscular injection or by a transdermal patch. Thus, *e.g.*, the compound may be formulated with a suitable polymeric or hydrophobic material (*e.g.*, as an emulsion in an acceptable oil) or ion exchange resin, or as a sparingly soluble derivative, *e.g.*, as a sparingly soluble salt.

The composition may, if desired, be presented in a pack or dispenser device that may contain one or more unit dosage forms containing the active ingredient. The pack may comprise, *e.g.*, metal or plastic foil, such as a blister pack. The pack or dispenser device may be accompanied by instructions for administration.

In another aspect of the invention, a system described above may be formulated into a kit. To this end, the MC4R or cells expressing the MC4R can be packaged in a variety of containers, *e.g.*, vials, tubes, microtiter well plates, bottles, and the like. Other reagents can be included in separate containers and provided with the kit; *e.g.*, positive controls samples, negative control samples, melanocortin peptides (including but not limited to α-MSH and ACTH derivatives), buffers, cell culture media, etc.

### Example 1

The following example describes the isolation of the nucleic acids encoding the feline and canine melanocortin receptor proteins disclosed herein.

In separate experiments, feline and canine hypothalamic tissues were prepared. cDNA libraries were prepared therefrom by Lifetechnologies Inc. (Rockville, MD). A biotinylated capture oligonucleotide having the sequence ttgactctgtgatctgtagctccttgct was designed from a conserved region of the MC4R gene. Feline and canine MC4R clones were isolated from their corresponding cDNA libraries using this oligonucleotide and the GENETRAPPER® system (Lifetechnologies Inc.). Positive selection was achieved by stringently hybridizing this oligonucleotide to clones in a cDNA library. The complex was separated from all other clones in the library using Streptaviden magnetic beads which were pelleted via magnet. MC4R clones were identified by PCR using a MC4R specific primer pair designed outside of the capture oligonucleotide site (forward primer: atgaggcagatgatgacagc; reverse primer: gtgatctgtagctccttgc). Six feline and one canine MC4R clones were isolated from the cDNA libraries. The clones ranged in size from 1.7 to 2.2 kilobases. Identity of the clones was confirmed by PCR using a different MC4R gene-specific primer pair (forward primer: tgagacatgaagcacac; reverse primer: gtgatctgtagctccttgc).

Sequencing of the feline and canine MC4R genes was completed using one standard and three primer walking reactions from both ends of each clone. Sequences of the MC4R gene fragments were assembled based on their overlapping regions.

The nucleotide sequences of the feline and canine MC4R genes are shown in Figures 1 and 2 (SEQ ID NOs: 1 and 2), respectively. Conceptual translation of the open reading frame of each nucleotide sequence revealed the predicted amino acid sequence of feline and canine MC4R, shown in Figures 3 and 4 (SEQ ID Nos: 4 and 5), respectively.

The feline and canine MC4R nucleotide sequences have been compared with each other and with all other available MC4R gene sequences from other species. The feline and canine MC4R nucleotide sequences were used as queries to search the public sequence databases. Many hits were obtained which include the MC4R genes of human, rat, etc. The DNA sequence, the open reading frame (ORF) and protein sequence identities between the queries and the hits were analyzed using the Jotun Hein method of the LASERGENE-MEGALIGN™ software package (DNASTAR, Inc., Madison, WI) to determine divergence. The Jotun Hein method builds a phylogenetic tree by examining sequence pairs and creating the best possible arrangement of ancestral branches. The method is most useful when aligned sequences are related by descent. The following TABLES I, II, and III are the result of the query.

**TABLE I**

| **PERCENTAGE IDENTITIES OF MC4R NUCLEOTIDE SEQUENCE AMONG DIFFERENT SPECIES** | | | | | | |
|---|---|---|---|---|---|---|
| | 1 Human | 2 Porcine | 3 Rat | 3 Chicken | 4 Canine | 5 Feline |
| . Human | 100 | 86.7 | 77.5 | 65.7 | 81.2 | 86.3 |
| . Porcine | | 100 | 73.8 | 60.7 | 81.3 | 87.4 |
| . Rat | | | 100 | 66.1 | 72.8 | 78.5 |
| . Chicken | | | | 100 | 61.8 | 66.0 |
| . Canine | | | | | 100 | 86.3 |
| . Feline | | | | | | 100 |

**TABLE II**

| **PERCENTAGE IDENTITIES OF MC4R OPEN READING FRAME AMONG DIFFERENT SPECIES** | | | | | | |
|---|---|---|---|---|---|---|
| | 1 Human | 2 Porcine | 3 Rat | 3 Chicken | 4 Canine | 5 Feline |
| 1.Human | 100 | 92.0 | 88.4 | 80.9 | 89.4 | 92.8 |
| 2. Porcine | | 100 | 88.2 | 80.0 | 90.1 | 92.0 |
| 3. Rat | | | 100 | 78.6 | 87.1 | 88.2 |
| 3. Chicken | | | | 100 | 79.7 | 81.1 |
| 4. Canine | | | | | 100 | 91.5 |
| 5. Feline | | | | | | 100 |

**TABLE III**

| **PERCENTAGE IDENTITIES OF MC4R AMINO ACID SEQUENCE AMONG DIFFERENT SPECIES** | | | | | | |
|---|---|---|---|---|---|---|
| | 1 Human | 2 Porcine | 3 Rat | 3 Chicken | 4 Canine | 5 Feline |
| 1. Human | 100 | 95.2 | 92.8 | 87.0 | 94.6 | 95.8 |
| 2. Porcine | | 100 | 94.0 | 88.0 | 96.1 | 98.2 |
| 3. Rat | | | 100 | 86.7 | 93.7 | 95.2 |
| 3. Chicken | | | | 100 | 86.7 | 87.7 |
| 4. Canine | | | | | 100 | 97.6 |
| 5. Feline | | | | | | 100 |

### Example 2

The following example was done to determine and compare the ability of feline, canine and human MC4R to bind MSH.

Transfected cell lines, each expressing a different canine or feline MC4R clone were constructed. Specifically, for each cell line, HEK293 cells grown to 50% confluence were transiently transfected with a vector construct comprising the MC4R gene from one species. 4 µl FuGENE 6™, a transfection carrier, was used per µg plasmid DNA.

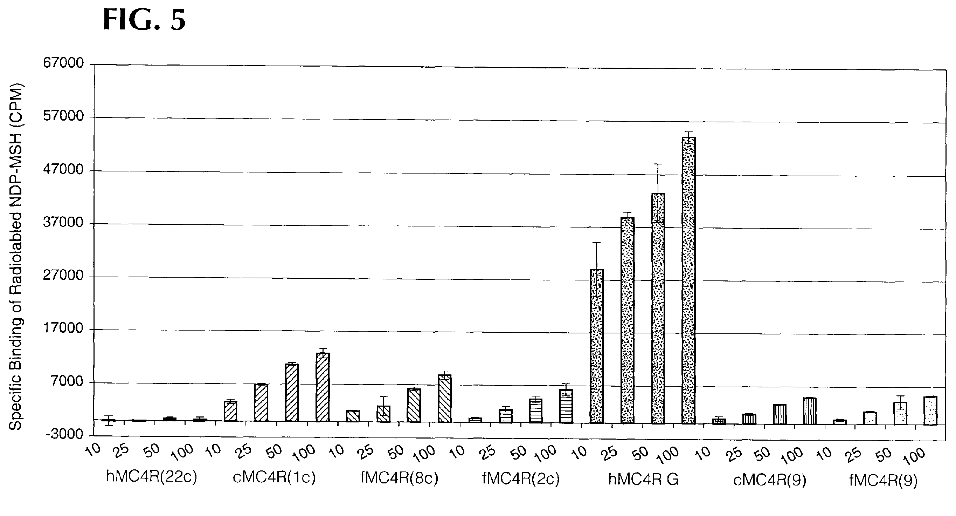

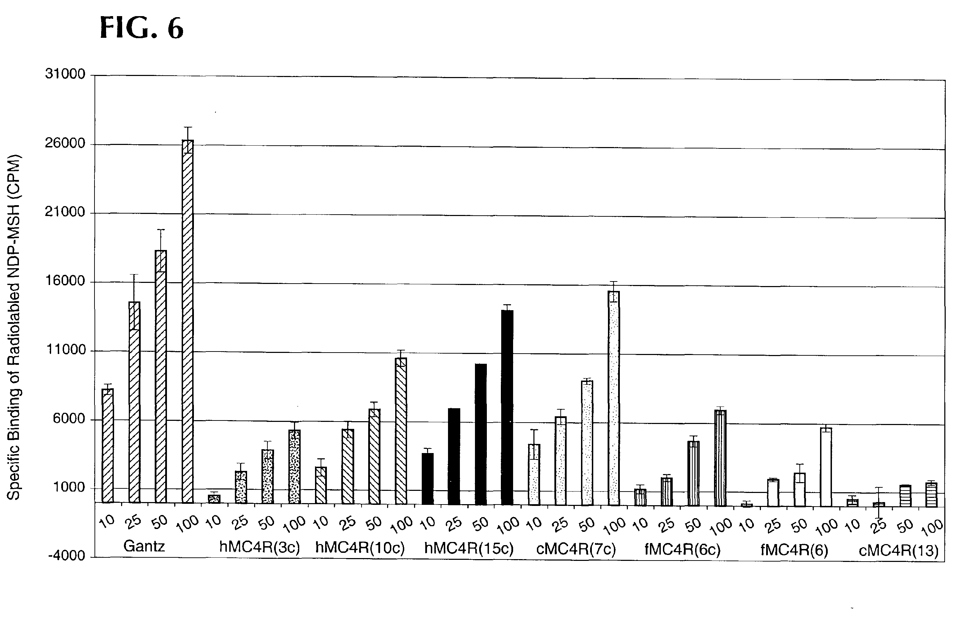

Cells were harvested 48 hours post-transfection using Sigma Dissociation Buffer, centrifuged and resuspended in Binding Buffer (50 mM HEPES, 5 mM MgCI, 0.1% BSA, and a protease inhibitor cocktail (Sigma P-8340™), pH=7.5). Whole cell binding was performed as a quick verification that the cell lines expressed melanocortin receptors. The resuspended cell population was counted using a hemacytometer. A cell volume titration was performed by pipetting varying volumes in triplicate into reaction tubes. Either buffer or excess non-radiolabeled NDP-MSH to a final concentration of 2 µM was added, and excess non-radiolabeled NDP-MSH was used to ascertain non-specific binding. Next, radiolabled NDP-MSH was added to a final concentration of 75 pM. The reaction was incubated at 37°C for 1 hour, then centrifuged at 5000xG for 10 minutes. The supernatant was aspirated and the amount of pelleted radiolabled NDP-MSH measured using a gamma counter. The results are shown in Figures 5 and 6.

Because whole cell binding assays cannot be used to accurately determine the number of receptors per cell (due to receptor endocytosis and recycling), membrane preparations were prepared from each cell line. Transfected cells were harvested cells using Sigma dissociation buffer, centrifuged and resuspended in ice cold homogenization buffer (1mM EDTA, 1mM EGTA, 10 mM HEPES and a protease inhibitor cocktail (Sigma P-8340™), pH=7.5). The cell resuspension was incubated on ice for at least 10 minutes then homogenized with 20 strokes of a tight fitting glass/glass dounce homogenizer. The homogenate was then centrifuged at 1000xG for 10 minutes at 4°C to pellet nuclei and unlysed cells. The supernatant was transferred to a new tube and centrifuged at 25000xG for 20 minutes at 4°C to pellet the plasma membrane. The supernatant was discarded. The pellet was resuspended in homogenization buffer in order to wash the plasma membrane. The resuspended pellet was homogenized with 20 strokes of a tight fitting glass/glass dounce homogenizer, then centrifuged at 25000xG for 20 minutes at 4°C. The supernatant was discarded and the pellet resuspended in a homogenization buffer to a protein concentration of 1-5 mg/ml. 500 µl aliquots were frozen at -70°C for long term storage. Protein concentration was determined by diluting the homogenate 5-10 fold and using the Pierce BCA kit (Pierce, Rockford, IL).

### Example 3

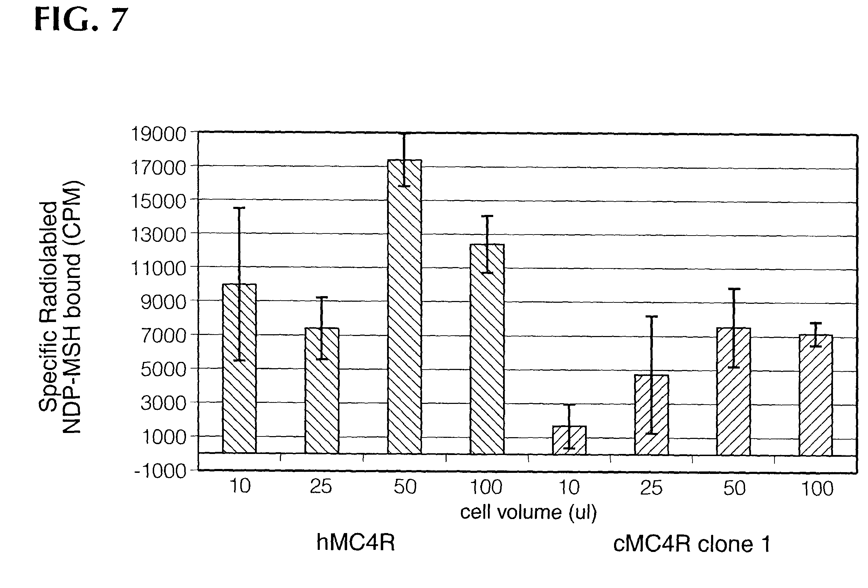

The following was done to determine the relative affinity of cMC4R, hMC3R and hMC4R for MSH and other MC4R ligands. HEK 293 cell lines expressing cMC4R, hMC3R or hMC4R were grown at 37°C, with 5% CO₂, in DMEM (Dulbecco's modified eagle medium; high glucose, with L-glutamine, 110 mg/L sodium pyruvate and pyridoxine hydrochloride) (Gibco BRL, Rockville, MD), supplemented with 10% fetal bovine serum, 100 units/ml penicillin, 100µg/ml streptomycin, and 300 µg/ml geneticin (all obtained from Gibco BRL). A whole cell binding assay was performed on each cell line as a quick verification that it expressed a melanocortin receptor. Cells from each cell line were harvested using Sigma Dissociation Buffer and washed with PBS. Cells were resuspended in Binding Buffer and counted using a hemacytometer. To determine specific binding, various amounts of cells were added in triplicate tubes with and without excess non-radiolabeled NDP-MSH at a final concentration of 5 mM. A final concentration of 50 pM ¹²⁵I-NDP-MSH was added to each tube and allowed to bind for 50 minutes at 37°C. Cells were pelleted by centrifugation, supernatant was removed and cell-bound radioactivity was counted on a Gamma counter. Results show that as the cell number increased, the specific binding to ¹²⁵I-NDP-MSH also increased in cMC4R cell lines (Figure 7) Because whole cell binding assays cannot accurately measure the number of receptor molecules per cell (due to receptor endocytosis and recycling) membrane preparations of each cell line were made. Cells were harvested using Sigma Dissociation Buffer, pelleted by centrifugation, reconstituted and washed with PBS, and pelleted by centrifugation. Total cell counts were performed by hemacytometry and recorded. The cell pellets were resuspended in homogenization buffer (25 mM HEPES, 1.5 mM CaCI, 1mM MgSO4, 100 mM NaCl, 10% Sucrose, Sigma Protease Inhibitor Cocktail). Homogenization was performed on ice with a Dounce homogenizer (10-15 strokes), followed by sonication (3x30 second bursts at high setting). Large cellular particulate matter was separated from the membrane fraction by centrifugation at 700xG. The supernatant fraction was then centrifuged at 150,000xG. The supernatant fraction from this centrifugation was discarded, and the membrane pellet was resuspended in 20 ml of homogenization buffer and stored in 0.5 ml aliquots at -70°C.

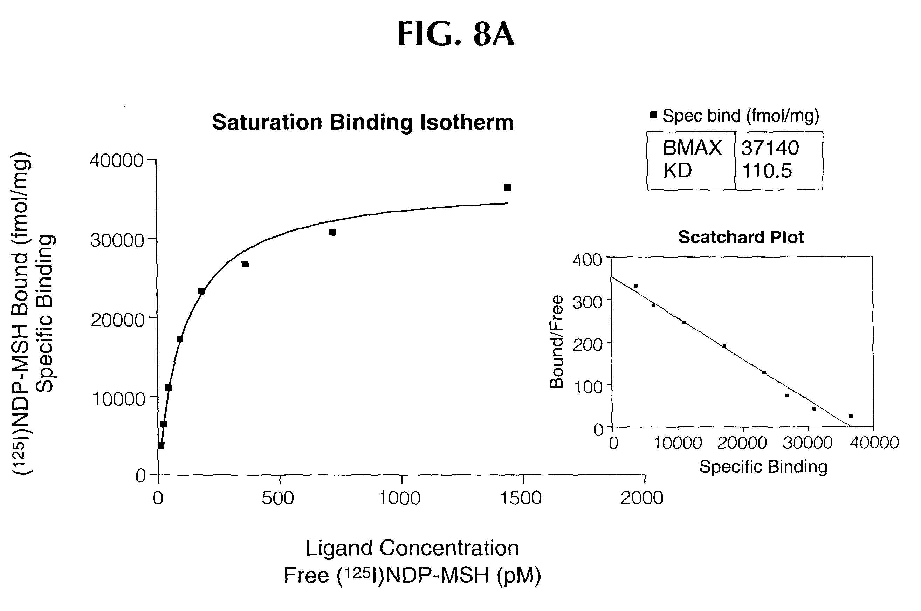

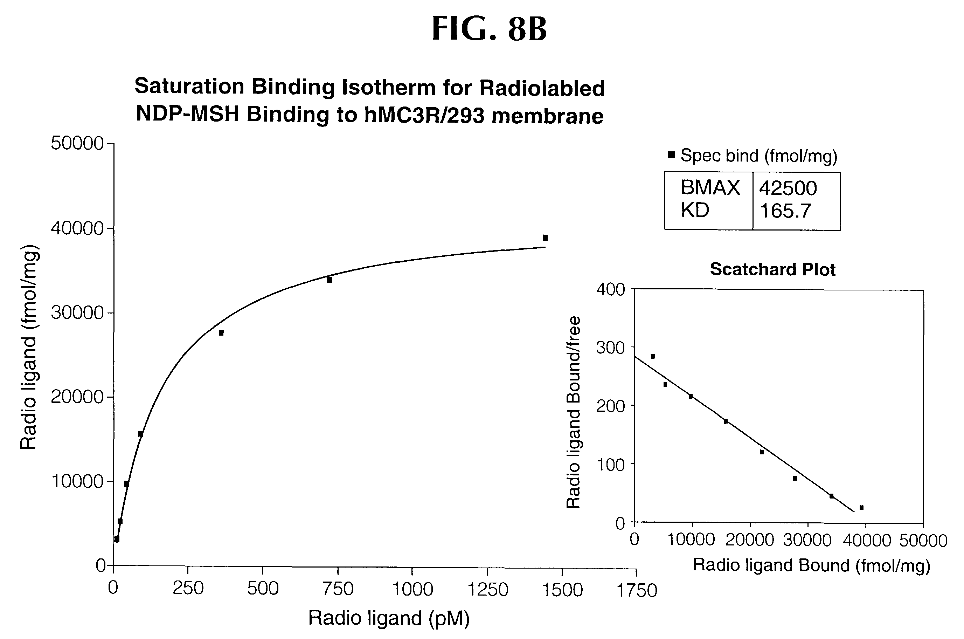

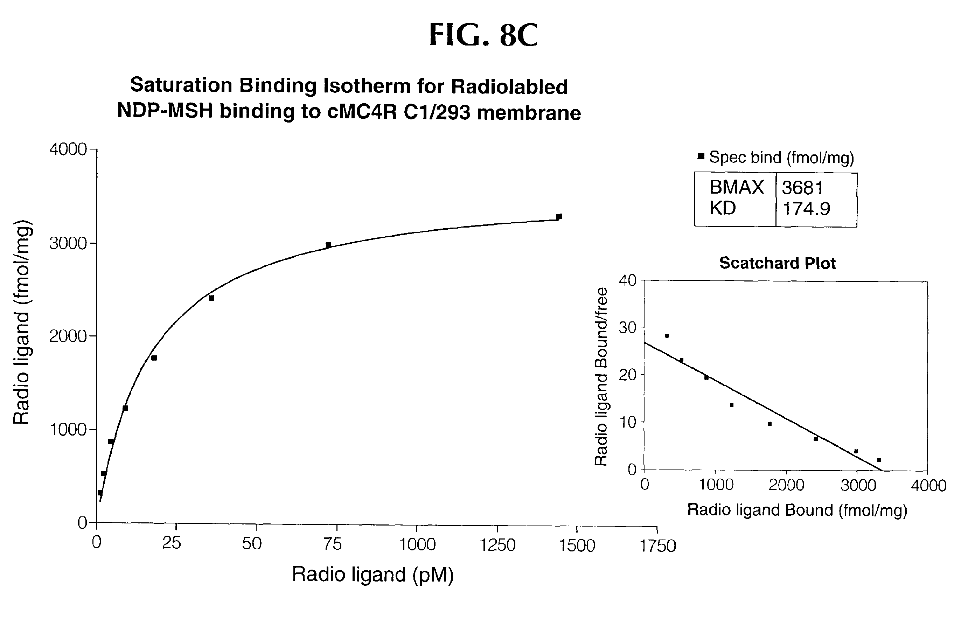

Saturation binding studies were performed on membrane preparations in order to investigate receptor number and ligand affinity, and for comparing current procedures with literature values. Protein concentrations were determined for each preparation using the BCA™ Protein Assay Kit in order to normalize the amount of membrane used in each assay. A constant amount of membrane, as measured by total peptide content, of each preparation was used for each assay (1 µg protein for hMC3R/HEK293 and hMC4R/HEK293; 2 µg protein for cMC4R C1/HEK293). Various amounts of ¹²⁵I-NDP-MSH were used in the assays to construct a saturation binding isotherm for each receptor. Transformed isotherms for cMC4R/HEK293, hMC3R/HEK293 and hMC4R/HEK293 membranes are shown in Figures 8A, 8B and 8C ([hMC4R/HEK293 membrane lot 1]; Figure 8A: Saturation Binding Isotherm of hMC4R/HEK293; Figure 8B: : Saturation Binding Isotherm of hMC3R/HEK293; Figure 8C: Saturation Binding Isotherm of CMC4R/HEK293). Scatchard analysis for the three membrane preparations indicated that the K_{d} for NDP-MSH is between 100-200 pM for each of these preparations (Figures 8A, 8B and 8C). Additionally, the linear Scatchard line, as calculated by GraphPad PRISM® (GraphPad Software Inc., San Diego, CA), indicates a single population of receptors for each cell line, as expected.

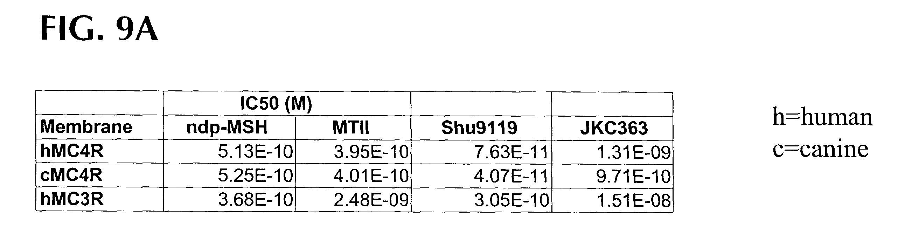

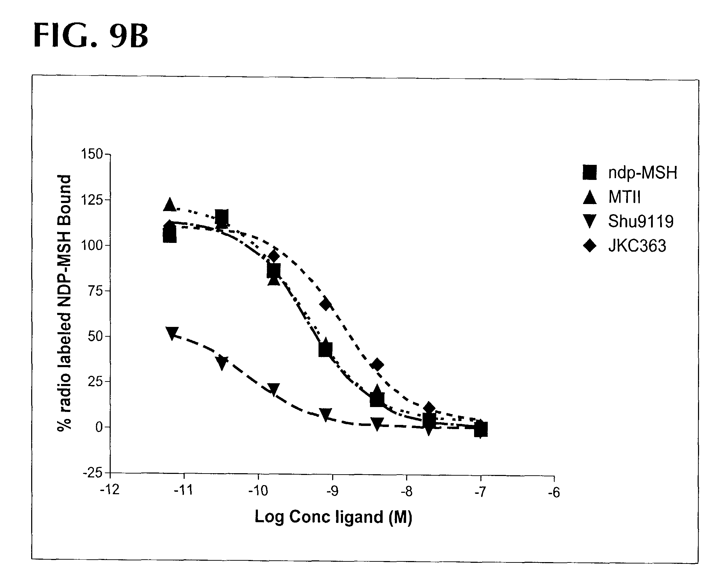

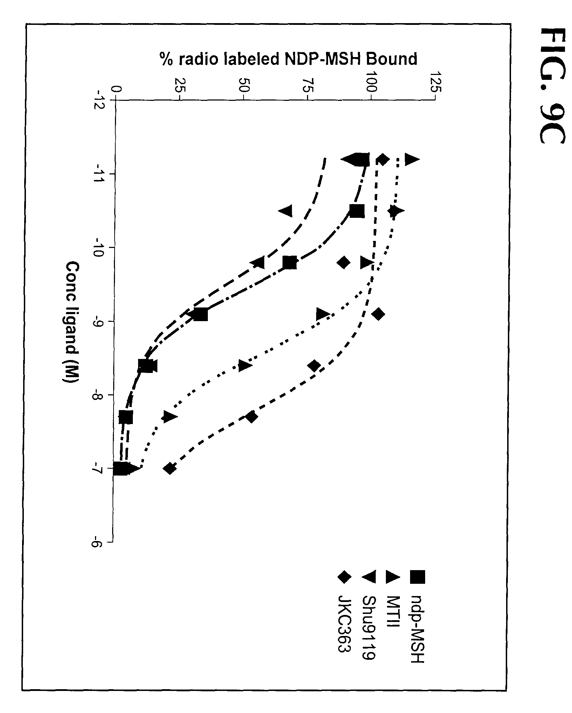

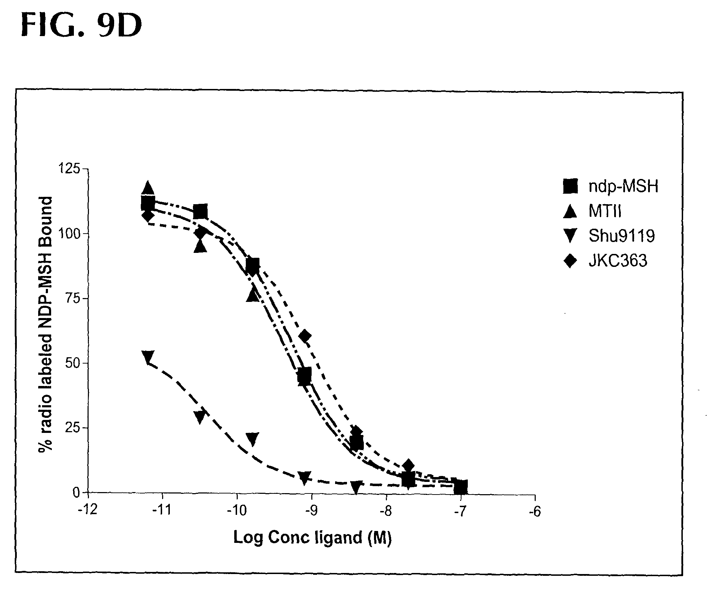

Using the same membrane preparations, binding profiles to commercially available ligands were performed. In an attempt to compensate for differences in receptor number/unit membrane protein between the membrane preparations, the amount of membrane preparation was adjusted to normalize receptor expression. For small molecule compounds, DMSO was used to solubilize each compound and then diluted to 0.2% with binding buffer. Titration curves follow are shown in Figure 9. IC₅₀ values were calculated using non-linear regression analysis provided by GraphPad PRISM®. The following TABLE IV summarizes the data.

**TABLE IV**

| IC₅₀ (nM) | | | | |
|---|---|---|---|---|
| Membrane | ndp-MSH | MTII | Shu9119 | JKC363 |
| hMC4R | 0.7766 | 0.8433 | 0.01163 | 2.112 |
| cMC4R | 0.8558 | 0.8774 | 0.009579 | 1.271 |
| hMC3R | 0.3973 | 4.582 | 0.1881 | 22.75 |

### Example 4

The following example was performed to measure the ability of canine MC4R to induce cAMP accumulation in response to α-MSH stimulation.

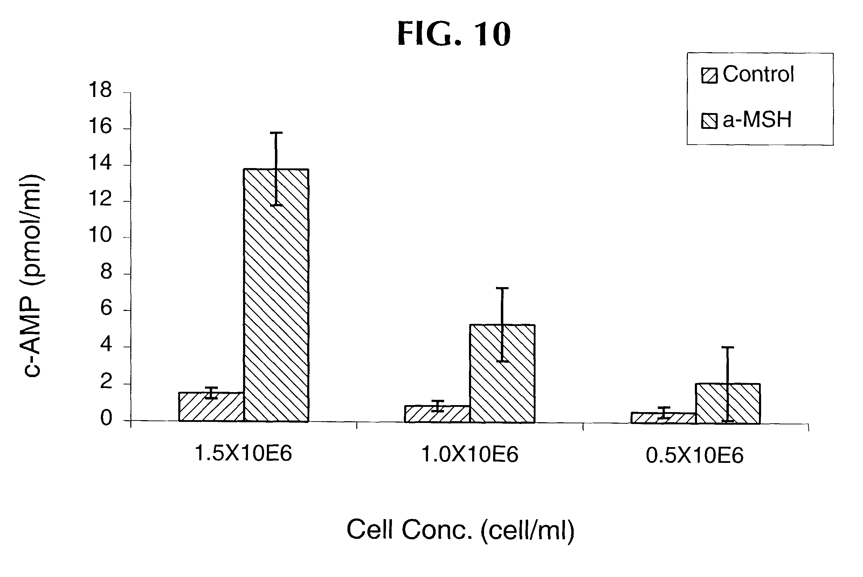

It has been shown in other species that MC4R is a G-protein coupled receptor that stimulates cAMP accumulation when stimulated by its agonist ligand. A commercially available kit (Adenylyl Cyclase Activation Flashplate Assay, NEN® Life Science Products, Inc., Boston, MA) was used to measure the accumulation of cAMP in cells transfected with canine MC4R after stimulation with its agonist ligand. An MC4R transfected cell line was prepared and harvested as described above. Harvested cells were then resuspended in Stimulation Buffer (provided in the kit). The number of resuspended cells was determined using a hemacytometer. The resuspended cells were centrifuged and resuspended again in Stimulation Buffer to three different final cell concentrations, 0.5x10⁶, 1.0x10⁶, and 1.5x10⁶ cells/ml in a 96-well plate. Cells at each concentration were contacted with either 1µM of the agonist α-MSH or a negative control. Incubation was terminated after 30 minutes by adding Detection Mix (provided in the kit). The plate was covered and incubated for 20 hours at room temperature. Stimulation of MC4R was determined by scintillation counting in a Wallac 1450 MICROBETA™ scintillation counter. α-MSH treated cells at each concentration showed a significant increase in cAMP levels over background (Figure 10). The effect was most pronounced in the most concentrated batch of cells, where α-MSH treated cells had a concentration of cAMP of 13.82 pmol/ml, compared to 1.53 pmol/ml for the negative control cells, a difference of almost ten-fold.

### Example 5

The following example was performed to demonstrate the feasibility of using a functionally-based high-throughput screen for identifying agonists or antagonists of MC4R using an MC4R coupled to the PLC pathway *via* a chimeric or promiscuous G-protein.

A HEK293a stable cell line expressing cMC4R was grown in culture medium (DMEM, 10% FBS, 100 units Penicillin/Streptomycin, 300 mg/ml GENETICIN® (GIBCO BRL, Rockville MD)), transfected with vectors carrying two different G-protein genes, Gα15 and Gα16 (see Figures 12A, 12B, 12C and 13A, 13B, 13C), and selected for stable incorporation of these DNA using a cell medium solution containing 300µg/ml of ZEOCIN™ (Invitrogen, Carlsbad, CA), over a four week period. Alternatively, a vector containing a chimeric G-protein (e.g. Gaqi5, a Gα with the last 5 amino acids replaced with those from Gαs) can be generated and used. Single clonal colonies were selected and expanded using cloning cylinders (Sigma-Aldrich Co., St. Louis, MO). Individual clones were tested for the receptor's ability to couple with the incorporated promiscuous G-proteins using a FLIPR® assay, as described below. Gα15 and Gα16 are known as promiscuous G-proteins because of their ability to functionally couple to any G-Protein receptor and transduce signaling to increase intracellular calcium. A FLIPR® machine (Molecular Devices, Sunnyvale CA) allows one to quantify such a calcium signaling cascade using fluorescent dyes available commercially.

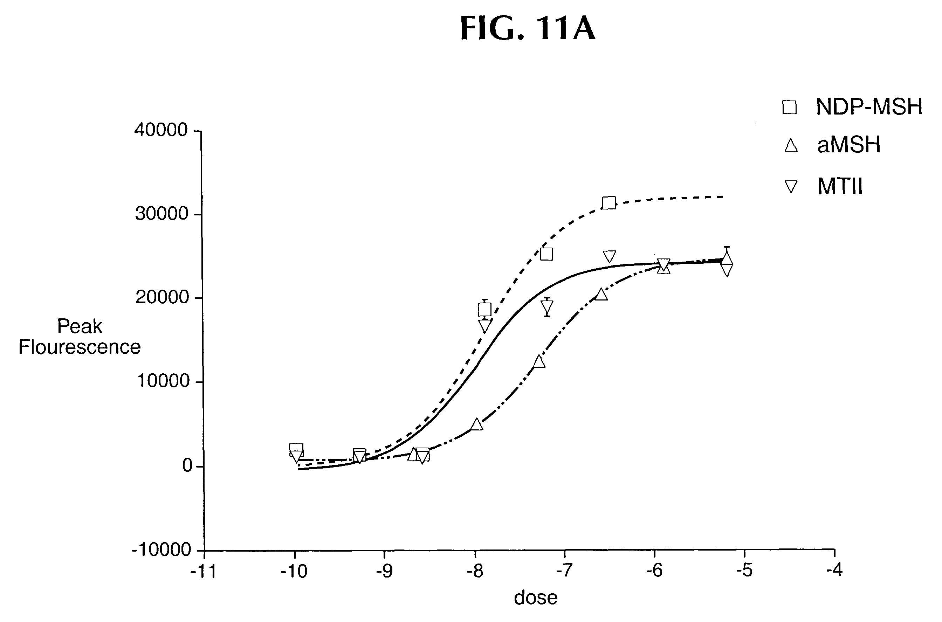

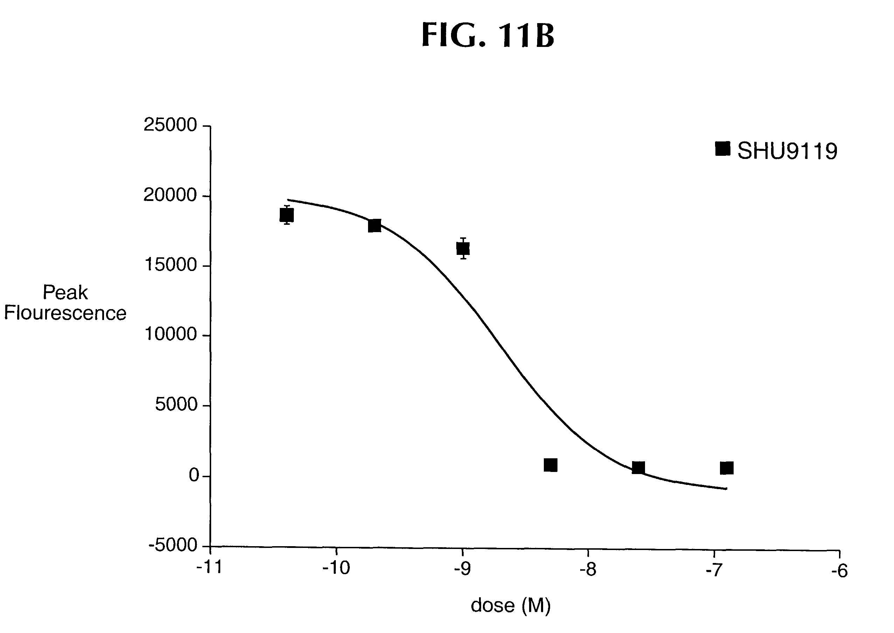

HEK293a/cMC4R/Gα15, Gα16 were grown to confluence and harvested with Trypsin-EDTA (GIBCO-BRL, Rockville MD). Cells were resuspended in fresh culture medium containing 300 µg/ml ZEOCIN™. The cell suspension was counted using a hemacytometer and approximately 50,000 cells per well were added to poly-d-lysine-coated black/clear 96 well plates (Becton Dickinson Labware, Bedford MA). Approximately 48 hours after plating, the growth medium was aspirated off, and replaced with serum-free medium containing 25 µg per 96 well plate of calcium-sensitive fluorescent dye Fluo-4 (Molecular Probes, Eugene OR) and 2.5 mM Probenicid (Sigma-Aldrich, St. Louis, MO). The plates were incubated for 1 hour at 37°C, after which the cells were washed 3 times with Hepes Saline solution containing 2.5 mM probenicid to remove excess dye. The plates were then added to the FLIPR® individually, and fluorescence levels were continuously monitored over a 2-minute period. Three well-known MC4R agonists, NDP-MSH, α-MSH, and MTII, and one well-known MC4R antagonist, SHU9119, were purchased from Bachem (Bachem Bioscience Inc., King of Prussia, PA) and tested in the above described assay system. All compounds were diluted with Hepes buffered saline. Figures 11A and 11B show dose response curves for agonist stimulated calcium release, as well as antagonist inhibition of calcium release in the presence of 20 nM NDP-MSH agonist. Graphpad PRISM® software (Graphpad Software, Inc., San Diego CA) was used to determine the EC₅₀ and IC₅₀ for agonist and antagonist respectively. The EC₅₀ for NDP-MSH was calculated to be 13+/- 6 nM, for α-MSH was calculated to be 55 +/- 15 nM, and MTII was calculated to be 10 +/- 6 nM. The IC₅₀ for SHU9119 was calculated to be 2 +/- 2 nM.

### Example 6

The following example was performed to demonstrate the feasibility of using laboratory animals to identify compounds of the instant invention that reduce or increase food intake or metabolic rate.

Healthy, (1-3 years of age) male and female beagles (Marshall Farms, North Rose NY) weighing 11-15 kg were employed as test subjects. The animals were surgically implanted with lateral ventricle cannulas using standard techniques. Wilsson-Rahmberg et *al*., 1998, *J. Investigative* Surgery, 11:207-214. Cannula placement and patency were confirmed with fluoroscopy. The dogs were housed individually in standard caging meeting or exceeding the USDA regulations (U.S. Department of Agriculture, Animal Welfare, Final Rules. 9 C.F.R. Parts 1-3, 1995).

Each subject received a single 50µl injection in one lateral ventricle (ICV injection) of one of 3 solutions: 30-100 nmol of the non-specific melanocortin (MC) receptor agonist [N1e⁴, D-Phe⁷]-α-MSH (hereinafter "NDP-MSH," Bachem Bioscience Inc., King of Prussia PA), 1-2nmol of the MC3/4 receptor antagonist SHU-9119 (Bachem Bioscience Inc., King of Prussia, PA) or vehicle which consisted of artificial cerebrospinal fluid (hereinafter "aCSF," Harvard Apparatus, Holliston, MA). NDP-MSH and SHU-9 119 were received as a dry powder and were reconstituted with aCSF to achieve an appropriate concentration for a 50µl injection. Following ICV injection, resulting effects on food intake or metabolic rate were determined.

The studies consisted of one group of animals containing five dogs. In study 1, dogs received either NDP-MSH or aCSF on day 0 in a cross-over design with two dogs receiving NDP-MSH first and three dogs receiving aCSF first. Resulting effects on food intake were determined. In study 2, dogs received either SHU-9119 or aCSF on day 0 in a cross-over design with two dogs receiving SHU-9119 first and three dogs receiving aCSF first. Resulting effects on food intake were determined. In study 3, dogs received either NDP-MSH or aCSF on day 0 in a cross-over design with three dogs receiving NDP-MSH first and two dogs receiving aCSF first. Resulting effects on metabolic rate were determined. In study 4, dogs received either SHU-9119 or aCSF on day 0 in a cross-over design with three dogs receiving SHU-9119 first and two dogs receiving aCSF first. Resulting effects on metabolic rate were determined. Each test animal was permitted *ad libitum* access to water and IAMS MINICHUNKS® (The IAMS Company, Dayton OH) dry food each day during the study unless otherwise noted.

Reduction or increase in food intake was measured by weighing individual food bowls each day prior to feeding and at the end of each 24 hour consumption period. The difference between these weights represents the amount of food consumed by the dog during the 24 hour consumption period. At the start of each study, there was a seven day baseline period (designated as Days -7 to -1), during which time the test animals baseline food intake was evaluated. The difference between the mean amount consumed on days -7 to -I and the amount consumed following ICV injection represents the reduction or increase in food intake attributable to ICV injection.

Reduction or increase in metabolic rate was measured by standard indirect calorimetry using an open circuit calorimeter (OXYMAX DELUXE®, Columbus Instruments, Columbus OH). Dogs were fasted for approximately 18 hours prior to ICV injection. Calorimetry sessions were initiated approximately 2 hours after ICV injection and lasted for 60-90 minutes.

**TABLE IV**

| Indirect Calorimetry Following ICV Injections in Lateral Ventricle Cannulated Obese Beagle Dogs | | | |
|---|---|---|---|
| Heat Produced (Kcal/hr) | | | |
| | Average | Std Dev | % Change |
| ACSF (100uL) | 22 | 5.0 | - |
| NDP-MSH (75 mol) | 38 | 15.1 | +73 * |
| SHU 9119 (2 nmol) | 13 | 2.8 | -37 * |

| VO₂ (mL/kg/hr) | | | |
|---|---|---|---|
| | Average | Std Dev | % Change |
| ACSF (100uL) | 347 | 54.6 | - |
| NDP-MSH (75 mol) | 598 | 152.4 | +75 * |
| SHU 9119 (2 nmol) | 216 | 64.4 | -38 * |

| | | | |
|---|---|---|---|
| * Significantly different from aCSF control, p<0.05 (Paired t-test) | | | |

**TABLE V**

| Food Intake Following ICV Injections in Lateral Ventricle Cannulated Obese Beagle Dogs | | | |
|---|---|---|---|
| 24 hr Food Intake (g) | | | |
| | Average | Std Dev | % Change |
| aCSF (100uL) | 158 | 66.5 | - |
| NDP-MSH (100 nmol) | 90 | 53.9 | -47 |
| SHU 9119 (2 nmol) | 366 | 103.0 | +132 * |

| | | | |
|---|---|---|---|
| * Significantly different from aCSF control, p<0.05 (Paired t-test) | | | |

The results are shown in Tables IV and V above.

### DEPOSIT OF BIOLOGICAL MATERIALS

A nucelotide sequence which hybridizes under conditions of moderate stringency to the coding region of feline MC4R and a nucleotide sequence which hybridizes under conditions of high stringency to the coding region of canine MC4R were deposited with the American Type Culture Collection (ATCC), at 10801 University Blvd, Manassas, VA 20110, USA on 25^{th} April 2000 and were assigned accession numbers PTA-1762 and PTA 1761 respectively.

## Claims

1. An isolated nucleic acid encoding a functional MC4R, or the complement thereof, said nucleic acid comprising a nucleotide sequence selected from the group consisting of:
(a) a nucleotide sequence which hybridizes under conditions of moderate stringency to the coding region of SEQ ID NO:1;
(b) a nucleotide sequence which hybridizes under conditions of moderate stringency to a polynucleotide which is complementary to the coding region of SEQ ID NO:1;
(c) a nucleotide sequence which hybridizes under conditions of moderate stringency to the coding region of the feline MC4R as deposited with the ATCC and having ATCC Accession No.**PTA-1762**; and
(d) a nucleotide sequence which hybridizes under conditions of moderate stringency to a polynucleotide which is complementary to the coding region of the feline MC4R as deposited with the ATCC and having ATCC Accession No. **PTA-1762**,
with the proviso that said functional MC4R is not human, porcine, murine, rat or chicken.

2. An isolated nucleic acid encoding a functional MC4R, or the complement thereof, said nucleic acid comprising a nucleotide sequence selected from the group consisting of:
(a) a nucleotide sequence which hybridizes under conditions of high stringency to the coding region of SEQ ID NO:1;
(b) a nucleotide sequence which hybridizes under conditions of high stringency to a polynucleotide which is complementary to the coding region of SEQ ID NO:1;
(c) a nucleotide sequence which hybridizes under conditions of high stringency to the coding region of the feline MC4R as deposited with the ATCC and having ATCC Accession No. **PTA-1762**; and
(d) a nucleotide sequence which hybridizes under conditions of high stringency to a polynucleotide which is complementary to the coding region of the feline MC4R as deposited with the ATCC and having ATCC Accession No. **PTA-1762.**

3. An isolated nucleic acid comprising a nucleotide sequence that:
(a) encodes a polypeptide according to SEQ ID NO:3; or
(b) encodes a polypeptide encoded by the feline MC4R clone as deposited with the ATCC and having ATCC Accession No. **PTA-1762.**

4. The isolated nucleic acid of Claim 3, wherein said nucleic acid has a nucleotide sequence according to SEQ ID NO:1 or the feline MC4R clone as deposited with the ATCC and having ATCC Accession No. **PTA-1762.**

5. An isolated nucleic acid comprising a nucleotide sequence having more than 87.4% identity to SEQ ID NO:1 or the feline MC4R clone as deposited with the ATCC and having ATCC Accession No. **PTA-1762.**

6. An isolated nucleic acid comprising a nucleotide sequence encoding a polypeptide having more than 98.2% identity to SEQ ID NO:3 or the polypeptide encoded by the feline MC4R clone as deposited with the ATCC and having ATCC Accession No. **PTA-1762.**

7. An isolated nucleic acid comprising a nucleotide sequence encoding an ECD of a feline MC4R corresponding to amino acids 1-46, 96-123, 186-190, or 268-279 of SEQ ID NO:3 or of the polypeptide encoded by the feline MC4R clone as deposited with the ATCC and having ATCC Accession No. **PTA-1762.**

8. An isolated nucleic acid comprising a nucleotide sequence encoding a CD of a feline MC4R corresponding to amino acids 70-76, 146-165, 212-244, or 302-333 of SEQ ID NO:3 or of the polypeptide encoded by the feline MC4R clone as deposited with the ATCC and having ATCC Accession No. **PTA-1762.**

9. An isolated nucleic acid encoding a functional MC4R, or the complement thereof, said nucleic acid comprising a nucleic selected from the group consisting of:
(a) a nucleotide sequence which hybridizes under conditions of moderate stringency to the coding region of SEQ ID NO:2;
(b) a nucleotide sequence which hybridizes under conditions of moderate stringency to a polynucleotide which is complementary to the coding region of SEQ ID NO:2;
(c) a nucleotide sequence which hybridizes under conditions of moderate stringency to the coding region of the canine MC4R as deposited with the ATCC and having ATCC Accession No. **PTA-1761**; and
(d) a nucleotide sequence which hybridizes under conditions of moderate stringency to a polynucleotide which is complementary to the coding region of the canine MC4R as deposited with the ATCC and having ATCC Accession No. **PTA-1761,**
with the proviso that said functional MC4R is not human, porcine, murine, rat or chicken.

10. An isolated nucleic acid encoding a functional MC4R, or the complement thereof, said nucleic acid comprising a nucleic selected from the group consisting of:
(a) a nucleotide sequence which hybridizes under conditions of high stringency to the coding region of SEQ ID NO:2;
(b) a nucleotide sequence which hybridizes under conditions of high stringency to a polynucleotide which is complementary to the coding region of SEQ ID NO:2;
(c) a nucleotide sequence which hybridizes under conditions of high stringency to the coding region of the canine MC4R as deposited with the ATCC and having ATCC Accession No. **PTA-1761;** and
(d) a nucleotide sequence which hybridizes under conditions of high stringency to a polynucleotide which is complementary to the coding region of the canine MC4R as deposited with the ATCC and having ATCC Accession No. **PTA-1761.**

11. An isolated nucleic acid comprising a nucleotide sequence that:
(a) encodes a polypeptide according to SEQ ID NO:4; or
(b) encodes a polypeptide encoded by the canine MC4R clone as deposited with the ATCC and having ATCC Accession No. **PTA-1761.**

12. The isolated nucleic acid of Claim 11, wherein said nucleic acid has a nucleotide sequence according to SEQ ID NO:2 or the canine MC4R clone as deposited with the ATCC and having ATCC Accession No. **PTA-1761.**

13. An isolated nucleic acid comprising a nucleotide sequence having more than 81.3% identity to SEQ ID NO:2 or the canine MC4R clone as deposited with the ATCC and having ATCC Accession No. **PTA-1761.**

14. An isolated nucleic acid comprising a nucleotide sequence encoding a polypeptide having more than 98.1% identity to SEQ ID NO:4 or to the polypeptide encoded by the canine MC4R clone as deposited with the ATCC and having ATCC Accession No. **PTA-1761.**

15. An isolated nucleic acid comprising a nucleotide sequence encoding an ECD of a canine MC4R corresponding to amino acids 1-46, 98-124, 187-191, or 268-279 of SEQ ID NO:4 or of the polypeptide encoded by the canine MC4R clone as deposited with the ATCC and having ATCC Accession No. **PTA-1761.**

16. An isolated nucleic acid comprising a nucleotide sequence encoding a CD of a canine MC4R corresponding to amino acids 69-77, 147-163, 216-244, or 302-333 of SEQ ID NO:4 or of the polypeptide encoded by the canine MC4R clone as deposited with the ATCC and having ATCC Accession No. **PTA-1761.**

17. A nucleotide vector comprising the nucleic acid of Claim 1, 2, 3, 4, 5, 6, 7, 8, 9, 10, 11, 12, 13, 14, 15 or 16.

18. An expression vector comprising the nucleic acid of Claim 1, 2, 3, 4, 5, 6, 7, 8, 9, 10, 11, 12, 13, 14, 15 or 16 in operative association with a nucleotide regulatory element that controls expression of the polypeptide encoded by said nucleotide sequence.

19. A genetically engineered host cell comprising the nucleic acid of Claim 1, 2, 3, 4 5, 6, 7, 8 9, 10, 11, 12, 13, 14, 15 or 16.

20. A genetically engineered host cell comprising the nucleic acid of Claim 1, 2, 3, 4, 5, 6, 7, 8, 9, 10, 11, 12, 13, 14, 15 or 16 wherein said nucleic acid is in operative association with a nucleotide regulatory element that controls expression of said nucleotide sequence in the host cell.

21. A substantially pure polypeptide encoded by the nucleic acid of Claim 1, 2, 3, 4, 5, 6, 7, 8, 9, 10, 11, 12, 13, 14, 15 or 16

22. A substantially pure polypeptide comprising the amino acid sequence of:
(a) SEQ ID NO:3;
(b) SEQ ID NO:4;
(c) the feline MC4R clone as deposited with the ATCC and having the ATCC Accession NO. **PTA-1762;**
(d) the canine MC4R clone as deposited with the ATCC and having the ATCC Accession NO. **PTA-1761;**
(e) an ECD of a feline MC4R corresponding to amino acids 1-46, 96-123,186-190, or 268-279 of SEQ ID NO:3 or of the polypeptide encoded by the feline MC4R clone as deposited with the ATCC and having ATCC Accession No. **PTA-1762;**
(f) an ECD of a canine MC4R corresponding to amino acids 1-46, 98-124,187-191, or 268-279 of SEQ ID NO:4 or of the polypeptide encoded by the canine MC4R clone as deposited with the ATCC and having ATCC Accession No. **PTA-1761**;
(g) a CD of a feline MC4R corresponding to amino acids 70-76, 146-165, 212-244, or 302-333 of SEQ ID NO:3 or of the polypeptide encoded by the feline MC4R clone as deposited with the ATCC and having ATCC Accession No. **PTA-1762;** or
(h) a CD of a canine MC4R corresponding to amino acids 69-77, 147-163, 216-244, or 302-333 of SEQ ID NO:4 or of the polypeptide encoded by the canine MC4R clone as deposited with the ATCC and having ATCC Accession No. **PTA-1761.**

23. An antibody that immunospecifically binds the polypeptide of Claim 21.

24. A method for producing a recombinant polypeptide, comprising:
(a) culturing a host cell transformed with the expression vector of Claim 18 and which expresses the recombinant polypeptide; and
(b) recovering the recombinant polypeptide from the cell culture.

25. A composition comprising the polypeptide of Claim 21 and a carrier.

26. A method for detecting a polynucleotide of Claim 1, 2, 3, 4, 5, 6, 7, 8, 9, 10, 11, 12, 13, 14, 15 or 16 in a sample, comprising:
(a) contacting the sample with a compound that binds to and forms a complex with the polynucleotide for a period sufficient to form the complex; and
(b) detecting the complex,
so that if a complex is detected, a polynucleotide of Claim 1, 2, 3, 4, 5, 6, 7, 8, 9, 10, 11, 12, 13, 14, 15 or 16 is detected.

27. A method for detecting a polynucleotide of Claim 1, 2, 3, 4, 5, 6, 7, 8, 9, 10, 11, 12, 13, 14, 15 or 16 in a sample, comprising:
(a) contacting the sample under stringent hybridization conditions with nucleic acid primers that anneal to a polynucleotide of Claim 1, 2, 3, 4, 5, 6, 7, 8, 9, 10, 11, 12, 13, 14, 15 or 16 under such conditions; and
b) amplifying the annealed polynucleotides,
so that if a polynucleotide is amplified, the polynucleotide of Claim 1, 2, 3, 4, 5, 6, 7, 8, 9, 10, 11, 12, 13, 14, 15 or 16 is detected.

28. The method of Claim 27, wherein the polynucleotide is an RNA molecule that encodes a functional MC4R, and the method further comprises reverse transcribing an annealed RNA molecule into a cDNA polynucleotide.

29. A method for identifying a compound that binds to the polypeptide of Claim 21, comprising:
(a) contacting a compound with the polypeptide of Claim 21 for a time sufficient to form a polypeptide/compound complex; and
b) detecting the complex,
so that if a polypeptide/compound complex is detected, a compound that binds to a polypeptide of Claim 19 is identified.

30. A method for identifying a compound that binds to the polypeptide of Claim 21, comprising:
(a) contacting a compound with a polypeptide of Claim 21, in a cell, for a time sufficient to form a polypeptide/compound complex, wherein the complex drives expression of a reporter gene sequence in said cell; and
b) detecting the complex by detecting reporter gene sequence expression,
so that if a polypeptide/compound complex is detected, a compound that binds to a polypeptide of Claim 21 is identified.

31. A method of modulating activity of the polypeptide of Claim 21, comprising contacting a cell that expresses the polypeptide with a compound that modulates activity of the polypeptide for a time sufficient to modulate said activity.

32. A method for screening and identifying antagonists of MC4R, comprising:
(a) contacting a cell line that expresses the polynucleotide of Claim 1, 2, 3, 4, 5, 6, 7, 8, 9, 10, 11, 12, 13, 14, 15 or 16 with a test compound in the presence of an MC4R agonist; and
(b) determining whether the test compound inhibits the binding and cellular effects of the MC4R agonist on the cell line,
in which antagonists are identified as those compounds that inhibit both the binding and cellular effects of the MC4R agonist on the cell line.

33. A method for screening and identifying agonists of MC4R, comprising:
(a) contacting a cell line that expresses the polynucleotide of Claim 1, 2, 3, 4, 5, 6, 7, 8, 9, 10, 11, 12, 13, 14, 15 or 16 with a test compound in the presence and in the absence of an MC4R agonist;
(b) determining whether, in the presence of the MC4R agonist, the test compound inhibits the binding of the MC4R agonist to the cell line; and
(c) determining whether, in the absence of the MC4R agonist, the test compound mimics the cellular effects of the MC4R agonist on the cell line,
in which agonists are identified as those test compounds that inhibit the binding but mimic the cellular effects of the MC4R agonist on the cell line.

34. The method of Claim 33 in which the cell line is a genetically engineered cell line.

35. A method for screening and identifying antagonists of MC4R comprising:
(a) contacting the polypeptide of Claim 21 with a random peptide library such that the polypeptide will recognize and bind to one or more peptide species within the library;
(b) isolating the polypeptide/peptide combination;
(c) determining the sequence of the peptide isolated in step (b); and
(d) determining whether the test compound inhibits the binding and cellular effects of an MC4R agonist,
in which antagonists are identified as those peptides that inhibit both the binding and cellular effects of the MC4R agonist.

36. A method for screening and identifying agonists of MC4R comprising:
(a) contacting the polypeptide of Claim 21 with a random peptide library such that the polypeptide will recognize and bind to one or more peptide species within the library;
(b) isolating a polypeptide/peptide combination;
(c) determining the sequence of the peptide isolated in step (b); and
(d) determining whether, in the absence of a MC4R agonist, the peptide mimics the cellular effects of the MC4R agonist,
in which agonists are identified as those peptides that inhibit the binding of the MC4R agonist to a MC4R polypeptide but mimic the cellular effects of the MC4R agonist.

37. A method of modulating activity of the polypeptide of Claim 21, comprising contacting the polypeptide with a compound that modulates activity of the polypeptide for a time sufficient to modulate said activity.

38. A method of modulating the endogenous enzymatic activity of MC4R in an animal comprising administering to the animal an amount of an MC4R ligand effective to modulate said endogenous enzymatic activity.

39. The method of Claim 38, wherein the animal is a cow, a pig, a goat, a sheep, a horse, a dog, or a cat.

40. The method of Claim 38 in which the ligand to said MC4R receptor is an MC4R agonist.

41. The method of Claim 38 in which the ligand to said MC4R receptor is an MC4R antagonist.

42. The antagonist of Claim 41 that is a monoclonal antibody that immunospecifically binds to an epitope of said MC4R.

43. The method of Claim 38 in which the enzymatic activity of said MC4R is increased.

44. The method of Claim 38 in which the enzymatic activity of said MC4R is decreased.

45. A transgenic animal in which the nucleic acid of Claim 1 or 9 is an expressed transgene contained in the genome of the animal.

46. A transgenic animal in which expression of genomic sequences encoding the polypeptide of Claim 21 is prevented or repressed.

47. A method for modulating the appetite and/or metabolic rate of an animal comprising administering to the animal an effective amount of an MC4R ligand.

48. The method of Claim 47, wherein the animal has an appetite-related or metabolic disorder.

49. The method of Claim 48 wherein the disorder causes, is caused by, or is **characterized by** a reduction in appetite, feeding behavior or body weight, or an increase in metabolic rate, and the ligand is an MC4R antagonist.

50. The method of Claim 49 wherein the disorder causes, is caused by, or is **characterized by** an increase in appetite, feeding behavior, or body weight, or a decrease in metabolic rate, and the ligand is an MC4R agonist.

51. The method of Claim 49 wherein the animal is a *post partum* sow or dairy cow.

52. The method of Claim 48 wherein the animal is a companion animal.

53. The method of Claim 48 wherein the animal is a livestock animal.

54. The method of Claim 48 wherein the animal is a poultry animal.

55. The method of Claim 49 wherein the animal suffers from shipping or crowding stress.

56. The method of Claim 49 wherein the animal is lactating.

57. The method of Claim 47 wherein the animal is gravid.

58. The method of Claim 49 wherein the disorder is cachexia, anorexia or weaning-induced inappetance and growth lag.

59. The method of Claim 49 wherein the disorder is a metabolic disorder.

60. The method of Claim 59 wherein the metabolic disorder is diabetes.

61. The method of Claim 49 wherein the disorder is a disease.

62. The method of Claim 61 wherein the disease is cancer, renal failure, cardiac disease, endotoxemia, fever, hepatic lipidosis, infection or inflammation.

63. The method of Claim 50 wherein the animal is obese.

64. The method of Claim 47 wherein the MC4R ligand is part of a pharmaceutical composition.

65. A method of increasing the reproductive performance of an animal comprising administering to the animal an effective amount of an MC4R ligand.

66. A method of increasing the growth performance of an animal comprising administering to the animal an effective amount of an MC4R ligand.

67. The method of Claim 47 wherein the ligand is part of a pharmaceutical composition.

68. The method of Claim 67 wherein the preparation is administered orally, transdermally, or by slow release subcutaneous implants/pellets.

69. A kit comprising a pharmaceutical composition comprising an MC4R ligand.


SEQUENCE LISTING

<110> Pfizer Products Inc.

<120> NOVEL MELANOCORTIN-4 RECEPTOR SEQUENCES AND
      SCREENING ASSAYS TO IDENTIFY COMPOUNDS USEFUL
      IN REGULATING ANIMAL APPETITE AND METABOLIC RATE

<130> PC10743A

<140>
<141>

<150> 60/213,909
<151> 2000-06-26

<160> 6

<170> PatentIn Ver. 2.1

<210> 1
<211> 1708
<212> DNA
<213> Feline MC4R Nucleotide Sequence

<400> 1
cataaaatca gcagcagcta ctaacactca aagcaatgct tcaggttggg aactaatacc 60
tcagaggcag ctggtgtgaa catgcaaaca cggattcagc tcccagtggc acagcagcca 120
ctaggaaaat tattttgaaa agacctgact gaatgcctca ggctaaagtt aaggtggaag 180
ggaggacaga aaagcaaaga gcagactctt tcaactgaga atgagtattt cagaagccta 240
agattttaca atgaaggtga tcagagccgt tcctgggaga cagtaaaaac tccatttcca 300
gcctgggagc acgtgacatt tactcacaac aggcatgcca atttcagcct canaactttc 360
gggcagacaa aggcgtggag aaaaacactg aggctacctg acccgagaga tcgaatcaat 420
tccgagggga tctgaatcca ctggtgcagg atgaactcca ctcatcacca tggaatgcac 480
acttctctcc acttctggaa ccgcagcacc tacggaccgc acagcaatgc cagtgagtcc 540
cttggaaaag gctactctga tggagggtgt tatgagcaac tttttgtctc ccctgaggtg 600
tttgtgactc tgggtgtcat cagcttgttg gagaatattc tggtgattgt ggcaatagcc 660
aagaacaaaa acctgcattc gcccatgtac tttttcatct gcagcctggc tgtggctgat 720
atgttggtga gcgtgtcaaa cggatccgaa accattgtca tcaccctatt aaacagtaca 780
gatacggacg cgcagagttt caccgtgaat attgataatg tcattgactc ggtgatctgt 840
agctccttgc ttgcatcgat ttgcagcctg ctctcaattg cagtggacag gtactttact 900
atcttttatg ctctccagta ccataacatc atgacggtca ggcgggttgg gatcatcata 960
agttgtatct gggcagcttg cacggtttcg ggcgttttgt tcatcatcta ctcagacagc 1020
agtgctgtca tcatctgcct catcaccatg ttcttcacca tgctggctct catggcctct 1080
ctctatgtcc acatgttcct catggccaga ctgcacatta agagaattgc tgtcctcccg 1140
ggcactggca ccatccgcca aggggccaac atgaagggtg caattaccct gaccatactg 1200
attggggtct ttgttgtctg ctgggccccg ttcttcctcc acttaatatt ctacatctct 1260
tgtccccaga atccttactg tgtgtgcttc atgtctcact ttaacctgta tctcatactg 1320

atcatgtgta attccatcat cgaccctcta atttatgcac tccggagcca agaactaagg 1380
aaaaccttca aagagatcat ctgttgctat cctctaggcg gcctctgtga tttgtctagc 1440
agatactaac tgtgcagata gaaacgtgca taagagactt cttcattctt acagaaccgg 1500
aacattgtgc tttgatgacc cttttctcct ctgtgtaagg catgggttga gactatctgt 1560
tgtataaatt taagttcatg actttttttt ggaatggaaa caatgcccag tctctgtaca 1620
tttctaatgt cttgctactt tttggctgta caatgttaat ccatattata ggttgtaggc 1680
actatgaatg tataaaaaaa aaaaaaaa 1708

<210> 2
<211> 1985
<212> DNA
<213> Canine MC4R Nucleotide Sequence

<400> 2
ctaagaccgt ggggaggcag ctgatgcgaa catgtgcacg cagattcagc tcctggtggc 60
tcggcggcaa ctcggagaat tacttgcaac agacctcact gaatgcccta gactaaagtt 120
aaggtgggag tgaggacaaa aaaaaaaaag aaaaagaaaa aagaaaaaaa gaaaaaaaag 180
aaaaagcaaa gagcagactc tttgaactaa gaatgagcat ttcagaaatc gaagatgtta 240
cagtgaaggt gatcggagct gtacctggaa gacagtaaga gctccactgc cagccttttg 300
gagcacggga caggtactca acacctggca ggccagctgg atcctcagaa ctttgggacg 360
cacggagagg gggagaacat caccggggct ccctggctgg agaggccgaa tcagtcccga 420
gggggtctgc atacacttgt tgcaggatga actccaccct tcagcacgga atgcacactt 480
ctctccactt ctggaaccgc agcacctacg gacagcacgg caacgccact gagtcccttg 540
gcaaaggcta ccccgacggg ggatgctacg agcaactctt cgtctccccg gaggtgttcg 600
tgactctggg ggtcataagc ttgctggaga acattctggt gatcgtggca atagccaaga 660
acaagaatct gcactcaccc atgtactttt tcatctgtag cctggctgtg gccgatatgc 720
tggtgagcgt ttccaacggg tcagagacca tcgtcatcac cctgttgaac agtacggata 780
cggacgcgca gagtttcacg gtgaatattg ataatgtcat tgactcggtg atctgtagct 840
ccttgctcgc ctcgatttgc agcctgctct caattgcagt ggacaggtac tttactatct 900
tttatgccct ccagtaccat aacatcatga cggtgaggcg ggttgggatc atcatcagtt 960
gcatctgggc ggcttgcacg gtgtcaggca tcttgttcat catttactcg gacagtactg 1020
ctgtcatcat ctgcctcatc accatgttct tcaccatgct ggccctcatg gcttctctct 1080
acgtccacat gttcctcatg gccagactgc acatcaagag aatcgccgtc ctcccgggca 1140
ccggcaccat ccgccaaggg gccaacatga agggtgccat taccttgacc atactcattg 1200
gggtcttcgt cgtctgctgg gctccattct tcctccactt gatattctac atctcttgtc 1260
cccagaatcc atactgtgtg tgcttcatgt ctcactttaa cttgtacctc attctgatca 1320
tgtgtaactc catcatcgac cctctcattt atgcactccg gagccaagag ctgaggaaaa 1380
ccttcaaaga gatcatctgt tgctatcctc tgggtggcct ttgtgacttg tctagcagat 1440
actagctggg gacagaggaa gtactaaaaa catgcaccag agacttcttc atcctcacac 1500
aacatgaact gtgtgcttgg acaacagctg cttcttcagt ataaggcagg agttgagaat 1560
atctgttgca caaattcaac tttatgatgt tttgatgtga aaaaaaaaat gcccaggctc 1620
tgtacattgc taatgtcatg ctacttttgg gctgtgcatt gttaatccat ttcgacgctg 1680
tagacacttt gaatttctag aaaagaaaaa agcttccatt aaaagcatat cagtgtttct 1740
tgttattcac gaggatttgg cactttgctt gctttaggaa acatagaaat catagaatca 1800
ttaactatgt agcctgataa gtaacttctt atattatact atatcacatg aaatgtgcag 1860
atttgaatgt agcatggggg gtggatattg aacaatagat acttggtcat taaaacaatc 1920
aactgaaatt ttaagtaata aaatgtgttc attctccctg ttgcagaaat aaaaaaaaaa 1980

aaaaa 1985

<210> 3
<211> 332
<212> PRT
<213> Feline MC4R protein Sequence

<400> 3
Met Asn Ser Thr His His His Gly Met His Thr Ser Leu His Phe Trp
1               5                   10                  15

Asn Arg Ser Thr Tyr Gly Pro His Ser Asn Ala Ser Glu Ser Leu Gly
            20                  25                  30

Lys Gly Tyr Ser Asp Gly Gly Cys Tyr Glu Gln Leu Phe Val Ser Pro
        35                  40                  45

Glu Val Phe Val Thr Leu Gly Val Ile Ser Leu Leu Glu Asn Ile Leu
    50                  55                  60

Val Ile Val Ala Ile Ala Lys Asn Lys Asn Leu His Ser Pro Met Tyr
65                  70                  75                  80

Phe Phe Ile Cys Ser Leu Ala Val Ala Asp Met Leu Val Ser Val Ser
                85                  90                  95

Asn Gly Ser Glu Thr Ile Val Ile Thr Leu Leu Asn Ser Thr Asp Thr
            100                 105                 110

Asp Ala Gln Ser Phe Thr Val Asn Ile Asp Asn Val Ile Asp Ser Val
        115                 120                 125

Ile Cys Ser Ser Leu Leu Ala Ser Ile Cys Ser Leu Leu Ser Ile Ala
    130                 135                 140

Val Asp Arg Tyr Phe Thr Ile Phe Tyr Ala Leu Gln Tyr His Asn Ile
145                 150                 155                 160

Met Thr Val Arg Arg Val Gly Ile Ile Ile Ser Cys Ile Trp Ala Ala
                165                 170                 175

Cys Thr Val Ser Gly Val Leu Phe Ile Ile Tyr Ser Asp Ser Ser Ala
            180                 185                 190

Val Ile Ile Cys Leu Ile Thr Met Phe Phe Thr Met Leu Ala Leu Met
        195                 200                 205

Ala Ser Leu Tyr Val His Met Phe Leu Met Ala Arg Leu His Ile Lys
    210                 215                 220

Arg Ile Ala Val Leu Pro Gly Thr Gly Thr Ile Arg Gln Gly Ala Asn
225                 230                 235                 240

Met Lys Gly Ala Ile Thr Leu Thr Ile Leu Ile Gly Val Phe Val Val
                245                 250                 255

Cys Trp Ala Pro Phe Phe Leu His Leu Ile Phe Tyr Ile Ser Cys Pro
            260                 265                 270

Gln Asn Pro Tyr Cys Val Cys Phe Met Ser His Phe Asn Leu Tyr Leu
        275                 280                 285

Ile Leu Ile Met Cys Asn Ser Ile Ile Asp Pro Leu Ile Tyr Ala Leu
    290                 295                 300

Arg Ser Gln Glu Leu Arg Lys Thr Phe Lys Glu Ile Ile Cys Cys Tyr
305                 310                 315                 320

Pro Leu Gly Gly Leu Cys Asp Leu Ser Ser Arg Tyr
                325                 330

<210> 4
<211> 332
<212> PRT
<213> Canine MC4R protein Sequence

<400> 4
Met Asn Ser Thr Leu Gln His Gly Met His Thr Ser Leu His Phe Trp
1               5                   10                  15

Asn Arg Ser Thr Tyr Gly Gln His Gly Asn Ala Thr Glu Ser Leu Gly
            20                  25                  30

Lys Gly Tyr Pro Asp Gly Gly Cys Tyr Glu Gln Leu Phe Val Ser Pro
        35                  40                  45

Glu Val Phe Val Thr Leu Gly Val Ile Ser Leu Leu Glu Asn Ile Leu
    50                  55                  60

Val Ile Val Ala Ile Ala Lys Asn Lys Asn Leu His Ser Pro Met Tyr
65                  70                  75                  80

Phe Phe Ile Cys Ser Leu Ala Val Ala Asp Met Leu Val Ser Val Ser
                85                  90                  95

Asn Gly Ser Glu Thr Ile Val Ile Thr Leu Leu Asn Ser Thr Asp Thr
            100                 105                 110

Asp Ala Gln Ser Phe Thr Val Asn Ile Asp Asn Val Ile Asp Ser Val
        115                 120                 125

Ile Cys Ser Ser Leu Leu Ala Ser Ile Cys Ser Leu Leu Ser Ile Ala
    130                 135                 140

Val Asp Arg Tyr Phe Thr Ile Phe Tyr Ala Leu Gln Tyr His Asn Ile
145                 150                 155                 160

Met Thr Val Arg Arg Val Gly Ile Ile Ile Ser Cys Ile Trp Ala Ala
                165                 170                 175

Cys Thr Val Ser Gly Ile Leu Phe Ile Ile Tyr Ser Asp Ser Thr Ala
            180                 185                 190

Val Ile Ile Cys Leu Ile Thr Met Phe Phe Thr Met Leu Ala Leu Met
        195                 200                 205

Ala Ser Leu Tyr Val His Met Phe Leu Met Ala Arg Leu His Ile Lys
    210                 215                 220

Arg Ile Ala Val Leu Pro Gly Thr Gly Thr Ile Arg Gln Gly Ala Asn
225                 230                 235                 240

Met Lys Gly Ala Ile Thr Leu Thr Ile Leu Ile Gly Val Phe Val Val
                245                 250                 255

Cys Trp Ala Pro Phe Phe Leu His Leu Ile Phe Tyr Ile Ser Cys Pro
            260                 265                 270

Gln Asn Pro Tyr Cys Val Cys Phe Met Ser His Phe Asn Leu Tyr Leu
        275                 280                 285

Ile Leu Ile Met Cys Asn Ser Ile Ile Asp Pro Leu Ile Tyr Ala Leu
    290                 295                 300

Arg Ser Gln Glu Leu Arg Lys Thr Phe Lys Glu Ile Ile Cys Cys Tyr
305                 310                 315                 320

Pro Leu Gly Gly Leu Cys Asp Leu Ser Ser Arg Tyr
                325                 330

<210> 5

<211> 6148
<212> DNA
<213> pcDNA3.1zeo/murine G-alpha 15

<400> 5
gacggatcgg gagatctccc gatcccctat ggtcgactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggac tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagcttggtg gtctgtgaag cgcccaccat ggcccggtcc ctgacttggg 960
gctgctgtcc ctggtgcctg acagaggagg agaagactgc cgccagaatc gaccaggaga 1020
tcaacaggat tttgttggaa cagaaaaaac aagagcgcga ggaattgaaa ctcctgctgt 1080
tggggcctgg tgagagcggg aagagtacgt tcatcaagca gatgcgcatc attcacggtg 1140
tgggctactc ggaggaggac cgcagagcct tccggctgct catctaccag aacatcttcg 1200
tctccatgca ggccatgata gatgcgatgg accggctgca gatccccttc agcaggcctg 1260
acagcaagca gcacgccagc ctagtgatga cccaggaccc ctataaagtg agcacattcg 1320
agaagccata tgcagtggcc atgcagtacc tgtggcggga cgcgggcatc cgtgcatgct 1380
acgagcgaag gcgtgaattc caccttctgg actccgcggt gtattacctg tcacacctgg 1440
agcgcatatc agaggacagc tacatcccca ctgcgcaaga cgtgctgcgc agtcgcatgc 1500
ccaccacagg catcaatgag tactgcttct ccgtgaagaa aaccaaactg cgcatcgtgg 1560
atgttggtgg ccagaggtca gagcgtagga aatggattca ctgtttcgag aacgtgattg 1620
ccctcatcta cctggcctcc ctgagcgagt atgaccagtg cctagaggag aacgatcagg 1680
agaaccgcat ggaggagagt ctcgctctgt tcagcacgat cctagagctg ccctggttca 1740
agagcacctc ggtcatcctc ttcctcaaca agacggacat cctggaagat aagattcaca 1800
cctcccacct ggccacatac ttccccagct tccagggacc ccggcgagac gcagaggccg 1860
ccaagagctt catcttggac atgtatgcgc gcgtgtacgc gagctgcgca gagccccagg 1920
acggtggcag gaaaggctcc cgcgcgcgcc gcttcttcgc acacttcacc tgtgccacgg 1980
acacgcaaag cgtccgcagc gtgttcaagg acgtgcggga ctcggtgctg gcccggtacc 2040
tggacgagat caacctgctg tgacgcagat ctaaagccga attctgcaga tatccatcac 2100
actggcggcc gctcgagcat gcatctagag ggcccgttta aacccgctga tcagcctcga 2160
ctgtgccttc tagttgccag ccatctgttg tttgcccctc ccccgtgcct tccttgaccc 2220
tggaaggtgc cactcccact gtcctttcct aataaaatga ggaaattgca tcgcattgtc 2280
tgagtaggtg tcattctatt ctggggggtg gggtggggca ggacagcaag ggggaggatt 2340
gggaagacaa tagcaggcat gctggggatg cggtgggctc tatggcttct gaggcggaaa 2400
gaaccagctg gggctctagg gggtatcccc acgcgccctg tagcggcgca ttaagcgcgg 2460
cgggtgtggt ggttacgcgc agcgtgaccg ctacacttgc cagcgcccta gcgcccgctc 2520
ctttcgcttt cttcccttcc tttctcgcca cgttcgccgg ctttccccgt caagctctaa 2580

atcggggcat ccctttaggg ttccgattta gtgctttacg gcacctcgac cccaaaaaac 2640
ttgattaggg tgatggttca cgtagtgggc catcgccctg atagacggtt tttcgccctt 2700
tgacgttgga gtccacgttc tttaatagtg gactcttgtt ccaaactgga acaacactca 2760
accctatctc ggtctattct tttgatttat aagggatttt ggggatttcg gcctattggt 2820
taaaaaatga gctgatttaa caaaaattta acgcgaatta attctgtgga atgtgtgtca 2880
gttagggtgt ggaaagtccc caggctcccc aggcaggcag aagtatgcaa agcatgcatc 2940
tcaattagtc agcaaccagg tgtggaaagt ccccaggctc cccagcaggc agaagtatgc 3000
aaagcatgca tctcaattag tcagcaacca tagtcccgcc cctaactccg cccatcccgc 3060
ccctaactcc gcccagttcc gcccattctc cgccccatgg ctgactaatt ttttttattt 3120
atgcagaggc cgaggccgcc tctgcctctg agctattcca gaagtagtga ggaggctttt 3180
ttggaggcct aggcttttgc aaaaagctcc cgggagcttg tatatccatt ttcggatctg 3240
atcagcacgt gttgacaatt aatcatcggc atagtatatc ggcatagtat aatacgacaa 3300
ggtgaggaac taaaccatgg ccaagttgac cagtgccgtt ccggtgctca ccgcgcgcga 3360
cgtcgccgga gcggtcgagt tctggaccga ccggctcggg ttctcccggg acttcgtgga 3420
ggacgacttc gccggtgtgg tccgggacga cgtgaccctg ttcatcagcg cggtccagga 3480
ccaggtggtg ccggacaaca ccctggcctg ggtgtgggtg cgcggcctgg acgagctgta 3540
cgccgagtgg tcggaggtcg tgtccacgaa cttccgggac gcctccgggc cggccatgac 3600
cgagatcggc gagcagccgt gggggcggga gttcgccctg cgcgacccgg ccggcaactg 3660
cgtgcacttc gtggccgagg agcaggactg acacgtgcta cgagatttcg attccaccgc 3720
cgccttctat gaaaggttgg gcttcggaat cgttttccgg gacgccggct ggatgatcct 3780
ccagcgcggg gatctcatgc tggagttctt cgcccacccc aacttgttta ttgcagctta 3840
taatggttac aaataaagca atagcatcac aaatttcaca aataaagcat ttttttcact 3900
gcattctagt tgtggtttgt ccaaactcat caatgtatct tatcatgtct gtataccgtc 3960
gacctctagc tagagcttgg cgtaatcatg gtcatagctg tttcctgtgt gaaattgtta 4020
tccgctcaca attccacaca acatacgagc cggaagcata aagtgtaaag cctggggtgc 4080
ctaatgagtg agctaactca cattaattgc gttgcgctca ctgcccgctt tccagtcggg 4140
aaacctgtcg tgccagctgc attaatgaat cggccaacgc gcggggagag gcggtttgcg 4200
tattgggcgc tcttccgctt cctcgctcac tgactcgctg cgctcggtcg ttcggctgcg 4260
gcgagcggta tcagctcact caaaggcggt aatacggtta tccacagaat caggggataa 4320
cgcaggaaag aacatgtgag caaaaggcca gcaaaaggcc aggaaccgta aaaaggccgc 4380
gttgctggcg tttttccata ggctccgccc ccctgacgag catcacaaaa atcgacgctc 4440
aagtcagagg tggcgaaacc cgacaggact ataaagatac caggcgtttc cccctggaag 4500
ctccctcgtg cgctctcctg ttccgaccct gccgcttacc ggatacctgt ccgcctttct 4560
cccttcggga agcgtggcgc tttctcaatg ctcacgctgt aggtatctca gttcggtgta 4620
ggtcgttcgc tccaagctgg gctgtgtgca cgaacccccc gttcagcccg accgctgcgc 4680
cttatccggt aactatcgtc ttgagtccaa cccggtaaga cacgacttat cgccactggc 4740
agcagccact ggtaacagga ttagcagagc gaggtatgta ggcggtgcta cagagttctt 4800
gaagtggtgg cctaactacg gctacactag aaggacagta tttggtatct gcgctctgct 4860
gaagccagtt accttcggaa aaagagttgg tagctcttga tccggcaaac aaaccaccgc 4920
tggtagcggt ggtttttttg tttgcaagca gcagattacg cgcagaaaaa aaggatctca 4980
agaagatcct ttgatctttt ctacggggtc tgacgctcag tggaacgaaa actcacgtta 5040
agggattttg gtcatgagat tatcaaaaag gatcttcacc tagatccttt taaattaaaa 5100
atgaagtttt aaatcaatct aaagtatata tgagtaaact tggtctgaca gttaccaatg 5160
cttaatcagt gaggcaccta tctcagcgat ctgtctattt cgttcatcca tagttgcctg 5220
actccccgtc gtgtagataa ctacgatacg ggagggctta ccatctggcc ccagtgctgc 5280
aatgataccg cgagacccac gctcaccggc tccagattta tcagcaataa accagccagc 5340
cggaagggcc gagcgcagaa gtggtcctgc aactttatcc gcctccatcc agtctattaa 5400
ttgttgccgg gaagctagag taagtagttc gccagttaat agtttgcgca acgttgttgc 5460

cattgctaca ggcatcgtgg tgtcacgctc gtcgtttggt atggcttcat tcagctccgg 5520
ttcccaacga tcaaggcgag ttacatgatc ccccatgttg tgcaaaaaag cggttagctc 5580
cttcggtcct ccgatcgttg tcagaagtaa gttggccgca gtgttatcac tcatggttat 5640
ggcagcactg cataattctc ttactgtcat gccatccgta agatgctttt ctgtgactgg 5700
tgagtactca accaagtcat tctgagaata gtgtatgcgg cgaccgagtt gctcttgccc 5760
ggcgtcaata cgggataata ccgcgccaca tagcagaact ttaaaagtgc tcatcattgg 5820
aaaacgttct tcggggcgaa aactctcaag gatcttaccg ctgttgagat ccagttcgat 5880
gtaacccact cgtgcaccca actgatcttc agcatctttt actttcacca gcgtttctgg 5940
gtgagcaaaa acaggaaggc aaaatgccgc aaaaaaggga ataagggcga cacggaaatg 6000
ttgaatactc atactcttcc tttttcaata ttattgaagc atttatcagg gttattgtct 6060
catgagcgga tacatatttg aatgtattta gaaaaataaa caaatagggg ttccgcgcac 6120
atttccccga aaagtgccac ctgacgtc 6148

<210> 6
<211> 6149
<212> DNA
<213> pcDNA3.1zeo/human G-alpha 16

<400> 6
gacggatcgg gagatctccc gatcccctat ggtcgactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggac tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagcttgact gaggccaccg caccatggcc cgctcgctga cctggcgctg 960
ctgcccctgg tgcctgacgg aggatgagaa ggccgccgcc cgggtggacc aggagatcaa 1020
caggatcctc ttggagcaga agaagcagga ccgcggggag ctgaagctgc tgcttttggg 1080
cccaggcgag agcgggaaga gcaccttcat caagcagatg cggatcatcc acggcgccgg 1140
ctactcggag gaggagcgca agggcttccg gcccctggtc taccagaaca tcttcgtgtc 1200
catgcgggcc atgatcgagg ccatggagcg gctgcagatt ccattcagca ggcccgagag 1260
caagcaccac gctagcctgg tcatgagcca ggacccctat aaagtgacca cgtttgagaa 1320
gcgctacgct gcggccatgc agtggctgtg gagggatgcc ggcatccggg cctgctatga 1380
gcgtcggcgg gaattccacc tgctcgattc agccgtgtac tacctgtccc acctggagcg 1440
catcaccgag gagggctacg tccccacagc tcaggacgtg ctccgcagcc gcatgcccac 1500
cactggcatc aacgagtact gcttctccgt gcagaaaacc aacctgcgga tcgtggacgt 1560
cgggggccag aagtcagagc gtaagaaatg gatccattgt ttcgagaacg tgatcgccct 1620
catctacctg gcctcactga gtgaatacga ccagtgcctg gaggagaaca accaggagaa 1680

ccgcatgaag gagagcctcg cattgtttgg gactatcctg gaactaccct ggttcaaaag 1740
cacatccgtc atcctctttc tcaacaaaac cgacatcctg gaggagaaaa tccccacctc 1800
ccacctggct acctatttcc ccagtttcca gggccctaag caggatgctg aggcagccaa 1860
gaggttcatc ctggacatgt acacgaggat gtacaccggg tgcgtggacg gccccgaggg 1920
cagcaagaag ggcgcacgat cccgacgcct tttcagccac tacacatgtg ccacagacac 1980
acagaacatc cgcaaggtct tcaaggacgt gcgggactcg gtgctcgccc gctacctgga 2040
cgagatcaac ctgctgtgac ccagatctaa agccgaattc tgcagatatc catcacactg 2100
gcggccgctc gagcatgcat ctagactaga gggcccgttt aaacccgctg atcagcctcg 2160
actgtgcctt ctagttgcca gccatctgtt gtttgcccct cccccgtgcc ttccttgacc 2220
ctggaaggtg ccactcccac tgtcctttcc taataaaatg aggaaattgc atcgcattgt 2280
ctgagtaggt gtcattctat tctggggggt ggggtggggc aggacagcaa gggggaggat 2340
tgggaagaca atagcaggca tgctggggat gcggtgggct ctatggcttc tgaggcggaa 2400
agaaccagct ggggctctag ggggtatccc cacgcgccct gtagcggcgc attaagcgcg 2460
gcgggtgtgg tggttacgcg cagcgtgacc gctacacttg ccagcgccct agcgcccgct 2520
cctttcgctt tcttcccttc ctttctcgcc acgttcgccg gctttccccg tcaagctcta 2580
aatcggggca tccctttagg gttccgattt agtgctttac ggcacctcga ccccaaaaaa 2640
cttgattagg gtgatggttc acgtagtggg ccatcgccct gatagacggt ttttcgccct 2700
ttgacgttgg agtccacgtt ctttaatagt ggactcttgt tccaaactgg aacaacactc 2760
aaccctatct cggtctattc ttttgattta taagggattt tggggatttc ggcctattgg 2820
ttaaaaaatg agctgattta acaaaaattt aacgcgaatt aattctgtgg aatgtgtgtc 2880
agttagggtg tggaaagtcc ccaggctccc caggcaggca gaagtatgca aagcatgcat 2940
ctcaattagt cagcaaccag gtgtggaaag tccccaggct ccccagcagg cagaagtatg 3000
caaagcatgc atctcaatta gtcagcaacc atagtcccgc ccctaactcc gcccatcccg 3060
cccctaactc cgcccagttc cgcccattct ccgccccatg gctgactaat tttttttatt 3120
tatgcagagg ccgaggccgc ctctgcctct gagctattcc agaagtagtg aggaggcttt 3180
tttggaggcc taggcttttg caaaaagctc ccgggagctt gtatatccat tttcggatct 3240
gatcagcacg tgttgacaat taatcatcgg catagtatat cggcatagta taatacgaca 3300
aggtgaggaa ctaaaccatg gccaagttga ccagtgccgt tccggtgctc accgcgcgcg 3360
acgtcgccgg agcggtcgag ttctggaccg accggctcgg gttctcccgg gacttcgtgg 3420
aggacgactt cgccggtgtg gtccgggacg acgtgaccct gttcatcagc gcggtccagg 3480
accaggtggt gccggacaac accctggcct gggtgtgggt gcgcggcctg gacgagctgt 3540
acgccgagtg gtcggaggtc gtgtccacga acttccggga cgcctccggg ccggccatga 3600
ccgagatcgg cgagcagccg tgggggcggg agttcgccct gcgcgacccg gccggcaact 3660
gcgtgcactt cgtggccgag gagcaggact gacacgtgct acgagatttc gattccaccg 3720
ccgccttcta tgaaaggttg ggcttcggaa tcgttttccg ggacgccggc tggatgatcc 3780
tccagcgcgg ggatctcatg ctggagttct tcgcccaccc caacttgttt attgcagctt 3840
ataatggtta caaataaagc aatagcatca caaatttcac aaataaagca tttttttcac 3900
tgcattctag ttgtggtttg tccaaactca tcaatgtatc ttatcatgtc tgtataccgt 3960
cgacctctag ctagagcttg gcgtaatcat ggtcatagct gtttcctgtg tgaaattgtt 4020
atccgctcac aattccacac aacatacgag ccggaagcat aaagtgtaaa gcctggggtg 4080
cctaatgagt gagctaactc acattaattg cgttgcgctc actgcccgct ttccagtcgg 4140
gaaacctgtc gtgccagctg cattaatgaa tcggccaacg cgcggggaga ggcggtttgc 4200
gtattgggcg ctcttccgct tcctcgctca ctgactcgct gcgctcggtc gttcggctgc 4260
ggcgagcggt atcagctcac tcaaaggcgg taatacggtt atccacagaa tcaggggata 4320
acgcaggaaa gaacatgtga gcaaaaggcc agcaaaaggc caggaaccgt aaaaaggccg 4380
cgttgctggc gtttttccat aggctccgcc cccctgacga gcatcacaaa aatcgacgct 4440
caagtcagag gtggcgaaac ccgacaggac tataaagata ccaggcgttt ccccctggaa 4500
gctccctcgt gcgctctcct gttccgaccc tgccgcttac cggatacctg tccgcctttc 4560

tcccttcggg aagcgtggcg ctttctcaat gctcacgctg taggtatctc agttcggtgt 4620
aggtcgttcg ctccaagctg ggctgtgtgc acgaaccccc cgttcagccc gaccgctgcg 4680
ccttatccgg taactatcgt cttgagtcca acccggtaag acacgactta tcgccactgg 4740
cagcagccac tggtaacagg attagcagag cgaggtatgt aggcggtgct acagagttct 4800
tgaagtggtg gcctaactac ggctacacta gaaggacagt atttggtatc tgcgctctgc 4860
tgaagccagt taccttcgga aaaagagttg gtagctcttg atccggcaaa caaaccaccg 4920
ctggtagcgg tggttttttt gtttgcaagc agcagattac gcgcagaaaa aaaggatctc 4980
aagaagatcc tttgatcttt tctacggggt ctgacgctca gtggaacgaa aactcacgtt 5040
aagggatttt ggtcatgaga ttatcaaaaa ggatcttcac ctagatcctt ttaaattaaa 5100
aatgaagttt taaatcaatc taaagtatat atgagtaaac ttggtctgac agttaccaat 5160
gcttaatcag tgaggcacct atctcagcga tctgtctatt tcgttcatcc atagttgcct 5220
gactccccgt cgtgtagata actacgatac gggagggctt accatctggc cccagtgctg 5280
caatgatacc gcgagaccca cgctcaccgg ctccagattt atcagcaata aaccagccag 5340
ccggaagggc cgagcgcaga agtggtcctg caactttatc cgcctccatc cagtctatta 5400
attgttgccg ggaagctaga gtaagtagtt cgccagttaa tagtttgcgc aacgttgttg 5460
ccattgctac aggcatcgtg gtgtcacgct cgtcgtttgg tatggcttca ttcagctccg 5520
gttcccaacg atcaaggcga gttacatgat cccccatgtt gtgcaaaaaa gcggttagct 5580
ccttcggtcc tccgatcgtt gtcagaagta agttggccgc agtgttatca ctcatggtta 5640
tggcagcact gcataattct cttactgtca tgccatccgt aagatgcttt tctgtgactg 5700
gtgagtactc aaccaagtca ttctgagaat agtgtatgcg gcgaccgagt tgctcttgcc 5760
cggcgtcaat acgggataat accgcgccac atagcagaac tttaaaagtg ctcatcattg 5820
gaaaacgttc ttcggggcga aaactctcaa ggatcttacc gctgttgaga tccagttcga 5880
tgtaacccac tcgtgcaccc aactgatctt cagcatcttt tactttcacc agcgtttctg 5940
ggtgagcaaa aacaggaagg caaaatgccg caaaaaaggg aataagggcg acacggaaat 6000
gttgaatact catactcttc ctttttcaat attattgaag catttatcag ggttattgtc 6060
tcatgagcgg atacatattt gaatgtattt agaaaaataa acaaataggg gttccgcgca 6120
catttccccg aaaagtgcca cctgacgtc 6149